# EUROPÄISCHE PATENTSCHRIFT

(11) **EP 0 682 030 B1**
(45) Veröffentlichungstag und Bekanntmachung des Hinweises auf die Patenterteilung: **05.07.2000**
(21) Anmeldenummer: 95106400.5
(22) Anmeldetag: 28.04.1995
(51) Int. Cl.: C07D 498/06, A61K 31/535

(54) **8-Amino-10-(azabicycloalkyl)-pyrido[1,2,3-d,e] [1,3,4]benzoxadiazinderivate, Verfahren zu ihrer Herstellung sowie diese enthaltende antibakterielle Mittel**
8-Amino-10(azabicycloalkyl)-pyrido[1,2,3-d,e] [1,3,4] benzoxadiazine derivatives, process for their preparation and antibacterial agents containing them
Dérivés de 8-amino-10(azabicycloalkyl)-pyrido[1,2,3-d,e] [1,3,4] benzoxadiazine, procédé pour leur préparation et des agents antibactériens les contenant

(30) Priorität: 11.05.1994 DE 4416622
(43) Veröffentlichungstag der Anmeldung: 15.11.1995
(73) Patentinhaber: BAYER AG, 51368 Leverkusen (DE)
(72) Erfinder: Jaetsch, Thomas, Dr., D-50668 Köln (DE); Mielke, Burkhard, Dr., D-51375 Leverkusen (DE); Petersen, Uwe, Dr., D-51375 Leverkusen (DE); Schenke, Thomas, Dr., D-51469 Bergisch Gladbach (DE); Bremm, Klaus-Dieter, Dr., D-45661 Recklinghausen (DE); Endermann, Rainer, Dr., D-42113 Wuppertal (DE); Metzger, Karl-Georg, Dr., D-42115 Wuppertal (DE); Scheer, Martin, Dr., D-42113 Wuppertal (DE); Stegemann, Michael, Dr., D-51381 Leverkusen (DE); Wetzstein, Heinz-Georg, Dr., D-51377 Leverkusen (DE)

(56) Entgegenhaltungen:
- EP-A- 0 259 804
- EP-A- 0 343 524
- EP-A- 0 683 169
- DE-A- 4 329 600
- JOURNAL OF MEDICINAL CHEMISTRY., Bd.33, Nr.8, 1990, WASHINGTON US Seiten 2270 - 2275 T. P. CULBERTSON ET AL 'Quinolone antibacterial agents substituted at the 7-position with spiroamines. Synthesis and structure-activity relationships'
- JOURNAL OF MEDICINAL CHEMISTRY., Bd.31, Nr.3, 1988, WASHINGTON US Seiten 503 - 506 J. M. DOMAGALA ET AL '7-Substituted 5-amin o-1cyclopropyl-6,8-difluoro-1,4-dihydro-4- oxo-3-quinolinecarboxylic acids: Synthesis and biological activity of a new class of quinolone antibacterials'
- EUROPEAN JOURNAL OF MEDICINAL CHEMISTRY.CHIMICA THERAPEUTICA., Bd.26, 1991, CHATENAY-MALABRY FR Seiten 889 - 906 M. OGATA ET AL 'Synthesis and antibacterial activity of new 7-(aminoazab icycloalkanyl)quinolonecarboxylic acids'

## Beschreibung

Die vorliegende Erfindung betrifft neue 8-Amino-10-(azabicycloalkyl)-pyrido[1,2,3-d,e][1,3,4]benzoxadiazinderivate, Verfahren zu ihrer Herstellung sowie diese enthaltende antibakterielle Mittel.

Es ist bereits bekannt geworden, daß derartige Pyridobenzoxadiazincarbonsäuren antibakteriell wirksam sind. Beispiele hierfür finden sich in EP-O 259 804, EP-O 343 524 und im European Journal of Medicinal Chemistry 26, 889 (1991).

Die vorliegende Erfindung betrifft:
1. Neue 8-Amino-10-(azabicycloalkyl)-pyrido[1,2,3-d,e][1,3,4]benzoxadiazinderivate der allgemeinen Formel (I) 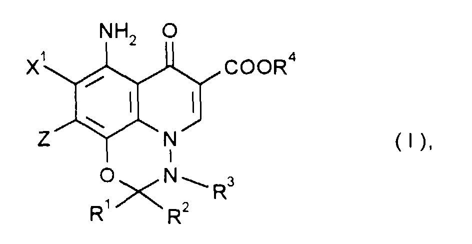 in welcher
   - R¹: für Wasserstoff oder gegebenenfalls durch Hydroxy oder Halogen substituiertes C₁-C₄-Alkyl steht,
   - R²: unabhängig von R¹ für Wasserstoff oder Methyl steht,
   - R³: für Wasserstoff oder C₁-C₄-Alkyl steht,
   - R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
   - X¹: für Wasserstoff oder Halogen steht,
   - Z: für Reste der Strukturen 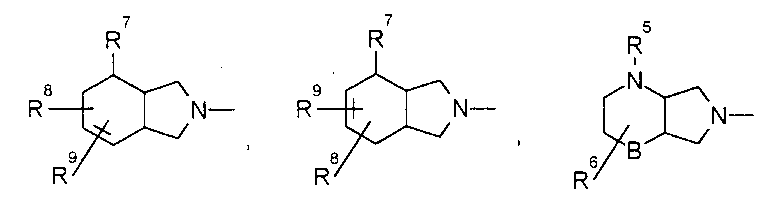
   steht,
   worin
   - R⁷: für Wasserstoff, Hydroxy, -NR¹⁰R¹¹, Hydroxymethyl, -CH₂-NR¹⁰R¹¹, Carboxyl, Methoxycarbonyl oder Ethoxycarbonyl steht,
   wobei
   R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
   R¹¹ für Wasserstoff oder Methyl steht,
   - R⁸: für Wasserstoff, geradkettiges oder verzweigtes C₁-C₃-Alkyl oder Cyclopropyl steht,
   - R⁹: für Wasserstoff oder Methyl steht,
   - R⁶: für Wasserstoff oder Methyl steht,
   - R⁵: für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
   - R^{5'}: für Methyl oder Ethyl steht,
   - B: für -CH₂-, O oder eine direkte Bindung steht.

   Die Verbindungen der Formel (I) können in Form von Racematen oder als enantiomerenreine Verbindungen sowie in Form ihrer pharmazeutisch verwendbaren Hydrate und Säureadditionssalze sowie in Form ihrer Alkali-, Erdalkali-, Silber- und Guanidiniumsalze vorliegen.
2. Verfahren zur Herstellung der
   1. Neue 8-Amino-10-(azabicycloalkyl-pyrido[1,2,3-d,e][1,3,4]benzoxadiazinderivate der allgemeinen Formel (I) 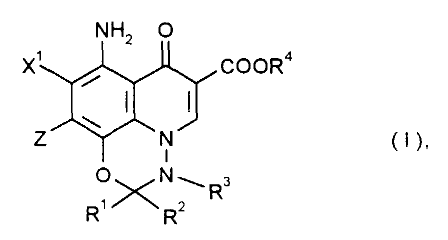 in welcher
   - R¹: für Wasserstoff oder gegebenenfalls durch Hydroxy oder Halogen substituiertes C₁-C₄-Alkyl steht,
   - R²: unabhängig von R¹ für Wasserstoff oder Methyl steht,
   - R³: für Wasserstoff oder C₁-C₄-Alkyl steht,
   - R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
   - X¹: für Wasserstoff oder Halogen steht,
   - Z: für Reste der Strukturen 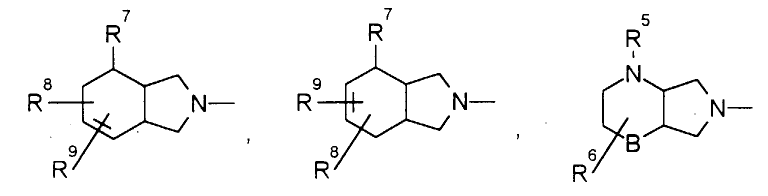 steht,
   worin
   - R⁷: für Wasserstoff, Hydroxy, -NR¹⁰R¹¹, Hydroxymethyl, -CH₂-NR¹⁰R¹¹, Carboxyl, Methoxycarbonyl oder Ethoxycarbonyl steht,
   wobei
   R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
   R¹¹ für Wasserstoff oder Methyl steht,
   - R⁸: für Wasserstoff, geradkettiges oder verzweigtes C₁-C₃-Alkyl oder Cyclopropyl steht,
   - R⁹: für Wasserstoff oder Methyl steht,
   - R⁶: für Wasserstoff oder Methyl steht,
   - R⁵: für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
   - R^{5'}: für Methyl oder Ethyl steht,
   - B: für -CH₂-, O oder eine direkte Bindung steht,
   dadurch, gekennzeichnet, daµ man Verbindungen der Formel (II) 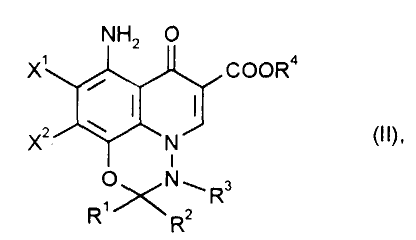 in welcher
   - R¹, R², R³, R⁴ und X¹: die oben angegebene Bedeutung haben und
   - X²: für Halogen, insbesondere Fluor oder Chlor, steht,
   mit Verbindungen der Formel (III)

   Z-H (III),

   in welcher
   - Z: die oben angegebene Bedeutung hat,
   gegebenenfalls in Gegenwart von Säurebindemitteln umsetzt.
3. Verbindungen der Formel (II) 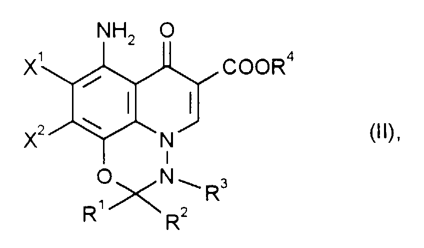 in welcher
   - R¹, R², R³, R⁴ und X¹: die unter Punkt 1 angegebene Bedeutung haben und
   - X²: für Halogen steht.
4. Verfahren zur Herstellung der Verbindungen der Formel (II) dadurch gekennzeichnet, daß man Verbindungen der Formel (IV) 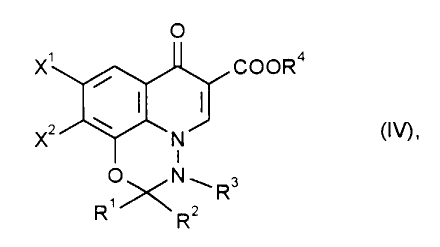 in welcher R¹, R², R³, R⁴, X¹ und X² die unter Punkt 3 angegebene Bedeutung haben, mit Nitrierungsreagenzien umsetzt und anschließend die erhaltenen Nitroverbindungen reduziert.

Die erfindungsgemäßen Verbindungen weisen im Vergleich zu bekannten Vertretern dieses Strukturtyps eine höhere antibakterielle Wirkung insbesondere im grampositiven Bereich auf. Sie eignen sich daher als Wirkstoffe für die Human- und Veterinärmedizin.

Bevorzugt sind Verbindungen der Formel (I), in welcher
- R¹: für Wasserstoff oder gegebenenfalls durch Hydroxy substituiertes C₁-C₃-Alkyl steht,
- R²: unabhängig von R¹ für Wasserstoff oder Methyl steht,
- R³: für Wasserstoff, Methyl oder Ethyl steht,
- R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)methyl steht,
- X¹: für Wasserstoff, Fluor oder Chlor steht,
- Z: für Reste der Strukturen 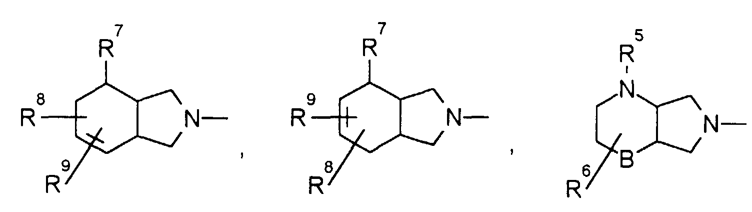 steht,
worin
- R⁷: für Wasserstoff, Hydroxy, -NR¹⁰R¹¹, Hydroxymethyl oder -CH₂-NR¹⁰R¹¹ steht,
wobei
R¹⁰ für Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl steht,
R¹¹ für Wasserstoff oder Methyl steht,
- R⁸: für Wasserstoff, geradkettiges oder verzweigtes C₁-C₃-Alkyl oder Cyclopropyl steht,
- R⁹: für Wasserstoff oder Methyl steht,
- R⁵: für Wasserstoff oder Methyl steht,
- R⁶: für Wasserstoff steht,
- B: für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

Besonders bevorzugt sind die Verbindungen der Formel (I), in welcher
- R¹: für Wasserstoff oder Methyl steht,
- R²: für Wasserstoff,
- R³: für Methyl oder Ethyl steht,
- R⁴: für Wasserstoff, Methyl oder Ethyl steht,
- X¹: für Fluor steht,
- Z: für Reste der Strukturen 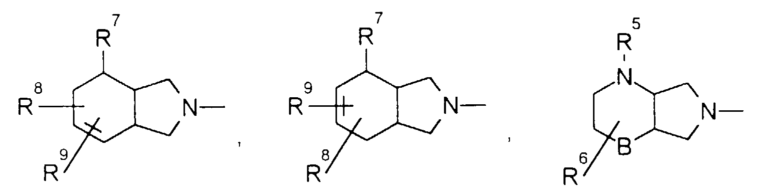 steht,
worin
- R⁷: für Wasserstoff, Hydroxy, -NR¹⁰R¹¹ , Hydroxymethyl oder -CH₂-NR¹⁰R¹¹ steht,
wobei
R¹⁰ für Wasserstoff, Methyl, Alkoxycarbonyl mit 1 bis 4 C-Atomen im Alkoxyteil oder C₁-C₃-Acyl steht,
R¹¹ für Wasserstoff oder Methyl steht,
- R⁸: für Wasserstoff, geradkettiges oder verzweigtes C₁-C₃-Alkyl oder Cyclopropyl steht,
- R⁶: für Wasserstoff steht,
- R⁹: für Wasserstoff oder Methyl steht,
- R⁵: für Wasserstoff oder Methyl steht,
- B: für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

Im einzelnen seien folgende Verbindungen der Formel (I) genannt:

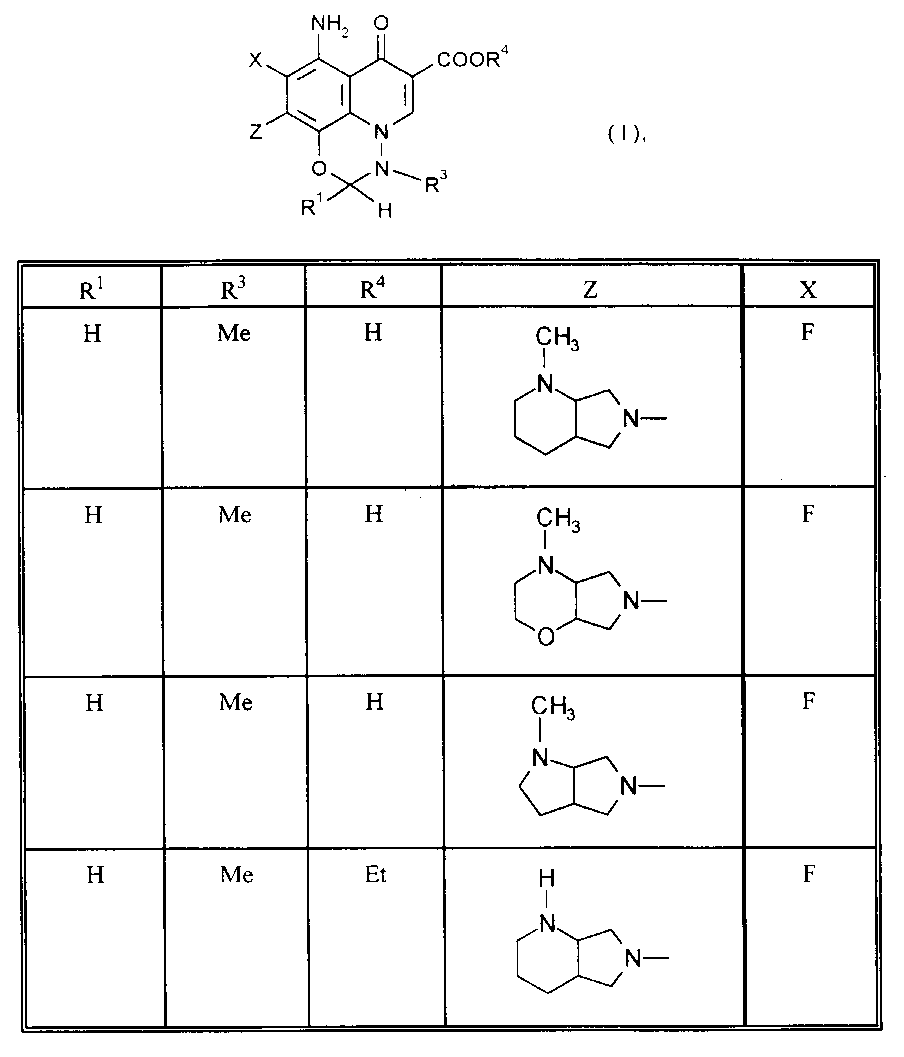

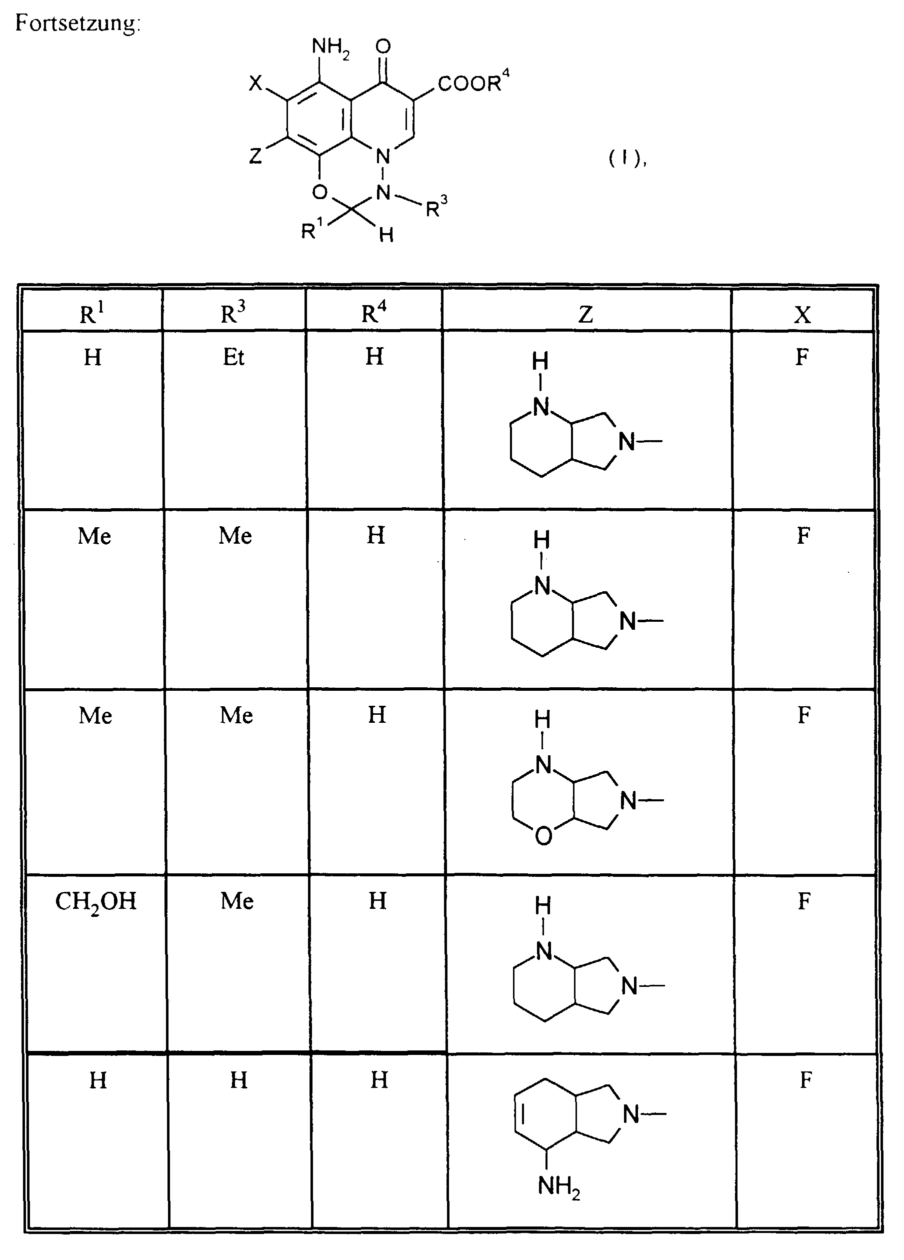

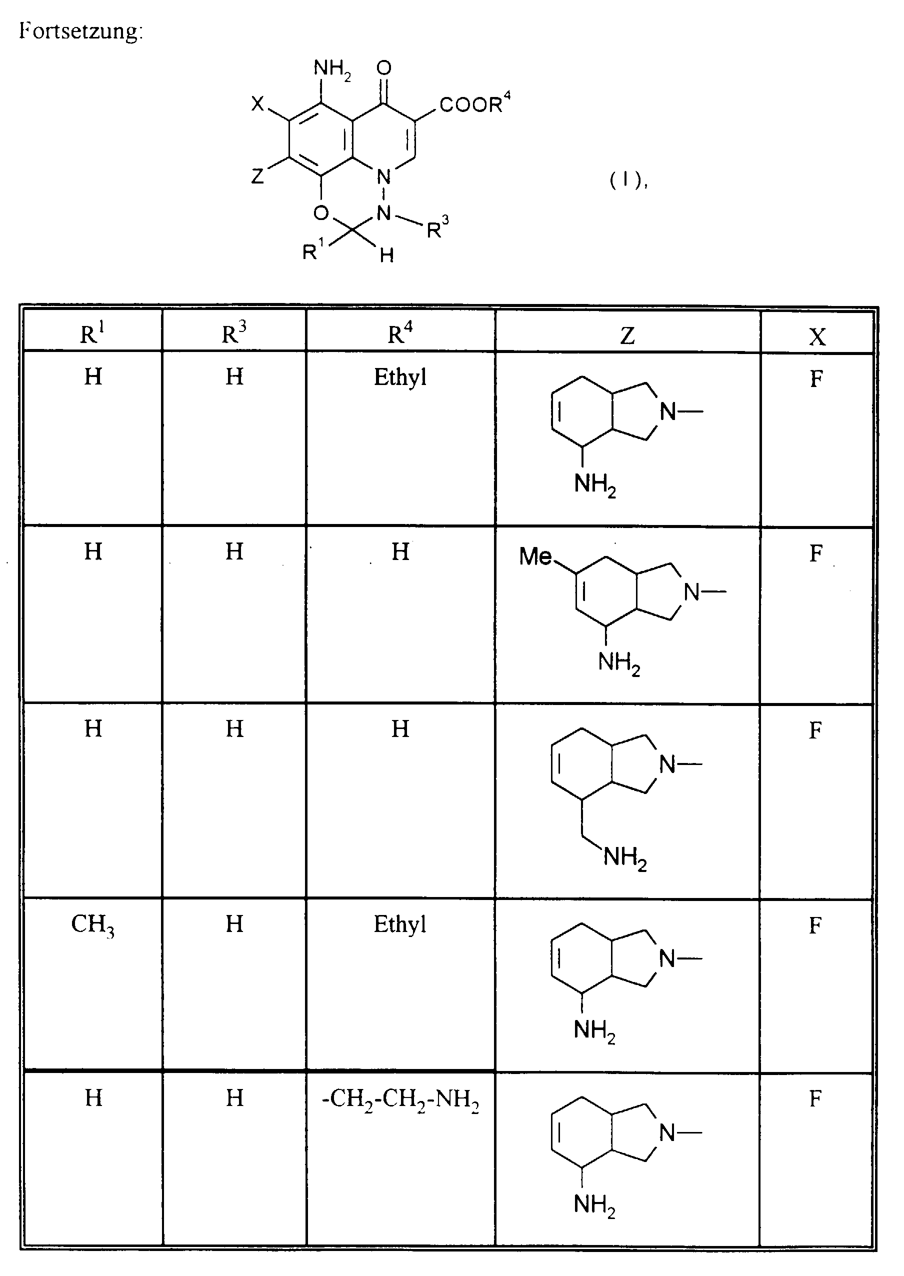

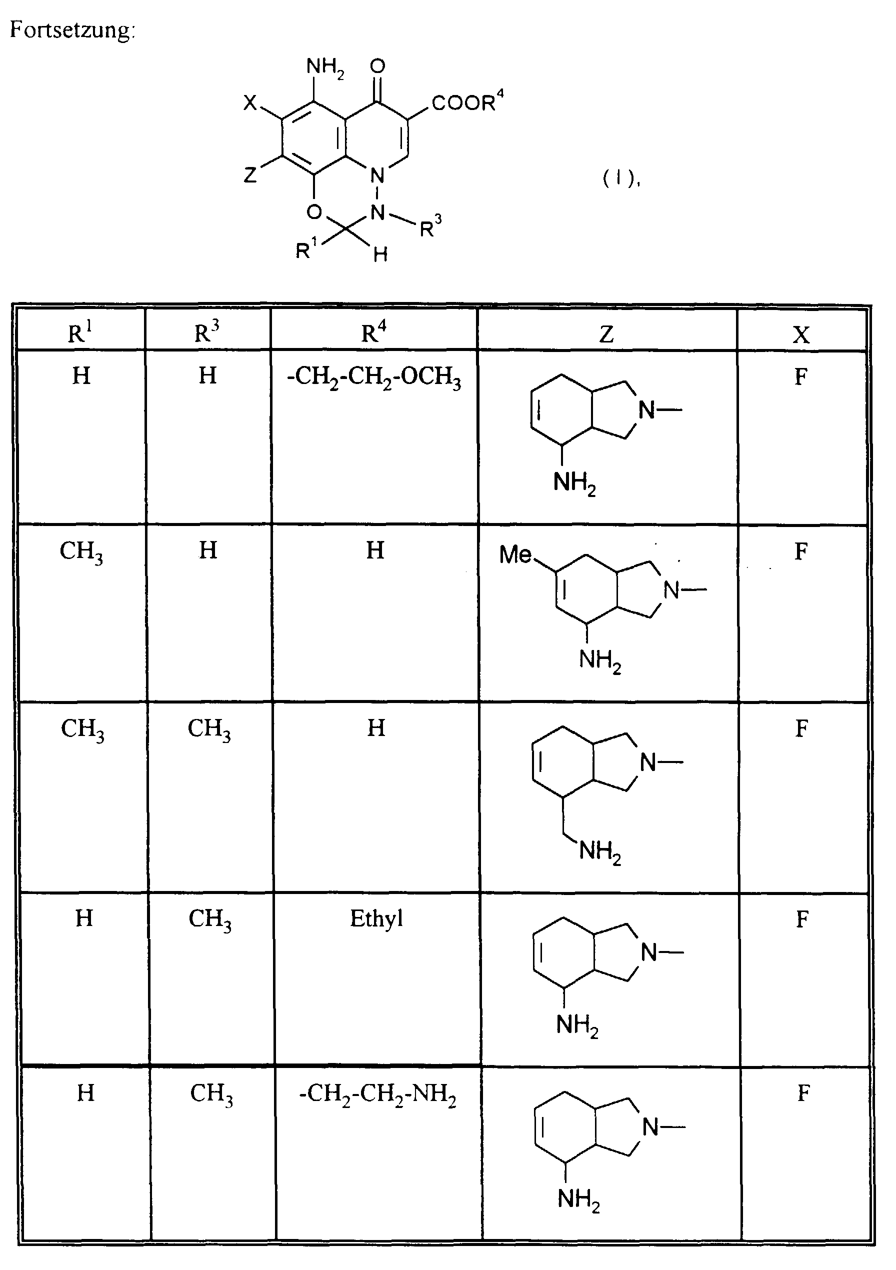

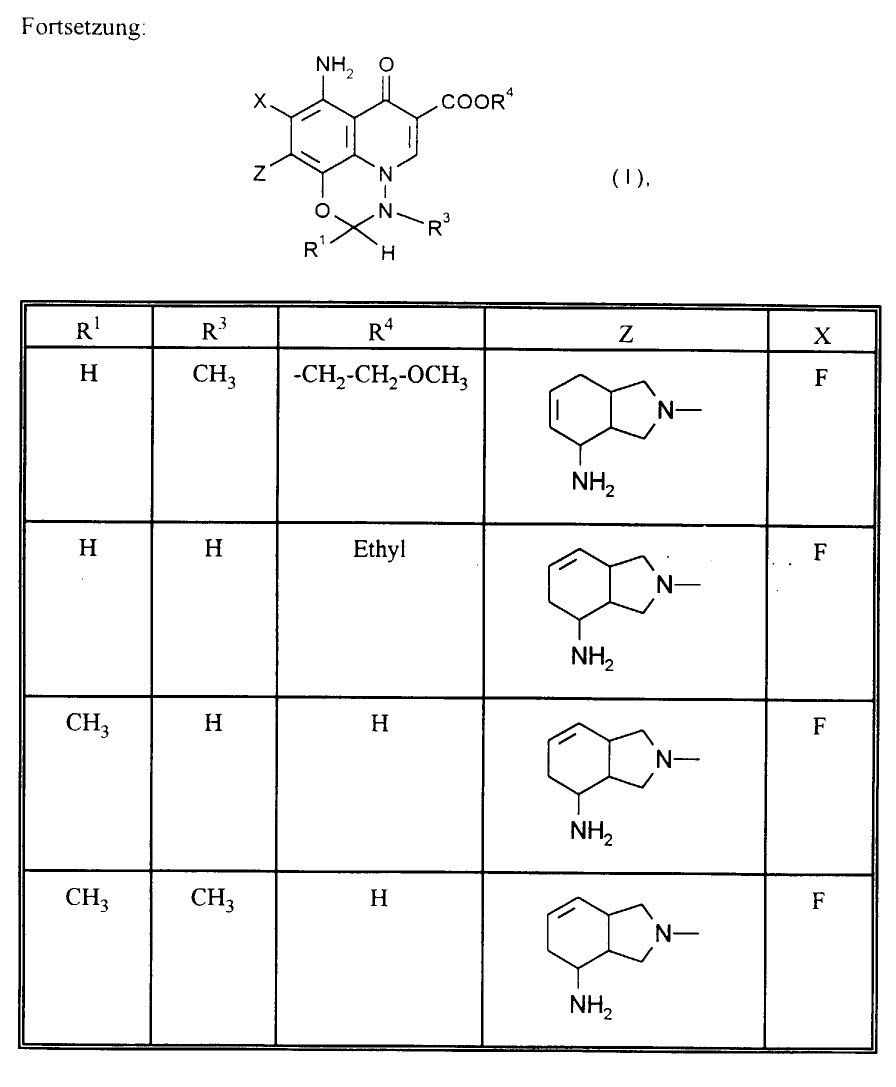

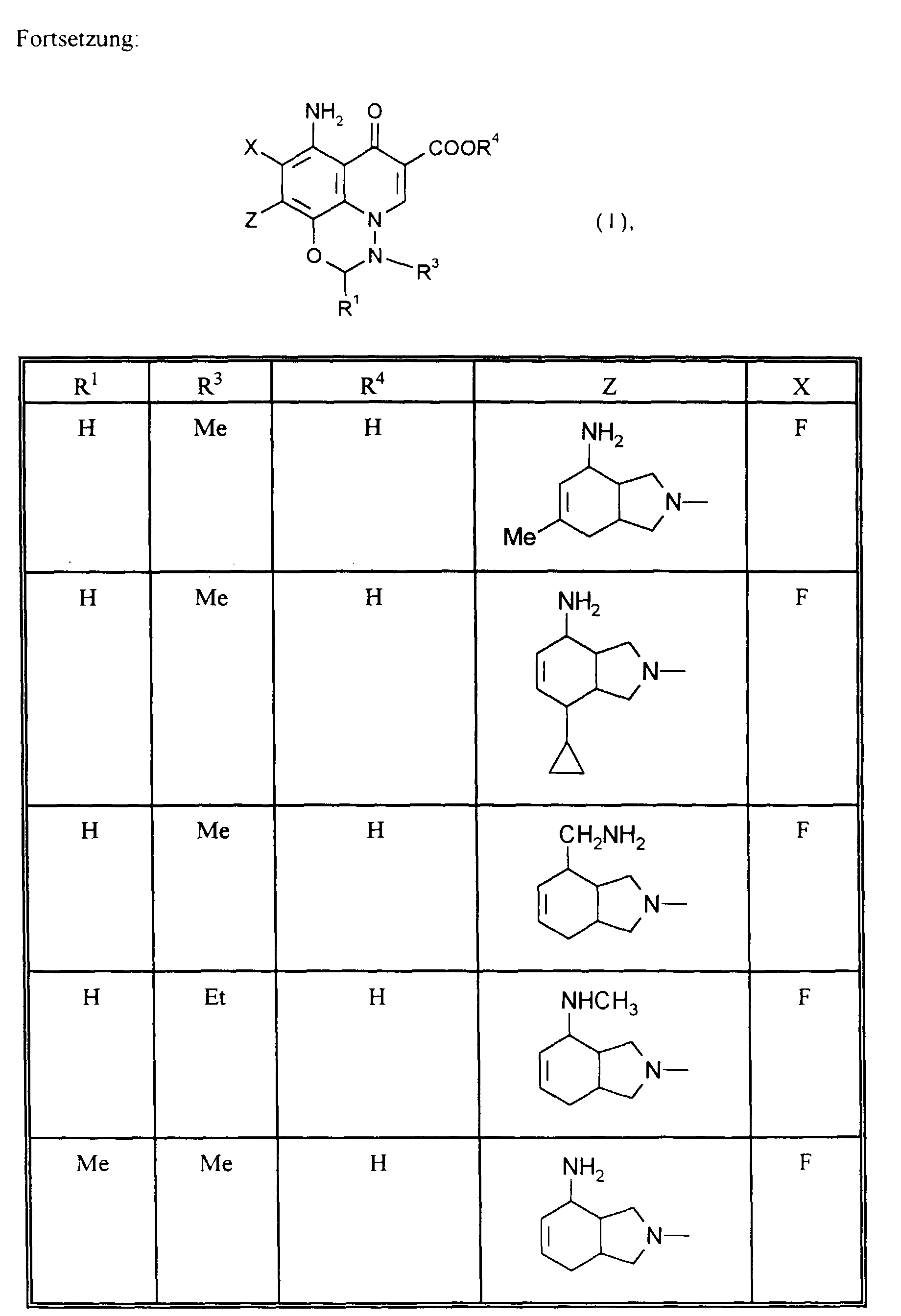

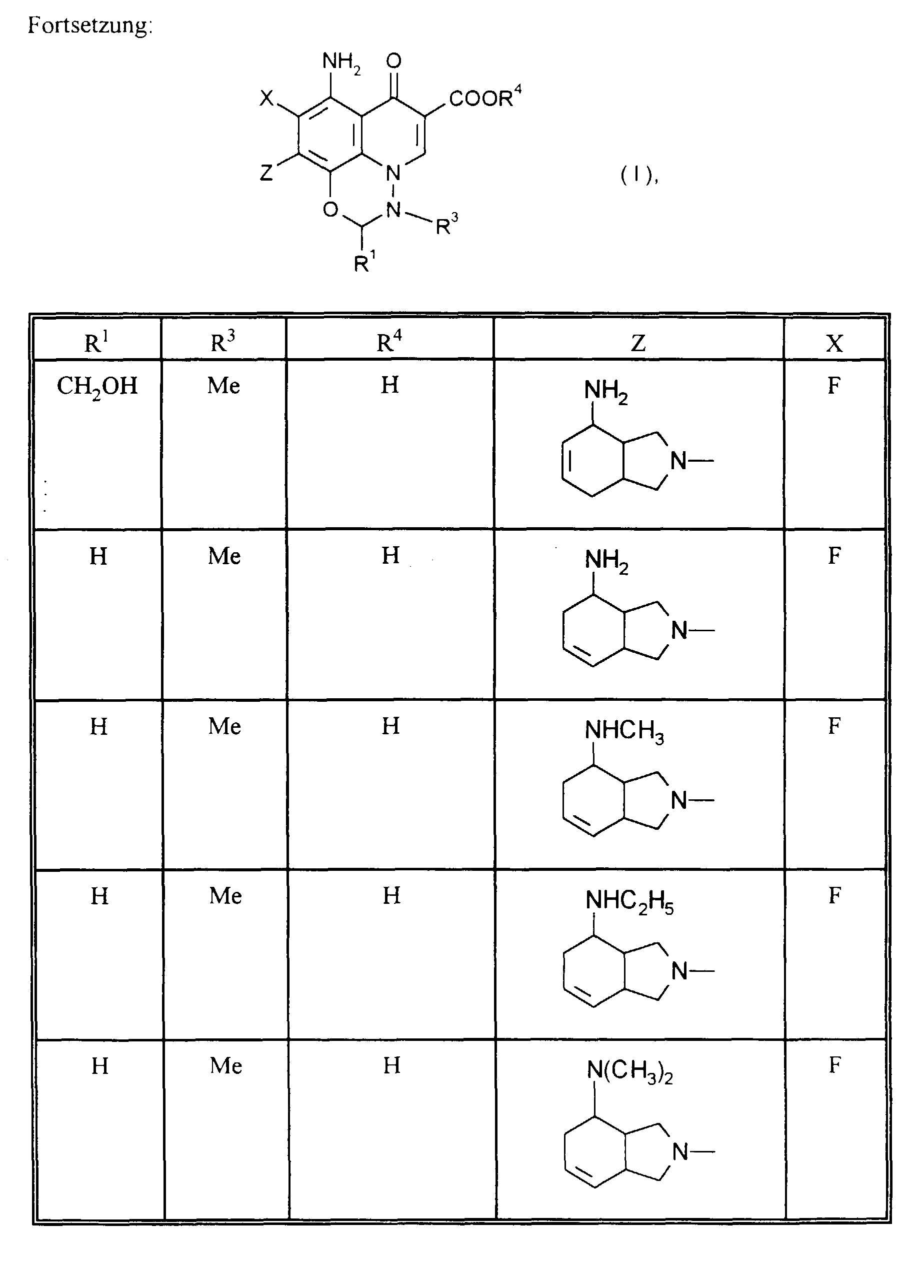

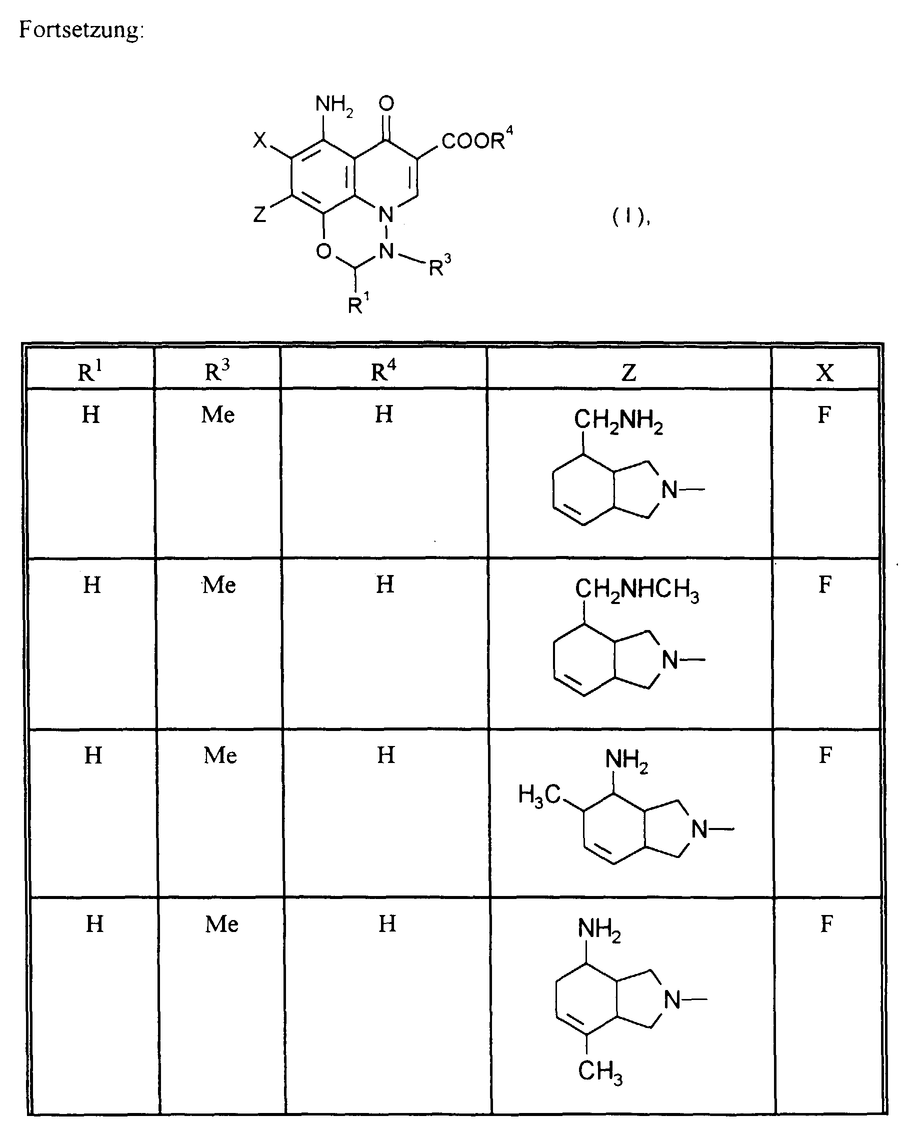

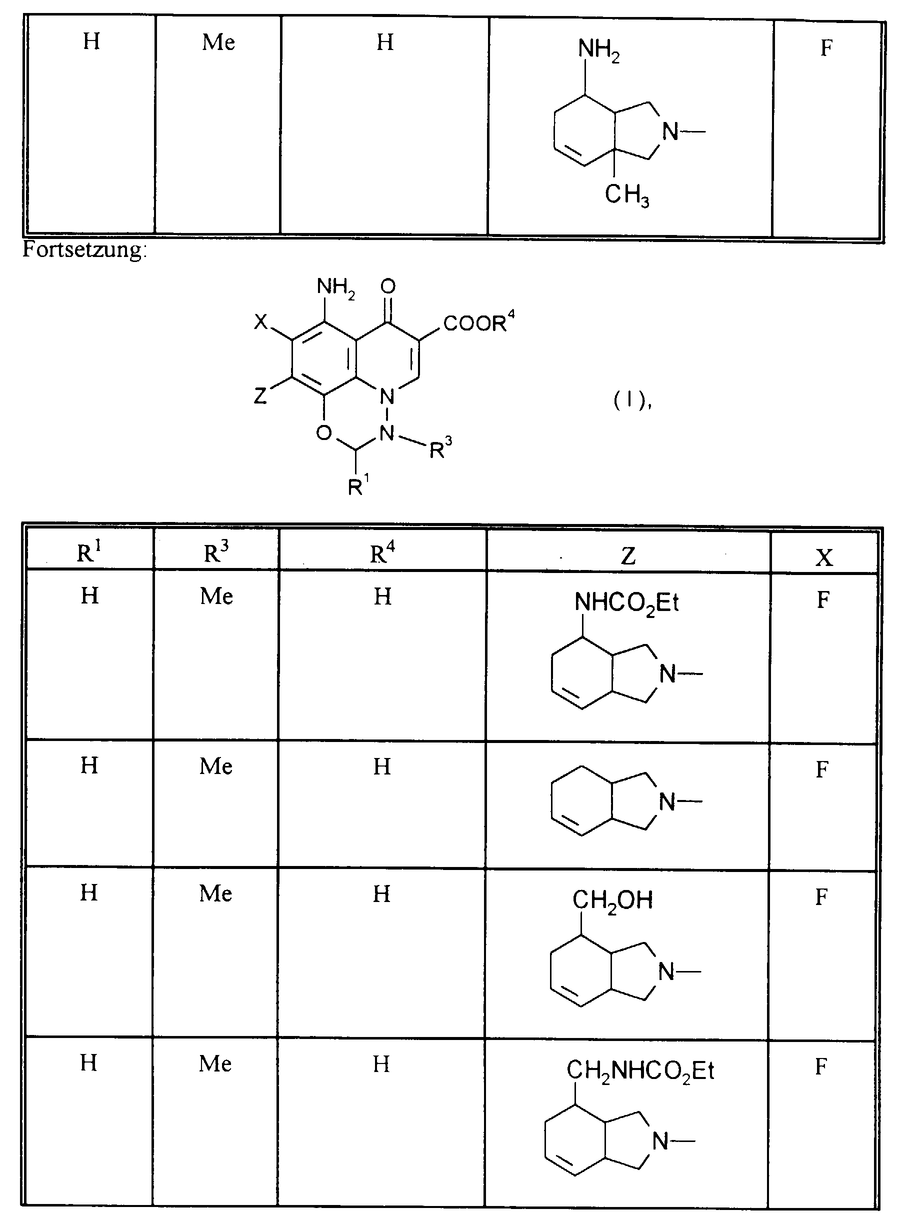

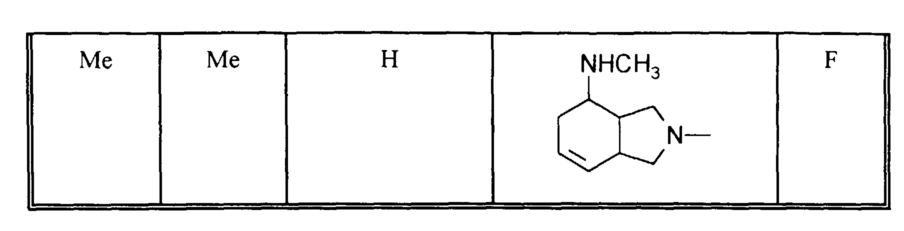

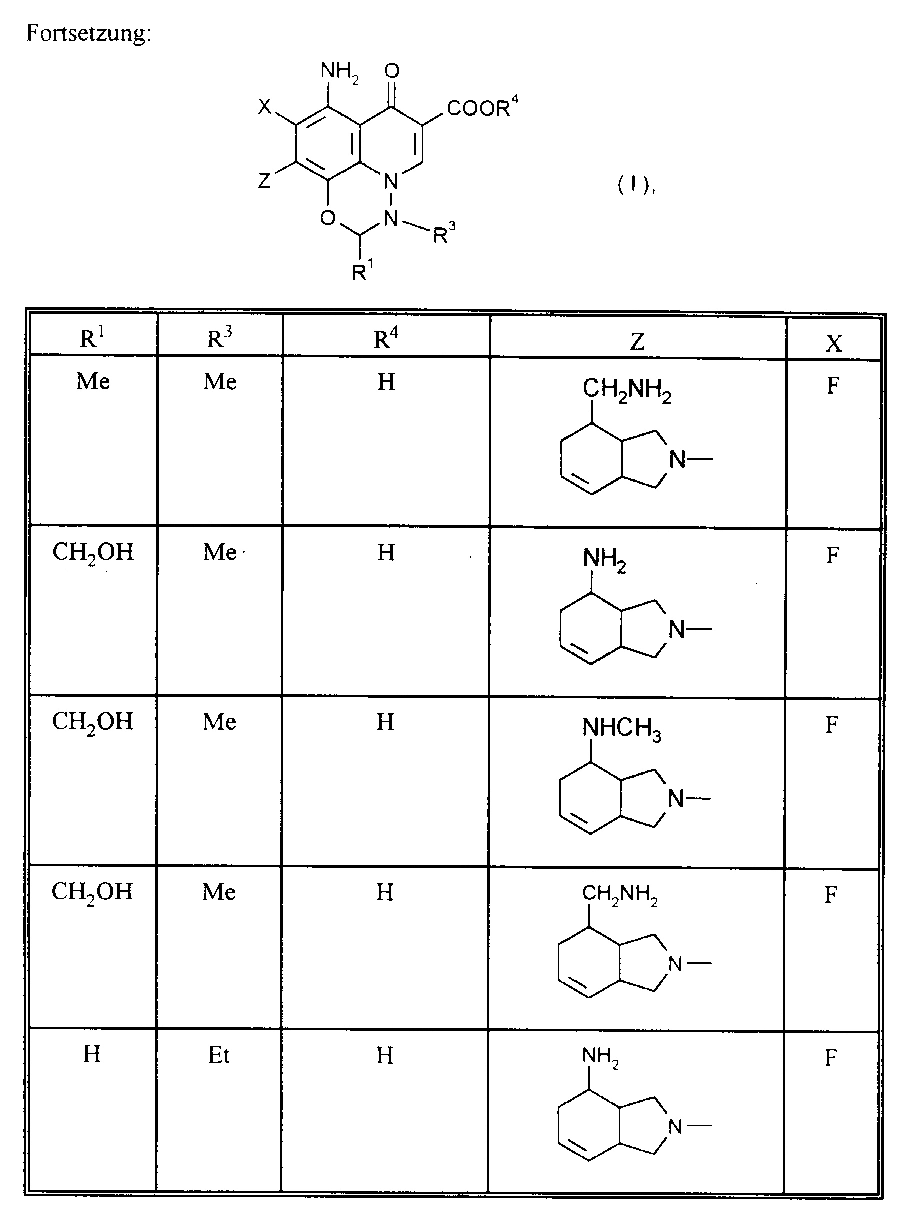

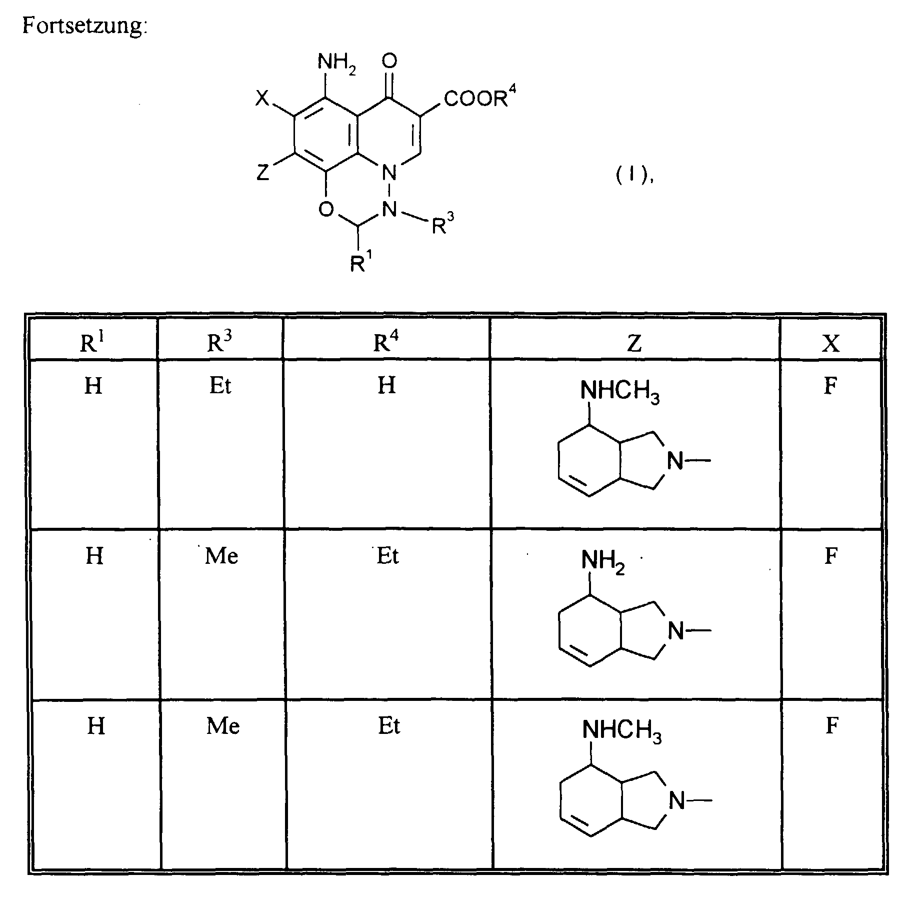

Verwendet man zur Herstellung von Verbindungen der Formel (1) beispielsweise 8-Amino-9,10-difluor-3-methyl-7-oxo-2,3-dihydro-7H-pyrido[1,2,3-d,e][1,3,4]benzoxadiazin-6-carbonsäure und 2,8-Diazabicyclo[4.3.0]nonan, so kann der Reaktionsverlauf durch das folgende Formelschema wiedergegeben werden: 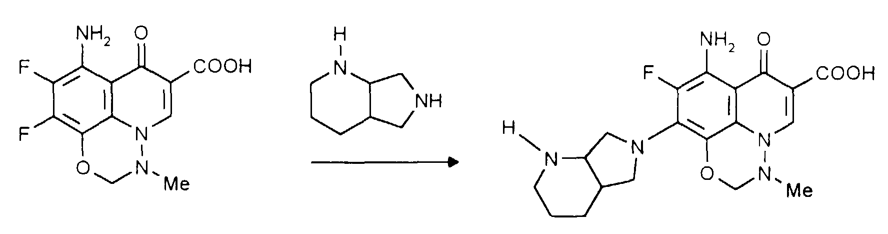 Die als Ausgangsverbindungen verwendeten Verbindungen der Formel (II) sind neu. Sie können hergestellt werden, indem man Verbindungen der Formel (IV) 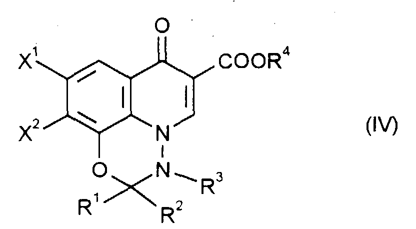 in welcher R¹, R², R³, R⁴, X¹ und X² die oben angegebene Bedeutung haben,
mit Nitrierungsreagenzien wie Salpetersäure und Nitraten in einem Lösungsmittel wie zum Beispiel Wasser, Schwefelsäure, Essigsäure, Acetanhydrid oder deren Gemischen bei -50 bis 200°C, vorzugsweise bei -20 bis 100°C umsetzt und anschließend die erhaltenen Nitroverbindungen reduziert.

Zur Reduktion der Nitrogruppe können Metallhydride, Übergangsmetalle und Übergangsmetallsalze eingesetzt werden; bevorzugt wird Wasserstoff in Gegenwart von Katalysatoren wie zum Beispiel Palladium-Kohle, Raney-Nickel und Platin verwendet. Als Lösungsmittel können zum Beispiel Wasser, Salzsäure, Alkohole, Essigsäure oder auch deren Gemische verwendet werden.

Verbindungen der Formel (II) können auch nach folgendem Reaktionsschema, in dem R¹, R², R³, R⁴, X¹ und X² die oben angegebene Bedeutung besitzen, hergestellt werden: 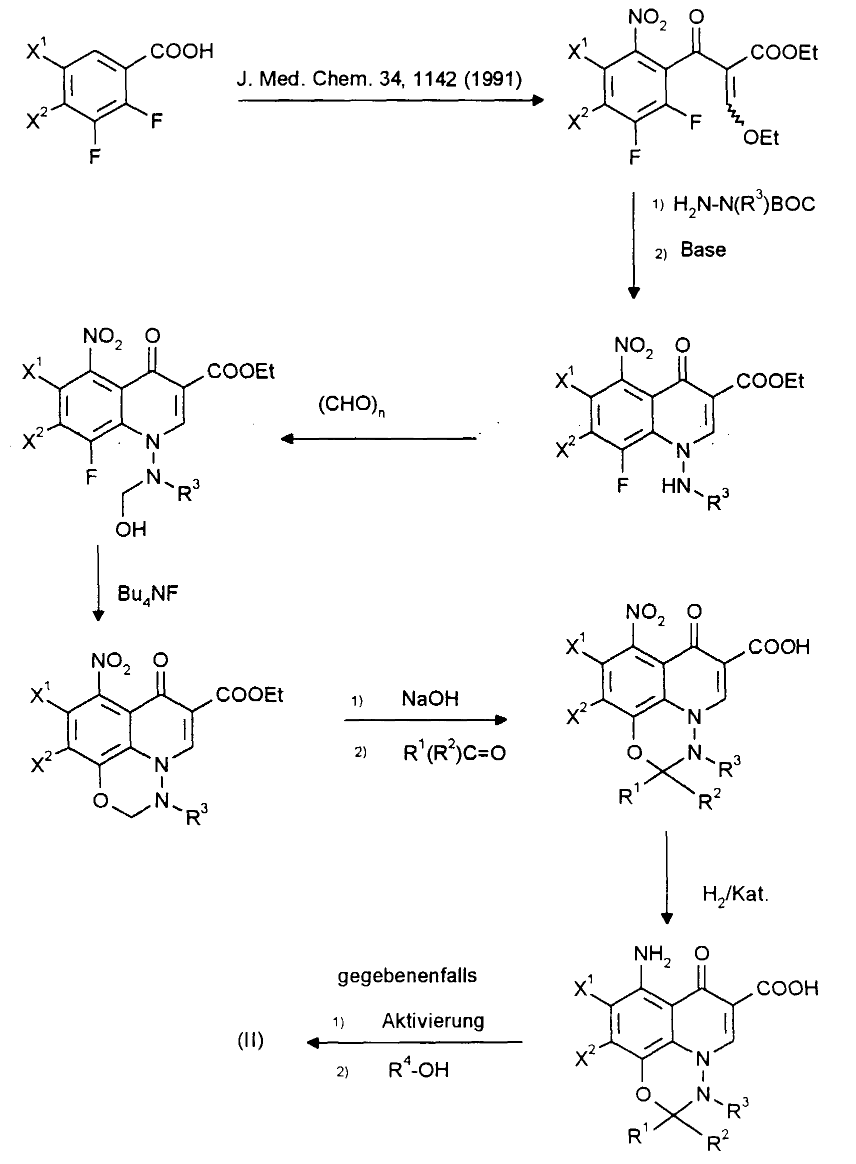 Sie können gegebenenfalls als Racemate, Enantiomere oder reine Diastereomere eingesetzt werden.

Als Beispiele seien genannt:
8-Amino-9,10-difluor-3-methyl-7-oxo-2,3-dihydro-7H-pyrido[1,2,3-d,e][1,3,4]benzoxadiazin-6-carbonsäure
8-Amino-9,10-difluor-2,3-dimethyl-7-oxo-2,3-dihydro-7H-pyrido[1,2,3-d,e][1,3,4]benzoxadiazin-6-carbonsäure
8-Amino-9,10-difluor-2-(hydroxymethyl)-3-methyl-7-oxo-2,3-dihydro-7H-pyrido[1,2,3-d,e]-[1,3,4]benzoxadiazin-6-carbonsäure
8-Amino-9,10-difluor-3-ethyl-7-oxo-2,3-dihydro-7H-pyrido[1,2,3-d,e][1,3,4]benzoxadiazin-6-carbonsäure
8-Amino-9,10-difluor-3-methyl-7-oxo-2,3-dihydro-7H-pyrido[1,2,3-d,e][1,3,4]benzoxadiazin-6-carbonsäureethylester
Die als Ausgangsverbindungen verwendeten Amine der Formel (III) sind bekannt. Chirale Amine können sowohl als Racemate, als auch als enantiomeren- oder diastereomerenreine Verbindungen eingesetzt werden.

Als Beispiele seien genannt:
2,7-Diazabicyclo[3.3.0]octan
2-Methyl-2,7-diazabicyclo[3.3.0]octan
2,8-Diazabicyclo[4.3.0]nonan
2-Methyl-2,8-diazabicyclo[4.3.0]nonan
2-Oxa-5,8-diazabicyclo[4.3.0]nonan
5-Methyl-2-oxa-5,8-diazabicyclo[4.3.0]nonan.
2-Amino-8-azabicyclo[4.3.0]non-3-en
2-Methylamino-8-azabicyclo[4.3.0]non-3-en
4-Methyl-2-methylamino-8-azabicyclo[4.3.0]non-3-en
5-Methyl-2-methylamino-8-azabicyclo[4.3.0]non-3-en
2-Dimethylamino-8-azabicyclo[4.3.0]non-3-en
2-Ethylamino-8-azabicyclo[4.3.0]non-3-en
2-Methylaminomethyl-8-azabicyclo[4.3.0]non-3-en
2-Hydroxy-8-azabicyclo[4.3.0]non-3-en
5-Isopropyl-2-methylamino-8-azabicyclo[4.3.0]non-3-en
2-Amino-5-isopropyl-8-azabicyclo[4.3.0]non-3-en
2-Amino-5-methyl-8-azabicyclo[4.3.0]non-3-en
2-Hydroxymethyl-8-azabicyclo[4.3.0]non-3-en
2-Amino-5-cyclopropyl-8-azabicyclo[4.3.0]non-3-en
8-Azabicyclo[4.3.0]non-2-en
8-Azabicyclo[4.3.0]non-4-en-2-carbonsäureethylester
2-Hydroxymethyl-8-azabicyclo[4.3.0]non-4-en
2-Amino-8-azabicyclo[4.3.0]non-4-en
2-Ethyloxycarbonylamino-8-azabicyclo[4.3.0]non-4-en
2-tert.-Butyloxycarbonylamino-8-azabicyclo[4.3.0]non-4-en
2-Benzyloxycarbonylamino-8-azabicyclo[4.3.0]non-4-en
2-Allyloxycarbonylaminomethyl-8-azabicyclo[4.3.0]non-4-en
2-Aminomethyl-8-azabicyclo[4.3.0]non-4-en
2-Ethyloxycarbonylaminomethyl-8-azabicyclo[4.3.0]non-4-en
2-tert.-Butyloxycarbonylaminomethyl-8-azabicyclo[4.3.0]non-4-en
2-Methylamino-8-azabicyclo[4.3.0]non-4-en
2-Ethylamino-8-azabicyclo[4.3.0]non-4-en
2-Cyclopropylamino-8-azabicyclo[4.3.0]non-4-en
2-Dimethylamino-8-azabicyclo[4.3.0]non-4-en
2-[(2-Hydroxyethyl)-amino]-8-azabicyclo[4.3.0]non-4-en
2-Amino-1-methyl-8-azabicyclo[4.3.0]non-4-en
2-Amino-2-methyl-8-azabicyclo[4.3.0]non-4-en
2-Amino-3-methyl-8-azabicyclo[4.3.0]non-4-en
2-Ethyloxycarbonylamino-3-methyl-8-azabicyclo[4.3.0]non-4-en
2-tert.-Butyloxycarbonylamino-3-methyl-8-azabicyclo[4.3.0]non-4-en
2-Benzyloxycarbonylamino-3-methyl-8-azabicyclo[4.3.0]non-4-en
2-Allyloxycarbonylaminomethyl-3-methyl-8-azabicyclo[4.3.0]non-4-en
2-Amino-4-methyl-8-azabicyclo[4.3.0]non-4-en
2-Amino-5-methyl-8-azabicyclo[4.3.0]non-4-en
2-Amino-6-methyl-8-azabicyclo[4.3.0]non-4-en
2-Amino-7-methyl-8-azabicyclo[4.3.0]non-4-en
2-Amino-9-methyl-8-azabicyclo[4.3.0]non-4-en

Die substituierten 8-Azabicyclo[4.3.0]non-4-ene und 8-Azabicyclo[4.3.0]non-2-en sind Gegenstand einer noch nicht zum Stand der Technik gehörenden deutschen Anmeldung der Anmelderin DE-P 4 230 804.6.

Verbindungen der allgemeinen Formel (IV) 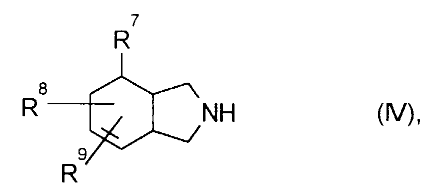 in welcher
- R⁷, R⁸ und R⁹: die oben angegebenen Bedeutungen haben,
werden erhalten, indem man geeignete Diene mit geeigneten Dienophilen in einer Diels-Alder Reaktion umsetzt, die intermolekular oder intramolekular durchgeführt werden kann, und gegebenenfalls anschließend weitere chemische Reaktionen durchführt, um gegebenenfalls den Pyrrolidinring aufzubauen und um für die biologische Wirkung gewünschte Substituenten einzuführen und als letzten Schritt die Schutzgruppe am Pyrrolidinstickstoff abspaltet.

Bei intramolekularer Durchführung der Diels-Alder-Reaktion werden Verbindungen der Formel (1) oder (2) 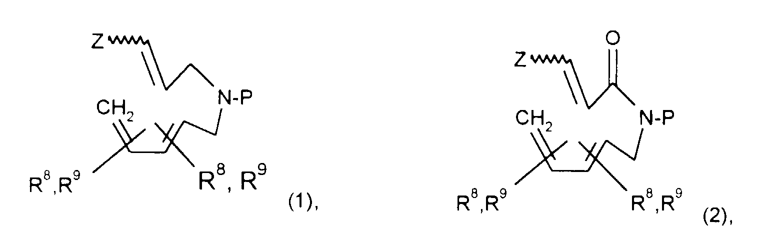 in denen
- R⁸ und R⁹: die oben angegebene Bedeutung haben und
- P: für eine Schutzgruppe (beispielsweise Allyl, Acyl, Carbamoyl oder Trityl) steht,
- Z: für Wasserstoff, eine Carboxyl-, Carbonester- oder Carbonamid-gruppe, CN oder NO₂ steht,
zu Verbindungen der Formel (3) [ausgehend von (1)] oder (4) [ausgehend von (2)] 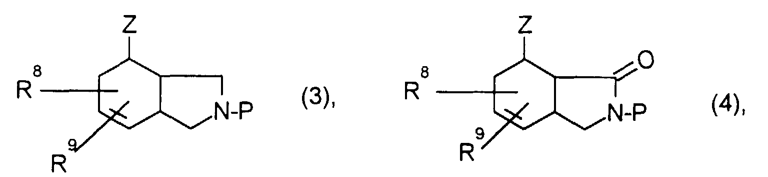 in denen
- R⁸, R⁹, P und Z: die oben angegebenen Bedeutungen haben,
umgesetzt.

Intramolekulare Diels-Alder-Reaktionen ähnlicher Art sind teilweise bekannt: J.M. Mellor, A.M. Wagland; J. Chem. Soc. Perkin I, 997-1005 (1989); W.R. Roush, S.E. Hall; J. Am. Chem. Soc. 103, 5200 (1980); E. Ciganek; Organic Reactions 32, 1-374 (1984). In diesen Arbeiten fehlen jedoch Hinweise auf Schutzgruppen, die gleichzeitig für die Reaktion geeignet und anschließend problemlos abspaltbar sind.

Bei intermolekularer Durchführung der Diels-Alder-Reaktion werden Diene der Formel (5) mit Dienophilen der Formel (6) zu Verbindungen der Formel (7) umgesetzt, und gegebenenfalls nach Modifizierung der Gruppen Z¹ und Z², beispielsweise Überführung eines cyclischen Carbonsäureanhydrids in einen Diester unter Abspaltung der Schutzgruppen P¹ oder P¹ und P², unter Cyclisierung zu den Lactamen der Formel (8) umgesetzt. 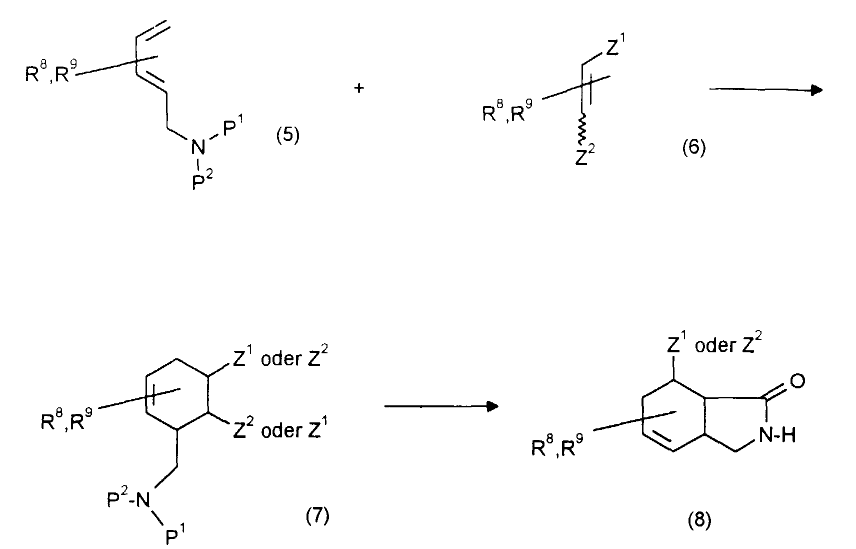 In der Formel (5), (6), (7) und (8) haben R⁸, R⁹ die oben angegebene Bedeutung,
- P¹: steht für eine Acyl- oder Carbamoylschutzgruppe, wenn
- P²: für Wasserstoff steht, oder
- P¹: bildet gemeinsam mit P² ein Imid,
- Z¹ und Z²: stehen für Wasserstoff Carboxyl, Carbonester- oder Carbonamid-gruppen, CN oder NO₂, wobei mindestens eine der beiden Gruppen Z¹ oder Z² eine Carbonestergruppe oder eine Carbonamidgruppe oder CN sein muß oder Z¹ und Z² bilden gemeinsam eine Brücke, so daß ein cyclisches Carbonsäureanhydrid gebildet wird.

Bevorzugte Schutzgruppen P, P¹, P² sind solche Schutzgruppen, bei denen unter den Bedingungen, die zu ihrer Abspaltung verwendet werden, die Cyclisierung zum Lactam und gegebenenfalls eine Veresterung einer zweiten, noch freien Carboxylfunktion mit dem als Lösungsmittel verwendeten Alkohol stattfindet, derart, daß alle Reaktionsschritte in einer Eintopfreaktion ausgeführt werden können, und eine unkontrollierte Umwandlung gegebenenfalls diastereomeren- und enantiomerenreiner Ausgangsstoffe in nicht oder schwer trennbare Isomerengemische nicht stattfindet.

Als Beispiele seien genannt:
1. die tert.-Butyloxycarbonylschutzgruppe
   (Abspaltung mit wäßrigen oder alkoholischen Säuren)
2. die Phthalimidoschutzgruppe
   (Aminolyse mit primären Aminen in wäßrigen oder wasserfreien Alkoholen als Lösungsmittel)

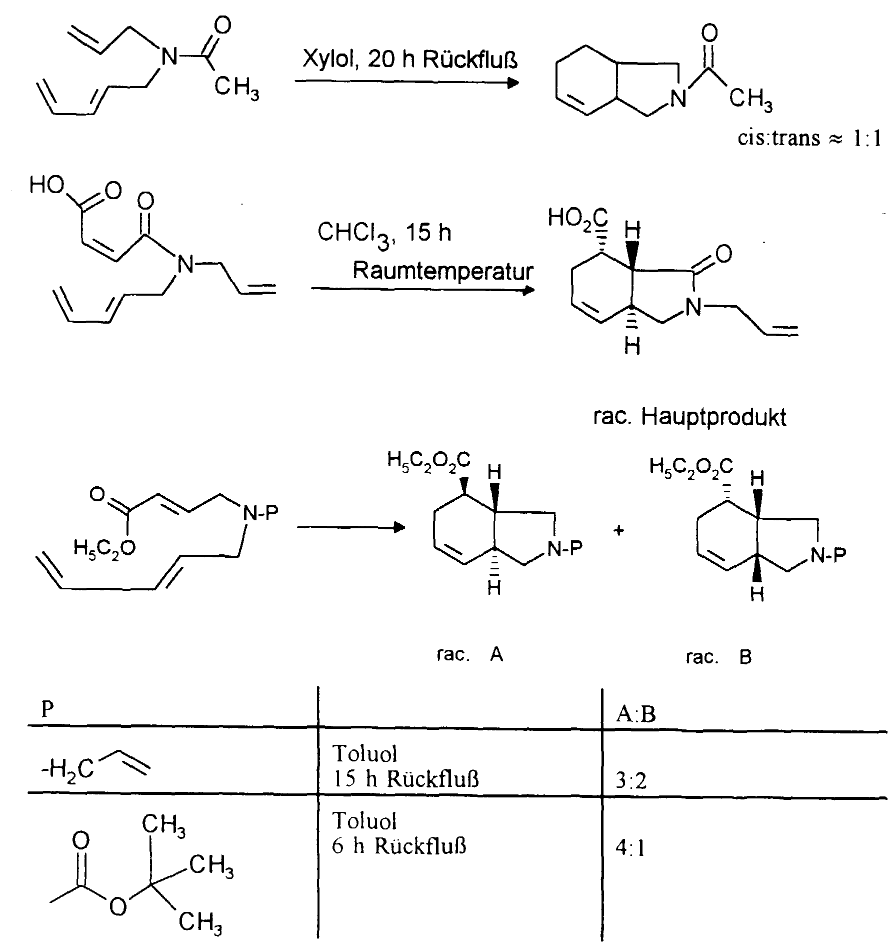

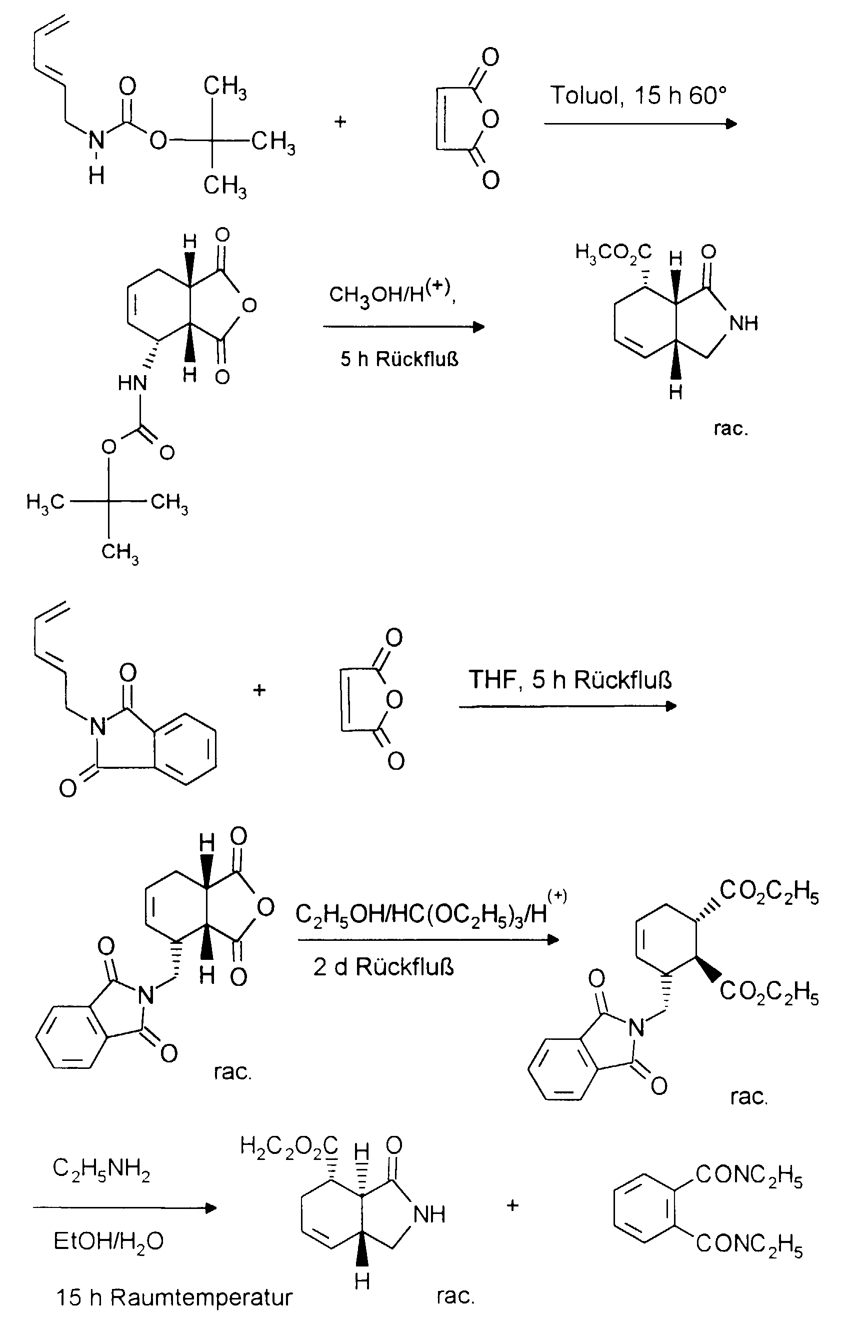

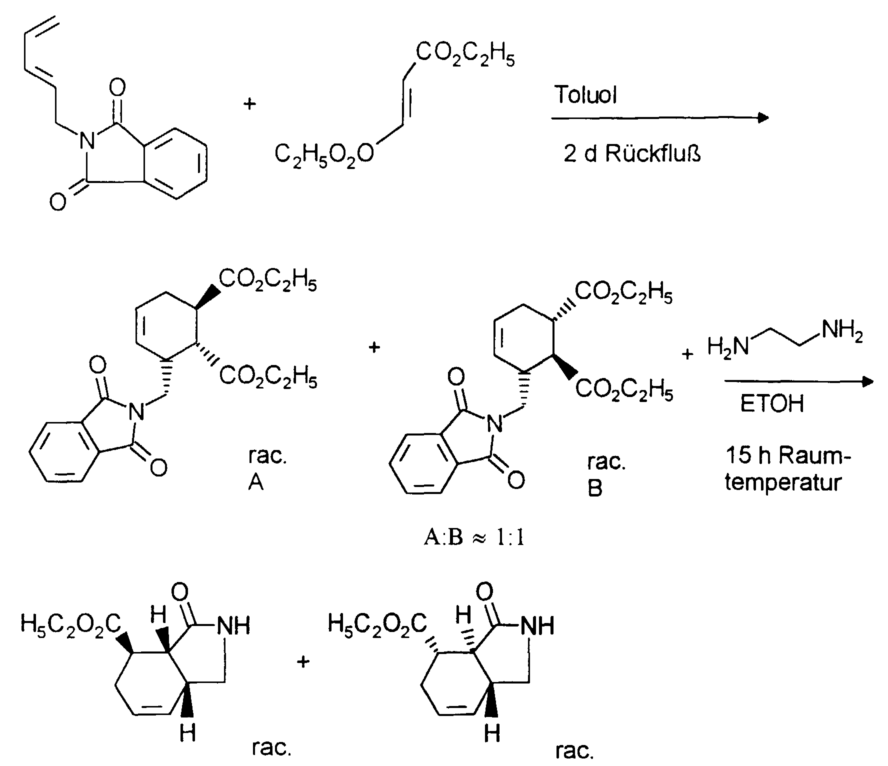

Für die Diels-Alder-Reaktion kommen als Verdünnungsmittel alle inerten organischen Lösungsmittel in Frage. Hierzu gehören vorzugsweise Ether wie Diisopropylether, Di-n-butylether, Dimethoxyethan, Tetrahydrofuran und Anisol, Kohlenwasserstoffe, wie z.B. Hexan, Methylcyclohexan, Toluol, Xylol und Mesitylen und halogenierte Kohlenwasserstoffe, wie z.B. Chloroform, 1,2-Dichlorethan und Chlorbenzol. Die Diels-Alder-Reaktion kann aber auch ohne Lösungsmittel durchgeführt werden.

Die Reaktionstemperaturen können in einem größeren Bereich variiert werden. Im allgemeinen arbeitet man zwischen etwa -20°C und +200°C, vorzugsweise zwischen -20°C und +150°C. Die Diels-Alder-Reaktion wird normalerweise bei Normaldruck durchgeführt. Zur Beschleunigung der Reaktion können aber auch Drucke bis zu 1,5 GPa eingesetzt werden.

Die weitere Umsetzung der Verbindungen der Formel (7) zu den Verbindungen der Formel (8) erfolgt wie in den Beispielen beschrieben oder nach bekannten Methoden der organischen Chemie.

Um ausgehend von den Verbindungen der Formel (3), (4) oder (8) zu den Verbindungen der Formel (III) zu gelangen, sind weitere Reaktionen erforderlich.

Beispielsweise genannt seien die Hydrolyse eines Esters zur Carbonsäure, die Reduktion von Carbonylgruppen, zum Beispiel von Estern, zu Aldehyden oder Alkoholen oder von Lactamgruppen zu den Pyrrolidinen, die Überführung einer Hydroxyfunktion in eine Aminofunktion, die Überführung einer Carboxylfunktion oder eines ihrer Derivate unter Abbau um ein Kohlenstoffatom in eine Aminfunktion, die reduktive Aminierung eines Aldehyds mit einer im Molekül vorhandenen Aminfunktion, die reduktive Aminierung einer im Molekül vorhandenen Aldehydfunktion mit einem Amin, die Einführung von Schutzgruppen, die Abspaltung der Schutzgruppe am Pyrrolidinstickstoff derart, daß im Molekül eventuell vorhandene weitere Schutzgruppen erhalten bleiben.

Diese Umsetzungen erfolgen wie in den Beispielen beschrieben oder nach in der organischen Chemie üblichen Methoden.

Die weitere Umsetzung der Verbindungen der Formel (3), (4) oder (8) zu den Verbindungen der Formel (III) können beispielsweise durch die nachfolgenden Formelschemata erläutert werden: 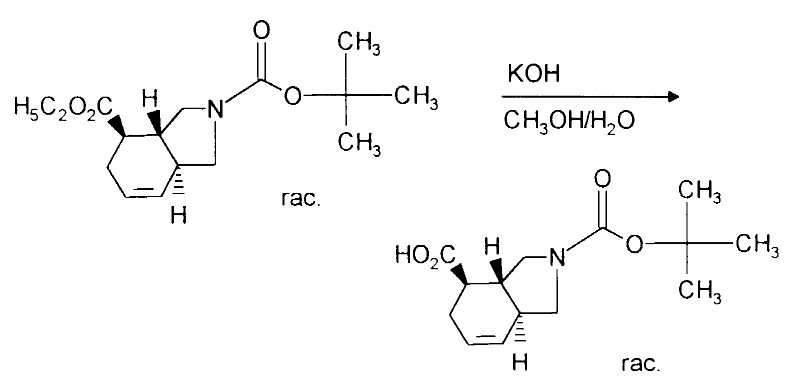 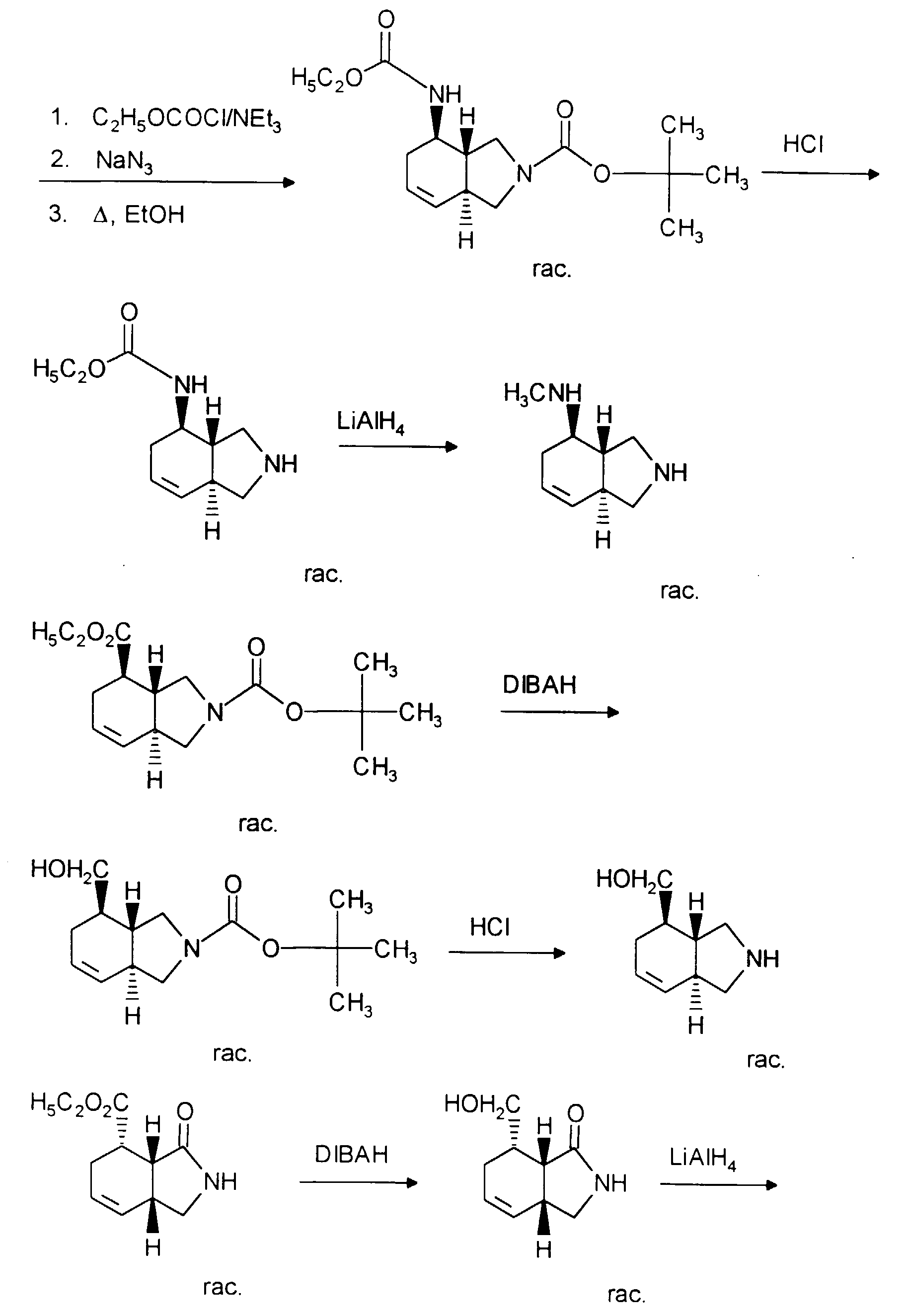 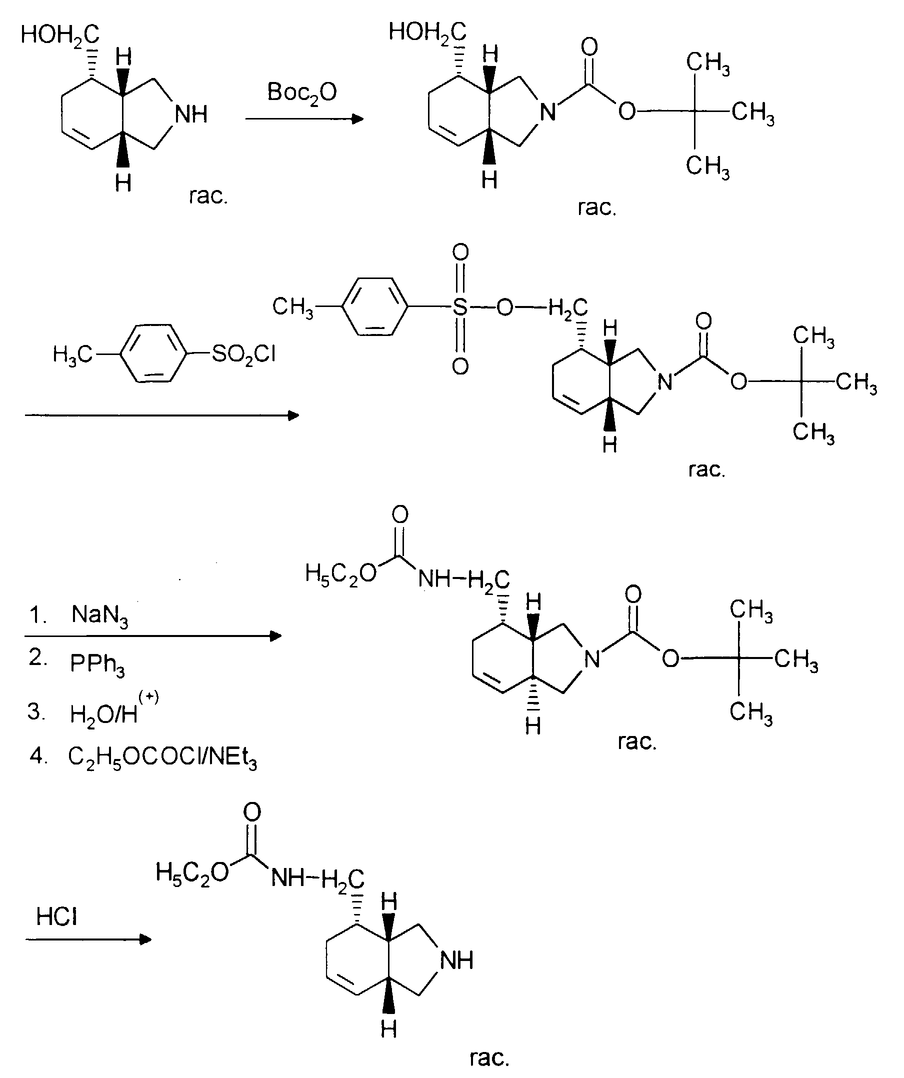 Die Ausgangsstoffe der Formeln (1), (2), (5) und (6) sind bekannt oder können nach bekannten Methoden der organischen Chemie hergestellt werden.

Die Umsetzung von Verbindungen der Formel (II) Verbindungen mit der Formel (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebindemittel können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen 1 bar und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäue, Embonsäure, Glutaminsäure oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Mittel in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen unterhalb von Konzentrationen ähnlicher Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Acinetobacter, Micrococcus luteus und Enterococcus faecalis beobachtet werden.

Auch gegenüber Bakterien, die gegenüber vergleichbaren Substanzen als weniger empfindlich eingestuft werden, insbesondere resistenten Staphylococcus aureus und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die Wirkstoffe eignen sich bei günstiger Warmblütertoxizität bevorzugt zur Bekämpfung von bakteriellen Erkrankungen die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien sowie gegen resistente und normal sensible Stämme wirksam. Durch die Bekämpfung der bakteriellen Erkrankungen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutz- und Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z. B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabream (Pagurus major), Seabass (Dicentrarchus labrax), Grey mullet (Mugilus cephalus), Pompano, Gilthread seabream (Sparus auratus), Tilapia spp., Chichliden-Arten wie z. B. Plagioscion, Channel catfish. Besonders geeignet sind die erfindungsgemäßen Mittel zur Behandlung von Fischbrut, z. B. Karpfen von 2 - 4 cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate,
Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie
Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs-und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 100 ppm, vorzugsweise 0,5 bis 50 ppm des Wirkstoffs in Kombination mit einem geeigneten eßbaren Material.

Ein solches Futter- und Nahrungsmittel kann sowohl für Heilzwecke als auch für prophylaktische Zwecke verwendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Täger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann dann dem vollständigen Futtermittel vor seiner Verfütterung an die Tiere zugesetzt werden.

Die minimalen Hemmkonzentrationen (MHK) der erfindungsgemäßen Verbindungen wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen aufgeführt.

**Tabelle**

| **MHK-Werte** | | | | | |
|---|---|---|---|---|---|
| Spezies | Stamm | Beispiel Nr. | | | |
| | | 4 | 5 | 7 | 11 |
| E. coli | Neumann | 0.03 | ≤0.015 | ≤0.015 | ≤0.015 |
| | ATCC 25922 | 0.03 | ≤0.015 | ≤0.015 | ≤0.015 |
| Klebsiella pneumoniae | 8085 | 0.06 | 0.03 | 0.03 | ≤0.015 |
| | 63 | 0.06 | 0.03 | 0.03 | ≤0.015 |
| Providencia sp. | 12012 | 0.06 | ≤0.015 | 0.03 | ≤0.015 |
| | 12052 | 4 | 2 | 2 | 16 |
| Micrococcus luteus | 9341 | ≤0.015 | ≤0.015 | ≤0.015 | 0,5 |
| Staphylococcus aureus | ICB 25701 | 0.5 | 0.125 | 0.25 | 0.5 |
| | ATCC 29213 | ≤0.015 | ≤0.015 | ≤0.015 | ≤0.015 |
| | 133 | ≤0.015 | ≤0.015 | ≤0.015 | ≤0.015 |
| | ICB 25768 | 1 | 0.25 | 0.5 | 16 |
| Enterococcus faecalis | 27101 | 0.06 | 0.03 | ≤0.015 | ≤0.015 |
| | 9790 | 0.06 | 0.03 | 0.03 | ≤0.015 |
| Acinetobacter | 14068 | ≤0.015 | ≤0.015 | ≤0.015 | ≤0.015 |

### Herstellung der Wirkstoffe

### Beispiel 1

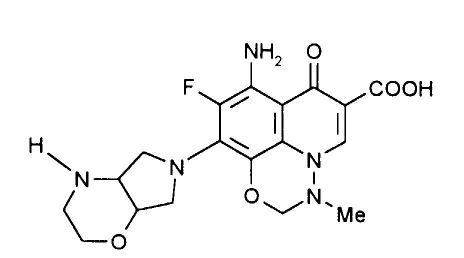

### 8-Amino-9-fluor-3-methyl-10-(2-oxa-5,8-diazabicyclo[4.3.0]nonan-8-yl)-7-oxo-2,3-dihydro-7H-pyrido[1,2,3-d,e][1,3,4]benzoxadiazin-6-carbonsäure

100 mg (0.336 mmol) 8-Amino-9,10-difluor-3-methyl-7-oxo-2,3-dihydro-7H-pyrido[1,2,3-d,e][1,3,4]-benzoxadiazin-6-carbonsäure werden mit 86 mg (0.671 mmol) 2-Oxa-5,8-diazabicyclo[4.3.0]nonan in 3 ml Pyridin sechs Stunden unter Argon auf 110°C erwärmt. Die Mischung wird im Hochvakuum eingeengt, der Rückstand aus Ethanol umkristallisiert und getrocknet.
Ausbeute: 102 mg (72 % der Theorie)
Schmelzpunkt: 295-296°C

### Beispiel 2

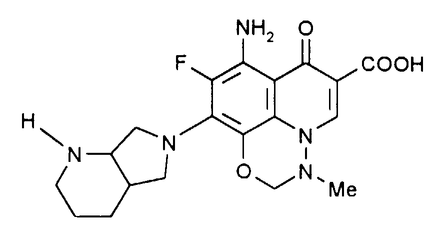

### 8-Amino-10-(2,8-diazabicyclo[4.3.0]nonan-8-yl)-9-fluor-3-methyl-7-oxo-2,3-dihydro-7H-pyrido[1,2,3-d,e][1,3,4]benzoxadiazin-6-carbonsäure

100 mg (0.336 mmol) 8-Amino-9,10-difluor-3-methyl-7-oxo-2,3-dihydro-7H-pyrido[1,2,3-d,e][1,3,4]benzoxadiazin-6-carbonsäure werden mit 127 mg (1.01 mmol) 2,8-Diazabicyclo[4.3.0]nonan in 3 ml Dimethylsulfoxid (DMSO) zwei Stunden unter Argon auf 120°C erwärmt. Die Mischung wird im Hochvakuum eingeengt, der Rückstand aus Ethanol umkristallisiert und getrocknet.
Ausbeute: 99 mg (73 % der Theorie)
Schmelzpunkt: 284°C (unter Zersetzung)

### Beispiel 3

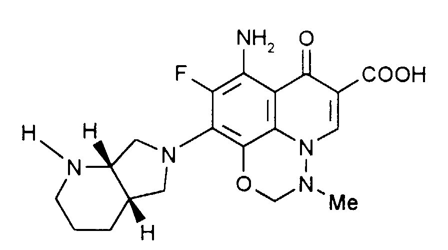

### 8-Amino-10-((1S,6S)-2,8-diazabicyclo[4.3.0]nonan-8-yl)-9-fluor-3-methyl-7-oxo-2,3-dihydro-7H-pyrido[1,2,3-d,e][1,3,4]benzoxadiazin-6-carbonsäure

100 mg (0.336 mmol) 8-Amino-9,10-difluor-3-methyl-7-oxo-2,3-dihydro-7H-pyrido[1,2,3-d,e][1,3,4]benzoxadiazin-6-carbonsäure werden mit 85 mg (0.674 mmol) (1S,6S)-2,8-Diazabicyclo[4.3.0]nonan in 3 ml Dimethylsulfoxid (DMSO) zwei Stunden unter Argon auf 130°C erwärmt. Die Mischung wird im Hochvakuum eingeengt, der Rückstand aus Ethanol umkristallisiert und getrocknet.
Ausbeute: 101 mg (74 % der Theorie)
Schmelzpunkt: >300°C (unter Zersetzung)

### Beispiel 4

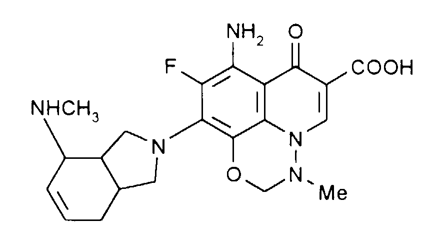

### 8-Amino-9-fluor-3-methyl-10-(2-methylamino-8-azabicyclo[4.3.0]non-3-en-8-yl)-7-oxo-2,3-dihydro-7H-pyrido[1,2,3-de][1,3,4]benzoxadiazin-6-carbonsäure

200 mg (0,673 mmol) 8-Amino-9,10-difluor-3-methyl-7-oxo-2,3-dihydro-7H-pyrido[1,2,3-de][1,3,4]-benzoxadiazin-6-carbonsäure werden mit 200 mg (1,31 mmol) 2-Methylamino-8-azabicyclo[4.3.0]non-3-en in 6 ml DMSO drei Stunden unter Argon auf 130°C erwärmt. Die Mischung wird im Hochvakuum eingeengt, der Rückstand aus Ethanol umkristallisiert und getrocknet.
Ausbeute: 274 mg (95 % der Theorie)
Schmelzpunkt: 256°C

### Beispiel 5

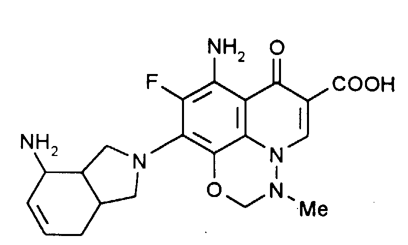

### 8-Amino-10-(2-amino-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

200 mg (0,673 mmol) 8-Amino-9,10-difluor-3-methyl-7-oxo-2,3-dihydro-7H-pyrido[1,2,3-de][1,3,4]-benzoxadiazin-6-carbonsäure werden mit 186 mg (1,35 mmol) 2-Amino-8-azabicyclo[4.3.0]non-3-en in 6 ml DMSO zwei Stunden unter Argon auf 120°C erwärmt. Die Mischung wird im Hochvakuum eingeengt, der Rückstand aus Ethanol umkristallisiert und getrocknet.
Ausbeute: 193 mg (69 % der Theorie)
Schmelzpunkt: 274-275°C

### Beispiel 6

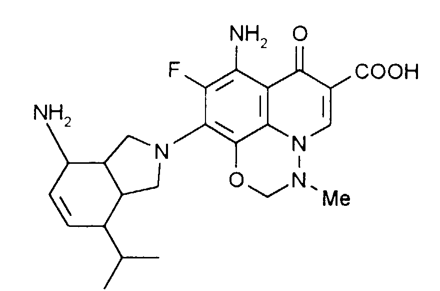

### 8-Amino-10-(2-amino-5-isopropyl-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

100 mg (0,336 mmol) 8-Amino-9,10-difluor-3-methyl-7-oxo-2,3-dihydro-7H-pyrido[1,2,3-de][1,3,4]-benzoxadiazin-6-carbonsäure werden mit 121 mg (6.671 mmol) 2-Amino-5-isopropyl-8-azabicyclo[4.3.0]non-3-en in 3 ml DMSO zwei Stunden unter Argon auf 130°C erwärmt. Die Mischung wird im Hochvakuum eingeengt, der Rückstand aus Ethanol umkristallisiert und getrocknet.
Ausbeute: 69 mg (45 % der Theorie)
Schmelzpunkt: 227°C

### Beispiel 7

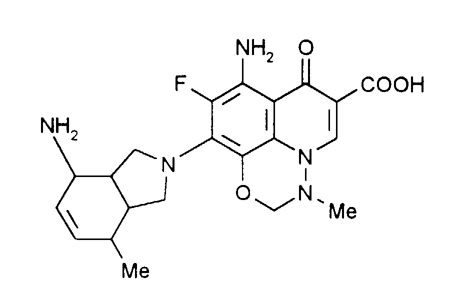

### 8-Amino-10-(2-amino-5-methyl-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

200 mg (0,673 mmol) 8-Amino-9,10-Difluor-3-methyl-7-oxo-2,3-dihydro-7H-pyrido[1,2,3-d,e][1,3,4]benzoxadiazin-6-carbonsäure werden mit 205 mg (1.35 mmol) 2-Amino-5-methyl-8-azabicyclo[4.3.0]non-3-en in 6 ml Pyridin fünf Stunden unter Argon auf 100°C erwärmt. Die Mischung wird im Hochvakuum eingeengt, der Rückstand aus Ethanol umkristallisiert und getrocknet.
Ausbeute: 233 mg (81 % der Theorie)
Schmelzpunkt: 225°C

### Beispiel 8

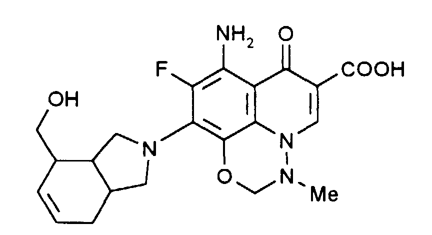

### 8-Amino-10-(2-hydroxymethyl-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

100 mg (0,336 mmol) 8-Amino-9,10-difluor-3-methyl-7-oxo-2,3-dihydro-7H-pyrido[1,2,3-d,e][1,3,4]benzoxadiazin-6-carbonsäure werden mit 103 mg (0,672 mmol) 2-Hydroxymethyl-8-azabicyclo[4.3.0]non-3-en in 3 ml DMSO drei Stunden unter Argon auf 130°C erwärmt. Die Mischung wird im Hochvakuum eingeengt, der Rückstand aus Ethanol umkristallisiert und getrocknet.
Ausbeute: 105 mg (73 % der Theorie)
Schmelzpunkt: 278-280°C

### Beispiel 9

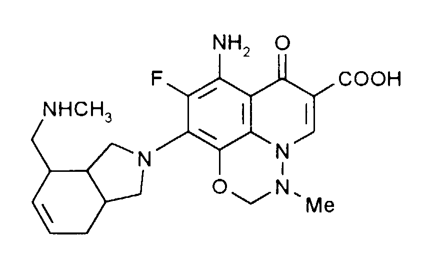

### 8-Amino-10-(2-methylaminomethyl-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

100 mg (0,336 mmol) 8-Amino-9,10-difluor-3-methyl-7-oxo-2,3-dihydro-7H-pyrido[1,2,3-d,e][1,3,4]benzoxadiazin-6-carbonsäure werden mit 112 mg (0,673 mmol) 2-Methylaminomethyl-8-azabicyclo[4.3.0]non-3-en in 3 ml Pyridin vierzehn Stunden unter Argon auf 110°C erwärmt. Die Mischung wird im Hochvakuum eingeengt, der Rückstand aus Ethanol umkristallisiert und getrocknet.
Ausbeute: 136 mg (91 % der Theorie)
Schmelzpunkt: 250°C

### Beispiel 10

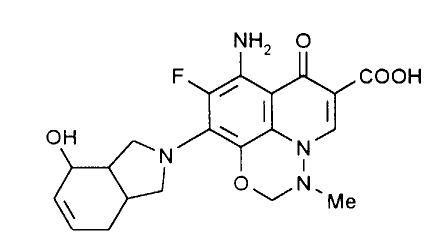

### 8-Amino-10-(2-hydroxy-8-azabicyclo[4.3.0]non-3-en-8-yl)-9-fluor-3-methyl-7-oxo-2,3-dihydro-7H-pyrido[1,2,3-d,e][1,3,4]benzoxadiazin-6-carbonsäure

100 mg (0,336 mmol) 8-Amino-9,10-Difluor-3-methyl-7-oxo-2,3-dihydro-7H-pyrido[1,2,3-d,e][1,3,4]benzoxadiazin-6-carbonsäure werden mit 94 mg (0,675 mmol) 2-Hydroxy-8-azabicyclo[4.3.0]non-3-en in 3 ml DMSO vier Stunden unter Argon auf 120°C erwärmt. Die Mischung wird im Hochvakuum eingeengt, der Rückstand aus Ethanol umkristallisiert und getrocknet.
Ausbeute: 83 mg (59 % der Theorie)
Schmelzpunkt: >300°C (unter Zersetzung)

### Beispiel 11

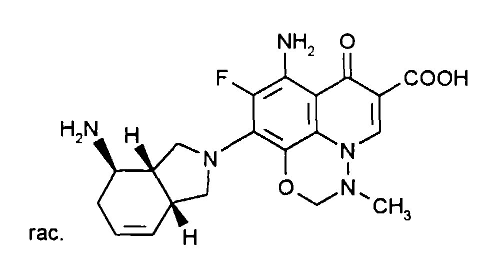

### 8-Amino-10-((1SR,2RS,6RS)-2-amino-8-azabicyclo[4.3.0]non-4-en-8-yl)-9-fluor-3-methyl-7-oxo-2,3-dihydro-7H-pyrido[1,2,3-d,e][1,3,4]benzoxadiazin-6-carbonsäure

90 mg (0.303 mmol) 8-Amino-9,10-difluor-3-methyl-7-oxo-2,3-dihydro-7H-pyrido-[1,2,3-d,e][1,3,4]benzoxadiazin-6-carbonsäure werden mit 54 mg (0.391 mmol) (1SR,2RS,6RS)-2-Amino-8-azabicyclo[4.3.0]non-4-en in 10 ml Pyridin sieben Stunden unter Stickstoff auf 60°C erwärmt. Die Mischung wird im Hochvakkuum eingeengt, der Rückstand aus Methanol umkristallisiert und getrocknet.
Ausbeute: 120 mg (96 % der Theorie)
Schmelzpunkt: >300°C

### Beispiel 12

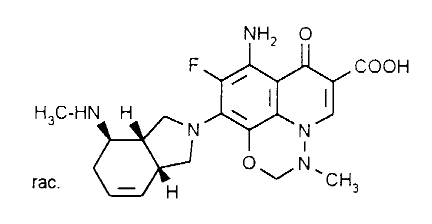

### 8-Amino-9-fluor-3-methyl-10-((1SR,2RS,6RS)-2-methylamino-8-azabicyclo[4.3.0]non-4-en-8-yl)-7-oxo-2,3-dihydro-7H-pyrido[1,2,3-d,e][1,3,4]benzoxadiazin-6-carbonsäure

445 mg (1.5 mmol) 8-Amino-9,10-difluor-3-methyl-7-oxo-2,3-dihydro-7H-pyrido[1,2,3-d,e][1,3,4]benzoxadiazin-6-carbonsäure und 295 mg (1.95 mmol) (1SR, 2RS, 6RS)-2-Methylamino-8-azabicyclo[4.3.0]non-4-en (Produkt aus Beispiel N) werden umgesetzt, wie im Beispiel 11 beschrieben.
Ausbeute: 540 mg (84 % der Theorie).
Schmelzpunkt: 292 °C (Zers.).

### Beispiel 13

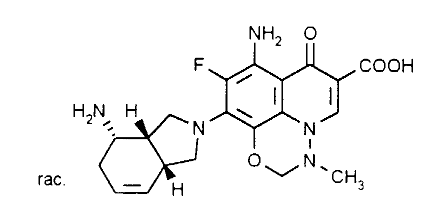

### 8-Amino-10-((1SR, 2SR, 6RS)-2-amino-8-azabicyclo[4.3.0]non-4-en-8-yl)-9-fluor-3-methyl-7-oxo-2,3-dihydro-7H-pyrido[1,2,3-d,e][1,3,4]benzoxadiazin-6-carbonsäure

445 mg (1.5 mmol) 8-Amino-9,10-difluor-3-methyl-7-oxo-2,3-dihydro-7H-pyrido[1,2,3-d,e][1,3,4]benzoxadiazin-6-carbonsäure und 270 mg (1.95 mmol) (1SR, 2SR, 6RS)-2-Amino-8-azabicyclo[4.3.0]non-4-en werden umgesetzt, wie im Beispiel 11 beschrieben.
Ausbeute: 490 mg (79 % der Theorie).
Schmelzpunkt: 246 °C (Zers.).

### Beispiel 14

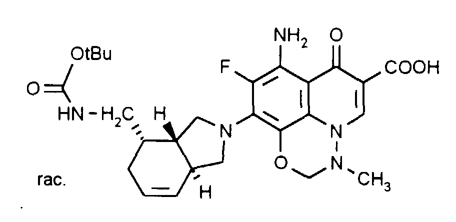

### 8-Amino-9-fluor-3-methyl-7-oxo-10-((1SR, 2SR, 6SR)-2-(tert.-butyloxycarbonyl)aminomethyl-8-azabicyclo[4.3.0]non-4-en-8-yl)-2,3-dihydro-7H-pyrido[1,2,3-d,e][1,3,4]benzoxadiazin-6-carbonsäure

370 mg (1.25 mmol) 8-Amino-9,10-difluor-3-methyl-7-oxo-2,3-dihydro-7H-pyrido[1,2,3-d,e][1,3,4]benzoxadiazin-6-carbonsäure und 410 mg (1.6 mmol) (1SR, 2SR, 6RS)-2-(*tert*.-Butyloxycarbonyl)aminomethyl-8-azabicyclo[4.3.0]non-4-en werden umgesetzt, wie im Beispiel 11 beschrieben.
Ausbeute: 470 mg (71 % der Theorie).
Schmelzpunkt: 227 °C (Zers.).

### Beispiel 15

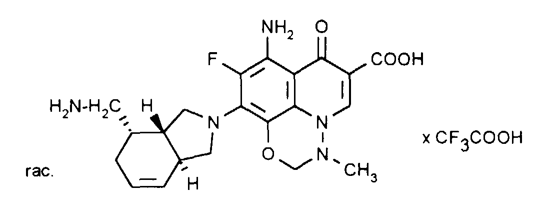

### 8-Amino-10-((1SR, 2SR, 6SR)-2-aminomethyl-8-azabicyclo[4.3.0]non-4-en-8-yl)-9-fluor-3-methyl-7-oxo-2,3-dihydro-7H-pyrido[1,2,3-d,e][1,3,4]benzoxadiazin-6-carbonsäure-Trifluoressigsäuresalz

400 mg (0.75 mmol) des Produktes aus Beispiel 14 wird in 10 ml eisgekühlter Trifluoressigsäure suspendiert. Die Mischung wird innerhalb einer Stunde auf Raumtemperatur erwärmt, wobei eine klare Lösung entsteht. Nach Zugabe von Methanol wird das ausgefallene Produkt abgesaugt und im Trockenschrank bei 50 °C getrocknet.
Ausbeute: 400 mg (quantitativ).
Schmelzpunkt: 235 °C (Zers.).

### Beispiel 16

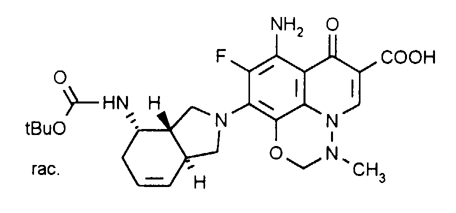

### 8-Amino-2-(tert.-butyloxycarbonyl)amino-8-azabicyclo[4.3.0]non-4-en-8-yl)-9-fluor-3-methyl-7-oxo-10-((1SR, 2SR, 6SR)-2,3-dihydro-7H-pyrido[1,2,3-d,e][1,3,4]benzoxadiazin-6-carbonsäure

595 mg (2.0 mmol) 8-Amino-9,10-difluor-3-methyl-7-oxo-2,3-dihydro-7H-pyrido[1,2,3-d,e][1,3,4]benzoxadiazin-6-carbonsäure und 620 mg (2.6 mmol) (1SR, 2SR, 6RS)-2-(*tert*.-Butyloxycarbonyl)amino-8-azabicyclo[4.3.0]non-4-en werden umgesetzt, wie im Beispiel 11 beschrieben.
Ausbeute: 850 mg (82 % der Theorie).
Schmelzpunkt: 259 °C (Zers.).

### Beispiel 17

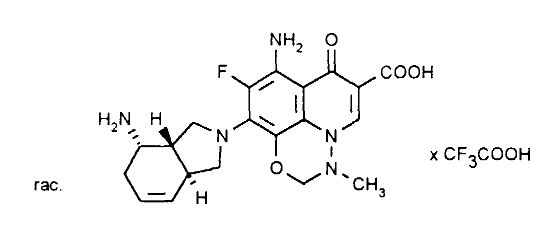

### 8-Amino-10-((1SR, 2SR, 6SR)-2-amino-8-azabicyclo[4.3.0]non-4-en-8-yl)-9-fluor-3-methyl-7-oxo-2,3-dihydro-7H-pyrido[1,2,3-d,e][1,3,4]benzoxadiazin-6-carbonsäure-Trifluoressig-säuresalz

Das Produkt aus Beispiel 16 (700 mg; 1.3 mmol) wird mit Trifluoressigsäure umgesetzt, wie im Beispiel 16 beschrieben.
Ausbeute: 600 mg (90 % der Theorie).
Schmelzpunkt: 258 °C (Zers.).

### Beispiel 18

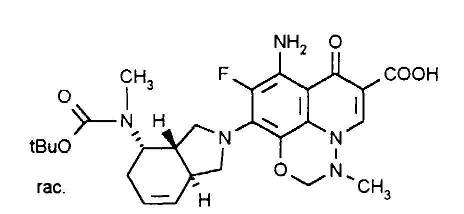

### 8-Amino-2-(N-tert.-butyloxycarbonyl-N-methyl)amino-8-azabicyclo[4.3.0]non-4-en-8-yl)-9-fluor-3-methyl-7-oxo-10-((1SR, 2SR, 6SR)-2,3-dihydro-7H-pyrido[1,2,3-d,e][1,3,4]benzoxadiazin-6-carbonsäure

595 mg (2.3 mmol) 8-Amino-9,10-difluor-3-methyl-7-oxo-2,3-dihydro-7H-pyrido[1,2,3-d,e][1,3,4]benzoxadiazin-6-carbonsäure und 755 mg (3.0 mmol) (1SR, 2SR, 6RS)-2-(N-*tert*.-Butyloxycarbonyl-N-methyl)amino-8-azabicyclo[4.3.0]non-4-en werden umgesetzt, wie im Beispiel 11 beschrieben.
Ausbeute: 1,05 g (66 % der Theorie).
Schmelzpunkt: 255 °C (Zers.).

### Beispiel 19

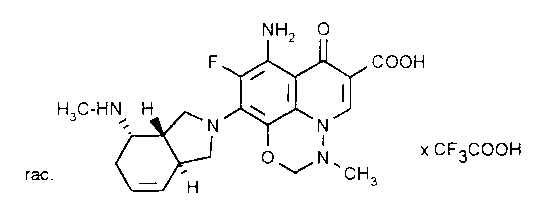

### Das Trifluoressigsäuresalz aus 8-Amino-9-fluor-3-methyl-10-((1SR, 2SR, 6SR)-2-methylamino-8-azabicyclo[4.3.0]non-4-en-8-yl)-7-oxo-2,3-dihydro-7H-pyrido[1,2,3-d,e][1,3,4]benzoxadiazin-6-carbonsäure

Das Produkt aus Beispiel 18 (1,0 g; 1.9 mmol) wird mit Trifluoressigsäure umgesetzt, wie im Beispiel 15 beschrieben.
Ausbeute: 1,0 g (97 % der Theorie).
Schmelzpunkt: 290 °C (Zers.).

### Herstellung der Zwischenprodukte:

### Beispiel A:

### 8-Azabicyclo[4.3.0]non-2-en

### A.1. (E)-1-Brom-2,4-pentadien

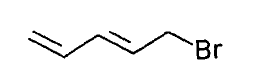

84 g (1,0 mol) 1,4-Pentadien-3-ol bei 0 °C vorlegen. Unter Rühren 150 ml (≈ 1,3 mol) 48 %-ige wäßrige Bromwasserstoffsäure so zutropfen, daß die Innentemperatur 5°C nicht überschreitet. Nach vollständiger Zugabe 1 h bei Raumtemperatur nachrühren. Die organische Phase wird abgetrennt und ohne Reinigung weiter umgesetzt.
Ausbeute: 107-129 g (73-88 % der Theorie)

### A.2. (E)-1-(2-Propenylamino)-2,4-pentadien

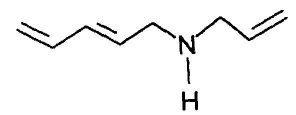

228 g (4,0 mol) 1-Amino-2-propen vorlegen. Unter Rühren 58,8 g (0,4 mol) (E)-1-Brom-2,4-pentadien (Titelverbindung aus Beispiel A.1.) zutropfen. Durch Kühlung die Innentemperatur im Bereich von 20-30°C halten. 5 h bei Raumtemperatur rühren. Ansatz bei 150 mbar einengen. 20 g (0,5 mol) Natriumhydroxid in 200 ml Wasser gelöst zugeben, mit zweimal je 100 ml Methylenchlorid extrahieren, mit Natriumsulfat trocknen, 0,1 g 4-Hydroxyanisol zusetzen, einengen, bei 40 mbar destillieren. Zur Stabilisierung werden dem Destillat 10-20 ppm 4-Hydroxyanisol zugesetzt.
Ausbeute: 33-35 g (67-72 % der Theorie)
Siedepunkt: 77-82 °C bei 40 mbar
¹H-NMR (CDCl₃): δ = 6,07-6,48 (m, 2H); 5,64-6,07 (m, 2H); 5,00-5,27 (m, 4H); 3,19-3,36 ppm (m, 4H).

### A.3. N-[(E)-2,4-Pentadienyl]-N-(2-propenyl)-acetamid

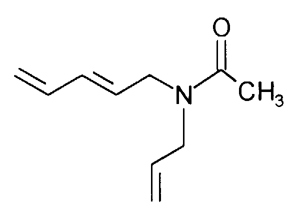

24,6 g (0,2 mol) (E)-1-(2-propenylamino)-2,4-pentadien (Titelverbindung aus Beispiel A.2.) vorlegen, 22,4 g Essigsäureanhydrid zutropfen und über Nacht bei Raumtemperatur rühren. Einengen und als Rohprodukt weiter umsetzen.

### A.4. 8-Acetyl-8-azabicyclo[4.3.0]non-2-en

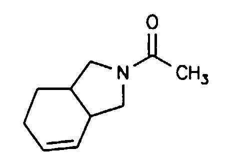

33,1 g (0,2 mol) N-[(E)-2,4-Pentadienyl]-N-(2-propenyl)-acetamid (Titelverbindung aus Beispiel A.3.) in 200 ml Xylol lösen, 15 min einen kräftigen Stickstoffstrom durchleiten, 0,1 g 4-Hydroxyanisol zusetzen, dann über Nacht zum Rückfluß erhitzen. Einengen, im Hochvakuum destillieren.
Ausbeute: 23,1 g (70 % der Theorie bezogen auf die Titelverbindung aus Beispiel A.2.)
Siedepunkt: 88-93 °C bei 0,05 mbar

### A. 5.8-Azabicyclo[4.3.0]non-2-en

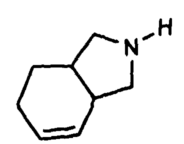

16,5 g (0,1 mol) 8-Acetyl-8-azabicyclo[4.3.0]non-2-en (Titelverbindung aus Beispiel A.4.) in einer Mischung aus 100 ml 45 %-iger Natronlauge, 50 ml Wasser und 100 ml 1,2-Ethandiol 3 h zum Rückfluß erhitzen. Nach Abkühlung viermal mit je 50 ml Diethylether extrahieren. Vereinigte organische Phasen mit Natriumsulfat trocknen, im Hochvakuum destillieren.
Ausbeute: 6,6 g (54 % der Theorie)
Siedepunkt: 36-44 °C bei 0,35 mbar
¹H-NMR (CDCl₃): δ = 5,79 (m, 1H); 5,74 (m, 1H) ; 3,02-3,17 (m, 2H); 2,47-2,72 (m, 2H); 2,06-2,30 (m, 2H); 1,91-2,06 (m, 2H); 1,68 (m, 1H); 1,45 ppm (m, 1H).

### Beispiel B:

### (1RS,2RS,6SR)-8-Azabicyclo[4.3.0]non-4-en-2-carbonsäureethylester (Diastereomer A) und

### (1RS,2RS,6RS)-8-Azabicyclo[4.3.0]non-4-en-2-carbonsäureethylester (Diastereomer B)

### B.1. N-[(E)-2,4-Pentadienyl]-phthalimid

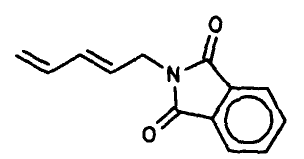

185 g (1,0 mol) Kaliumphthalimid in 800 ml DMF vorlegen. Unter Rühren 147 g (1,0 mol) (E)-1-Brom-2,4-pentadien (Titelverbindung aus Beispiel A.1.) zutropfen, dabei die Innentemperatur durch Kühlen unter 30°C halten. Über Nacht bei Raumtemperatur rühren. Anschließend den Ansatz unter Rühren auf 1,6 l Eiswasser gießen, den Niederschlag absaugen, mit Wasser waschen, bei Raumtemperatur bis zum Erreichen der Gewichtskonstanz trocknen.
Ausbeute: 177-200 g (83-94 % der Theorie)
Schmelzpunkt: 118-121°C (Probe aus Ethanol umkrist.)
¹H-NMR (CDCl₃): δ = 7,85 und 7,72 (m, 4H, Aryl-H); 6,2-6,4 (m, 2H, H an C-3 und C-4); 5,75 (dt, 1H, H an C-2, J = 14 und 6 Hz); 5,20 (d, 1H, Hₐ an C-5, J = 15 Hz); 5,10 (d, 1H, H_{b} an C-5, J = 8 Hz); 4,33 ppm (d, 2H, H an C-1, J = 6 Hz).

### B.2. (E)-1-Amino-2,4-pentadien

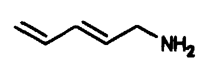

In einer 2l-Destillationsapparatur mit 10 cm Vigreuxkolonne werden 400 g Bis-(2-aminoethyl)-amin und 213 g (1.0 mol) N-[(E)-2,4-Pentadienyl]-phthalimid (Titelverbindung aus Beispiel B.1.) vorgelegt und bei 60 mbar zum Sieden erhitzt. Das Produkt destilliert im Bereich von 45-60 °C bei 60 mbar. Zur Stabilisierung werden dem Destillat 10-20 ppm 4-Hydroxyanisol zugesetzt.
Ausbeute: 71-80 g (86-96 % der Theorie)

### B.3. (E)-4-[(E)-2,4-Pentadienylamino]-2-butensäureethylester

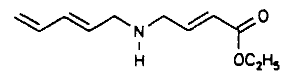

41,6 g (0,5 mol) (E)-1-Amino-2,4-pentadien (Titelverbindung aus Beispiel B.2.) und 50,6 g (0,5 mol) Triethylamin in 250 ml THF bei 0 °C vorlegen und 96,5 g (0,5 mol) (E)-4-Brom-2-butensäureethylester in 250 ml THF gelöst zutropfen. Innentemperatur durch Eiskühlung unter 5°C halten. 5 h bei 0 °C anschließend über Nacht bei Raumtemperatur rühren. 500 ml MTBE, dann 500 ml 1M Natronlauge zugeben, schütteln, Phasentrennung, wäßrige Phase einmal mit 100 ml MTBE extrahieren, vereinigte organische Phasen mit Natriumsulfat trocknen, 100 ml Toluol und 0,1 g 4-Hydroxyanisol zusetzen, einengen (dabei Temperaturen über 40°C vermeiden). Rückstand an 1 kg Kieselgel (63-200 µm) mit Cyclohexan/Aceton 2:1 säulenchromatographisch reinigen. Vor dem Einengen erneut 0,1 g 4-Hydroxyanisol zusetzen und beim Einengen Temperaturen über 40°C vermeiden.
Ausbeute: 52,7-58,6 g (54-60 % der Theorie) gelbliches Öl
Rf = 0,24
¹H-NMR (CDCl₃): δ = 6,99 (dt, 1H, J = 15 und 5,5 Hz); 6,1-6,45 (m, 2H); 5,98 (d, 1H, J = 15 Hz); 5,75 (dt, 1H, 1 = 15 und 6,5 Hz), 5,18 (d, 1H, J = 15 Hz); 5,06 (d, 1H, J = 10 Hz); 4,19 (q, 2H); 3,42 (dd, 2H); 3,31 (d, 2H); 1,29 ppm (t, 3H).

### B.4. (1RS,2RS,6SR)-8-tert.-Butyloxycarbonyl-8-azabicyclo[4.3.0]non-4-en-2-carbonsäureethylester (Diastereomer A) und

### (1RS,2RS,6RS)-8-tert.-Butyloxycarbonyl-8-azabicyclo[4.3.0]non-4-en-2-carbonsäureethylester (Diastereomer B)

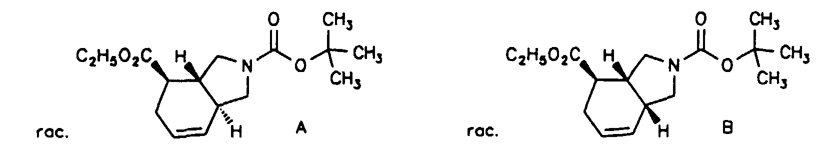

97,5 g (0,5 mol) (E)-4-[(E)-2,4-Pentadienylamino]-2-butensäureethylester (Titelverbindung aus Beispiel B.3.) in 250 ml Toluol gelöst vorlegen. 114,5 g (0,525 mol) Di-tert.-butyl-dicarbonat in 250 ml Toluol gelöst zutropfen, über Nacht bei Raumtemperatur rühren. Anschließend 15 min einen kräftigen Stickstoffstrom durchleiten, 0,1 g 4-Hydroxyanisol zusetzen, dann 6 h zum Rückfluß erhitzen. Einengen, Rückstand an 1 kg Kieselgel (63-200 µm) mit Cyclohexan/Aceton 8:1 säulenchromatographisch reinigen.
Ausbeute: 109-134 g (74-91 % der Theorie) gelbliches Öl; Gemisch aus zwei Diastereomeren A und B im Verhältnis A:B = 4:1
Rf = 0,25
¹H-NMR (Cl₂DC-CDCl₂; 80°C): δ = 5,77 (m, 1H(A) und 1H(B)); 5,68 (m, 1H(A) und 1H(B)); 4,14 (m, 2H(A) und 2H(B)); 3,65 (m, 2H(A) und 1H(B)); 3,48 (dd, 1H(B)); 3,27 (dd, 1H(B)); 3,00 (m, 1H(A) und 1H(B)); 2,85 (dd, 1H(A)); 2,76 (m, 1H(B)); 2,60 (m, 1H(A)); 2,25-2,55 (m, 3H(A) und 4H(B)); 1,93 (m, 1H(A)); 1,51 (s, 9H(B)); 1,44 (s, 9H(A)); 1,25 ppm (t, 3H(A) und 3H(B)).

### B.5. (1RS,2RS,6SR)-8-Azabicyclo[4.3.0]non-4-en-2-carbonsäureethylester (Diastereomer A) und

### (1RS,2RS,6RS)-8-Azabicyclo[4.3.0]non-4-en-2-carbonsäureethylester (Diastereomer B)

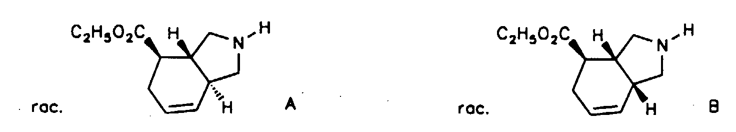

6,0 g (20 mmol) der Titelverbindung aus Beispiel B.4. in 20 ml Dioxan vorlegen. 20 ml konz. Salzsäure unter Kühlung so zutropfen, daß die Innentemperatur 30°C nicht übersteigt. Nach vollständiger Zugabe 10 min nachrühren. 40 ml Methylenchlorid zugeben und 40 ml 20 %-ige eisgekühlte Natronlauge unter Eiskühlung zutropfen. Organische Phase abtrennen, wäßrige Phase einmal mit Methylenchlorid extrahieren, vereinigte organische Phasen mit Natriumsulfat trocknen, einengen. 3,0 g Rohprodukt an 100 g Kieselgel (63-200 µm) mit Cyclohexan/Ethanol/17%-iges wäßriges Ammoniak (1:2:0,1) säulenchromatographisch reinigen.
- Ausbeute:: 0,8 g Diastereomer A und
0,8 g Diastereomer B
- Rf =: 0,79 Titelverbindung aus Beispiel B.4.
0,21 Diastereomer B
0,11 Diastereomer A
¹H-NMR (CDCl₃):
Diastereomer A: δ = 5,83 (d, 1H); 5,69 (m, 1H); 4,15 (q, 2H); 3,21-3,38 (m, 2H); 2,52-2,89 (m, 3H); 2,21-2,52 (m, 3H); 1,95 (m, 1H); 1,28 ppm (t, 3H).
Diastereomer B: δ = 5,64-5,87 (m, 2H); 4,16 (q, 2H); 3,14-3,33 (m, 2H); 2,82 (dd, 1H); 2,15-2,74 (m, 6H); 1,28 ppm (t, 3H).

### Beispiel C:

### (1SR,2RS,6SR)-2-Ethyloxycarbonylamino-8-azabicyclo[4.3.0]non-4-en

### C.1. (1RS,2RS,6SR)-8-tert.-Butyloxycarbonyl-8-azabicyclo-[4.3.0]non-4-en-2-carbonsäure

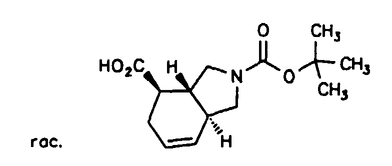

30,8 g (0,55 mol) Kaliumhydroxid in 500 ml Wasser gelöst vorlegen. 147,7 g (0,5 mol) der Titelverbindung aus Beispiel B.4. in 500 ml Methanol gelöst. zugeben und 8 h bei 60°C unter einer Stickstoffatmosphäre rühren. Nach Abkühlung Reaktionslösung mit 500 ml Wasser verdünnen und unter Rühren 125 ml Essigsäure langsam zugießen. Nach vollständiger Zugabe 30 min im Eisbad stehen lassen, Niederschlag absaugen, mit Wasser nachwaschen, bei 50°C bis zur Gewichtskonstanz trocknen.
Ausbeute: 84-98 g (63-73 % der Theorie)
Schmelzpunkt: 174-176 °C (Probe aus Isopropanol/Wasser 1:1 umkristallisiert.)
¹H-NMR (Cl₂DC-CDCl₂; 80°C): δ = 5,83 (m, 1H, H an C-5); 5,74 (m, 1H, H an C-4); 3,65-3,80 (m, 2H, Hₐ an C-7 und Hₐ an C-9); 3,09 (dd, 1H, H_{b} an C-9); 2,92 (dd, 1H, H_{b} an C-7); 2,70 (m, 1H, H an C-2); 2,35-2,60 (m, 3H, Hₐ und H_{b} an C-3 und H an C-6); 2,01 (m, 1H, H an C-1); 1,5 ppm (s, 9H).

### C.2. (1SR,2RS,6SR)-8-tert.-Butyloxycarbonyl-2-ethyloxycarbonylamino-8-azabicyclo[4.3.0]non-4-en

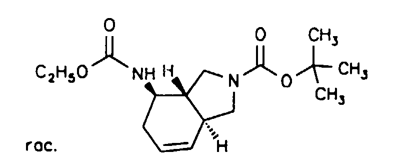

53,3 g (0,2 mol) der Titelverbindung aus Beispiel C.1. und 22,2 g (0,22 mol) Triethylamin in 200 ml wasserfreiem THF gelöst vorlegen. Unter Kühlung mit einer Eis/Kochsalz-Mischung 22,8 g (0,21 mol) Chlorameisensäureethylester in 40 ml THF gelöst so zutropfen, daß die Innentemperatur -10°C nicht überschreitet. Nach vollständiger Zugabe 1 h bei tiefer Temperatur nachrühren. Anschließend eine eisgekühlte Lösung von 15,6 g (0,24 mol) Natriumazid in 50 ml Wasser unter kräftigem Rühren so zutropfen, daß die Innentemperatur -10°C nicht überschreitet. Nach vollständiger Zugabe 30 min bei tiefer Temperatur nachrühren. Anschließend nacheinander 300 ml Wasser und 400 ml Toluol zugeben.

Die organische Phase abtrennen, mit Natriumsulfat trocknen, bei 15 mbar auf die Hälfte des ursprünglichen Volumens einengen (Badtemperatur unter 25°C). Zugabe von 100 ml Ethanol, unter Rühren langsam aufheizen (in dem Maße, wie es die Stickstoffentwicklung zuläßt) und nach beendeter Stickstoffentwicklung 4 h zum Rückfluß kochen. Einengen und Rohprodukt aus Methanol/Wasser 85:15 umkristallisieren, bei 50 °C bis zur Gewichtskonstanz trocknen.
Ausbeute: 24,2-28,5 g (39-46 % der Theorie) der Titelverbindung
Schmelzpunkt: 120-122 °C
¹H-NMR (CDCl₃): δ = 5,78 und 5,73 (2d, 1H, H an C-5); 5,64 (m, 1H, H an C-4); (4,59 br. s, 1H, NH); 4,12 (m, 2H, Ethoxy-CH₂); 3,90 (m, 1H, H an C-2); 3,74 und 3,67 (2m, 1H, Hₐ an C-7); 3,67 und 3,56 (2m, 1H, Hₐ an C-9); 3,12 (m, 1H, H_{b} an C-9); 2,92 (m, 1H, H_{b} an C-7); 2,67 (m, 1H, Hₐ an C-3); 2,49 (m, 1H, H an C-6); 1,95 (m, 1H, H_{b} an C-3); 1,83 (m, 1H, H an C-1); 1,46 (s, 9H); 1,24 ppm (m, 3H, Ethoxy-CH₃).

Die wäßrige Phase durch Zugabe von 10 %-iger Salzsäure auf einen pH von 2-3 einstellen, 30 min im Eisbad stehen lassen, den Niederschlag absaugen, mit Wasser waschen, bei 50 °C bis zur Gewichtskonstanz trocknen.
Ausbeute: 16,0-19,2 g (30-36 % der Titelverbindung aus Beispiel C.1.) (zurückgewonnene Ausgangsverbindung)

### C.3. (1SR,2RS,6SR)-2-Ethyloxycarbonylamino-8-azabicyclo[4.3.0]non-4-en

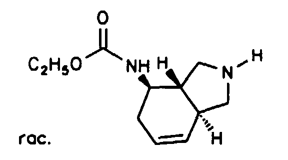

31,0 g (0,1 mol) der Titelverbindung aus Beispiel C.2. in 100 ml einer Mischung aus Methanol/Wasser (1:1) vorlegen (Suspension). 100 ml konz. Salzsäure rasch zulaufen lassen (leicht exotherm bis etwa 40°C, (man erhält eine homogene Lösung) und bis zum Ende der Gasentwicklung (etwa 10 min) nachrühren. 200 ml Eiswasser zugeben und 70 ml 45 %-ige Natronlauge unter Rühren und Eiskühlung zutropfen. Viermal mit je 50 ml Methylenchlorid extrahieren, die vereinigten organischen Phasen mit Natriumsulfat trocknen, einengen, im Hochvakuum Lösungsmittelreste abziehen. Die Substanz wird beim Einengen fest.
- Ausbeute:: 13,7-16,6 g (65-79 % der Theorie) braun-rosafarbener, amorpher Feststoff
- Rf =: 0,81 Titelverbindung aus Beispiel C.2.
0,11 Titelverbindung
Methylenchlorid/Methanol/17 %-iges wäßriges Ammoniak (15:4:0,5)
¹H-NMR (CDCl₃): δ = 5,78 (d, 1H, H an C-5); 5,63 (m, 1H, H an C-4); 4,94 (br.d, 1H, NH); 4,10 (m, 2H, Ethoxy-CH₂); 3,88 (m, 1H, H an C-2); 3,28 (m, 1H, Hₐ an C-7); 3,19 (m, 1H, Hₐ an C-9); 2,84 (m, 1H, H_{b} an C-9); 2,57-2,62 (m, 2H, Hₐ an C-3 und H_{b} an C-7); 2,43 (m, 1H, H an C-6); 1,95 (m, 1H, H_{b} an C-3); 1,79 (m, 1H, H an C-1); 1,23 ppm (m, 3H, Ethoxy-CH₃).

### Beispiel D:

### (1SR,2RS,6SR)-2-Methylamino-8-azabicyclo[4.3.0]non-4-en

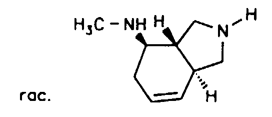

1,9 g (50 mmol) Lithiumaluminiumhydrid in 25 ml wasserfreiem Diethylether in einer Stickstoffatmosphäre vorlegen. 5,25 g (25 mmol) der Titelverbindung aus Beispiel C.3. in 50 ml wasserfreiem Tetrahydrofuran gelöst zutropfen und 3 h zum Rückfluß erhitzen. Weitere 0,95 g (25 mmol) Lithiumaluminiumhydrid zusetzen und nochmals 3 h zum Rückfluß erhitzen. Unter Eiskühlung langsam Wasser zutropfen, bis ein weißer Niederschlag entstanden ist. Niederschlag absaugen, zweimal mit je 100 ml Ethanol auskochen. Ethanolextrakte mit der Mutterlauge der Reaktion vereinigen, 50 ml Toluol zusetzen, einengen, Lösungsmittelreste im Hochvakuum abziehen.
Ausbeute: 1,95 g (77 % der Theorie) amorpher Feststoff
Rf = 0,11
Methylenchlorid/Methanol/17 %-iges wäßriges Ammoniak (2:4:1)
¹H-NMR (CDCl₃): δ = 5,77 (d, 1H, H an C-5); 5,67 (m, 1H, H an C-4); 3,33 (dd, 1H, Hₐ an C-7); 3,26 (dd, 1H, Hₐ an C-9); 2,73-2,82 und 2,54-2,63 (2m, 4H, H an C-2, Hₐ an C-3, H_{b} an C-7 und H_{b} an C-9); 2,41 (s, 3H, CH₃N); 2,34 (m, 1H, H an C-6); 1,90 (m, 1H, H_{b} an C-3); 1,70 ppm (m, 1H, H an C-1).

### Beispiel E:

### (1RS,2RS,6SR)-2-Hydroxymethyl-8-azabicyclo[4.3.0]non-4-en

### E.1. (1RS,2RS,6SR)-8-tert.-Butyloxycarbonyl-2-hydroxymethyl-8-azabicyclo[4.3.0]non-4-en (Diastereomer A) und

### (1RS,2RS,6RS)-8-tert.-Butyloxycarbonyl-2-hydroxymethyl-8-azabicyclo[4.3.0]non-4-en (Diastereomer B)

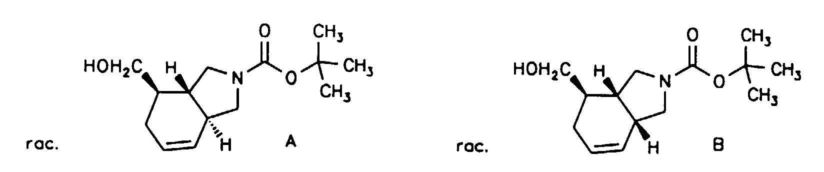

29,5 g (0,1 mol) der Titelverbindung aus Beispiel B.4. in 200 ml wasserfreiem 1,2-Dimethoxyethan in einer Stickstoffatmosphäre vorlegen. Bei einer Innentemperatur <-65°C 150 ml einer 1,5 m DIBAH-Lösung in Toluol (0,225 mol) zutropfen. Nach vollständiger Zugabe Kühlbad entfernen und auf Raumtemperatur kommen lassen. 2 h bei Raumtemperatur nachrühren.

Unter kräftigem Rühren 60 ml Methanol zutropfen (exotherme Reaktion); Innentemperatur durch Kühlung mit einem Kaltwasserbad zwischen 35 und 45 °C halten. Anschließend 20 ml 5 %-ige Natronlauge zutropfen. Nach vollständiger Zugabe 10 min nachrühren. Niederschlag absaugen, zweimal unter Rühren mit je 150 ml Ethanol auskochen, Ethanolextrakte und Reaktionslösung vereinigen, einengen, im Hochvakuum Lösungsmittelreste abziehen, Rückstand an 250 g Kieselgel (63-200 µm) mit Cyclohexan/Aceton (4:1) säulenchromatographisch reinigen.
- Ausbeute:: 12,9-17,7 g (51-70 % der Theorie) gelbliches Öl; Gemisch der Diastereomeren A und B im Verhältnis 4:1
- Rf =: 0,36 Titelverbindung aus Beispiel B.4.
0,12 Titelverbindung A und B

Das Rohprodukt wird nach längerem Stehen fest. Durch Umkristallisieren aus Ether/Petrolether kann eine diastereomerenreine Probe des Hauptdiastereomeren A erhalten werden.
¹H-NMR (CDCl₃): (Diastereomer A) δ = 5,67-5,82 (m, 2H, H an C-4 und C-5); 3,50-3,77 (m, 4H, Hₐ an C-7, Hₐ an C-9 und Hydroxymethyl-CH₂); 3,02 (dt, 1H, H_{b} an C-9); 2,85 (m, 1H, H_{b} an C-7); 2,2-2,4 (m, 3H); 1,87-2,00 (m, 3H); 1,62 (m, 1H, H an C-1); 1,46 ppm (s, 9H).

### E.2. (1RS,2RS,6SR)-2-Hydroxymethyl-8-azabicyclo[4.3.0]non-4-en

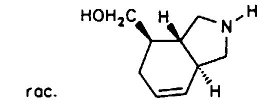

2,5 g (10 mmol) der Titelverbindung A aus Beispiel E.1. in 10 ml Methanol vorlegen. 10 ml konz. Salzsäure rasch zulaufen lassen und 30 min nachrühren. Mit Wasser auf das doppelte Volumen verdünnen dann 45 %-ige Natronlauge unter Rühren und Eiskühlung zutropfen, bis zu einem pH-Wert von ≧12. Einengen, Rückstand zweimal unter Rühren mit Ethanol auskochen, Ethanolextrakte einengen, im Hochvakuum Lösungsmittelreste abziehen.
Ausbeute: 2,1 g (Produkt enthält NaCl-Rückstände)
Rf = 0,20
Methylenchlorid/Methanol/17 %-iges wäßriges Ammoniak (2:4:1)
¹H-NMR (d₆-DMSO): δ 5,76 (d, 1H); 5,62 (d, 1H); 3,47-3,56 (m, 2H, Hₐ an C-7 und Hₐ an C-9); 3,32-3,47 (m, 1H, Hₐ von Hydroxymethyl-CH₂); 3,23-3,32 (m, 1H, H_{b} von Hydroxymethyl-CH₂); 2,77 (t, 1H, H_{b} an C-9); 2,64 (t, 1H, H_{b} an C-7); 2,10-2,24 (m, 2H, Hₐ an C-3 und H an C-6); 1,77-1,88 (m, 1H, H_{b} an C-3); 1,69 (m, 1H, H an C-2); 1,40 ppm (m, 1H, H an C-1).

### Beispiel F:

### (1RS,2RS,6SR)-2-Ethyloxycarbonylaminomethyl-8-azabicyclo[4.3.0]non-4-en

### F.1. (1RS,2RS,6SR)-8-tert.-Butyloxycarbonyl-2-(4-toluolsulfonyloxymethyl)-8-azabicyclo[4.3.0]non-4-en (Diastereomer A) und

### (1RS,2RS,6RS)-8-tert.-Butyloxycarbonyl-2-(4-toluolsulfonyloxymethyl)-8-azabicyclo[4.3.0]non-4-en (Diastereomer B)

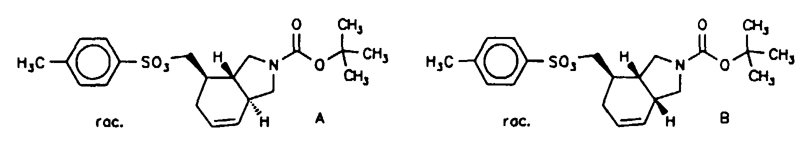

12,7 g (0,05 mol) der Titelverbindung aus Beispiel E.1. (Rohgemisch der Diastereomere A und B) in 25 ml wasserfreiem Pyridin vorlegen und auf -15°C kühlen. 11,0 g (0,0575 mol) 4-Toluolsulfonsäurechlorid portionsweise so zugeben, daß die Innentemperatur -5°C nicht übersteigt. Nach vollständiger Zugabe 2 h bei einer Temperatur von -5 bis - 15 °C, dann 3 h bei Raumtemperatur nachrühren. 5 g Eis zugeben, 5 min rühren, auf 50 ml Wasser geben, Niederschlag absaugen, mit Wasser waschen, bei 50 °C bis zur Gewichtskonstanz trocknen.
- Ausbeute:: 14,4-16,3 g (71-80 % der Theorie)
blaßrosafarbener Feststoff
Gemisch der Diastereomeren A und B

Durch Umkristallisieren aus Methanol kann eine diastereomerenreine Probe des Hauptdiastereomeren A erhalten werden.
Schmelzpunkt: 111-113 °C
¹H-NMR (CDCl₃): (Diastereomer A) δ = 7,79 (m, 2H, Aryl-H); 7,36 (d, 2H, Aryl-H); 5,74 und 5,78 (2d, 1H, H an C-5); 5,64 (m, 1H, H an C-4); 3,87-3,97 (m, 2H, Tosyl-OCH₂-); 3,59 und 3,67 (2dd, 1H, Hₐ an C-7); 3,48 (dd, 1H, Hₐ an C-9); 2,78-2,96 (m, 2H, H_{b} an C-7 und H_{b} an C-9); 2,47 (s, 3H, Aryl-CH₃); 2,22-2,36 (m, 2H, Hₐ an C-3 und H an C-6); 2,06 (m, 1H, H an C-2); 1,80-1,98 (m, 1H, H_{b} an C-3); 1,59 (m, 1H, H an C-1); 1,45 und 1,47 ppm (2s, 9H).

### F.2. (1RS,2RS,6SR)-8-tert.-Butyloxycarbonyl-2-ethyloxycarbonylaminomethyl-8-azabicyclo[4.3.0]non-4-en (Diastereomer A) und

### (1RS,2RS,6RS)-8-tert.-Butyloxycarbonyl-2-ethyloxycarbonylaminomethyl-8-azabicyclo[4.3.0]non-4-en (Diastereomer B)

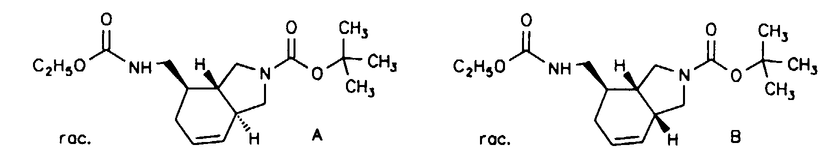

20,5 g (0,05 mol) der Titelverbindung aus Beispiel F.1. (Rohgemisch der Diastereomere A und B) und 6,5 g (0,1 mol) Natriumazid in 100 ml DMF 4 h auf 70 °C erhitzen. Reaktionslösung auf 200 ml Wasser geben, einmal mit 200 ml Petrolether extrahieren, die Petroletherphase einmal mit 50 ml Wasser waschen, mit Natriumsulfat trocknen und bei Raumtemperatur einengen.

Den Rückstand in 80 ml THF aufnehmen und 13,1 g (0,05 mol) Triphenylphosphin in 80 ml THF gelöst zutropfen. Nach vollständiger Zugabe 20 h bei Raumtemperatur rühren, dann 150 ml Wasser langsam zutropfen, nach vollständiger Zugabe 15 min nachrühren. Unter Kühlung Salzsäure zutropfen (konz. HCl/Wasser 1:3), bis ein pH-Wert von 3-4 erreicht ist, THF bei Raumtemperatur im Vakuum abziehen, Reaktionslösung auf 0°C kühlen, ausgefallenes Triphenylphosphinoxid absaugen (oder mit MTBE aufnehmen, falls ölig).

Wäßrige Phase durch Zugabe von 10 %-iger Natronlauge auf einen pH-Wert ≧12 einstellen, zweimal mit je 100 ml Methylenchlorid extrahieren, vereinigte Extrakte mit Natriumsulfat trocknen, anschließend 6,0 g (0,06 mol) Triethylamin zugeben, unter Rühren 6,0 g (0,055 mol) Chlorameisensäureethylester in 20 ml Methylenchlorid gelöst zutropfen, über Nacht bei Raumtemperatur rühren, Reaktionslösung einmal mit 100 ml Wasser waschen, mit Natriumsulfat trocknen und einengen.

23 g Rohprodukt an 100 g Kieselgel (63-200 µm) mit Cyclohexan/ Aceton (4:1) säulenchromatographisch reinigen.
- Ausbeute:: 12,4 g (76 % der Theorie) zähes Öl
Gemisch der Diastereomeren A und B
- Rf-Werte (Cyclohexan/Aceton 2:1):: 0,32 Diastereomer A
0,29 Diastereomer B

Die Diastereomere A und B werden an 250 g Kieselgel (35-70 µm) mit Cyclohexan/Aceton (8:1) säulenchromatographisch getrennt.
- Ausbeute:: 4,3 g (26 % der Theorie) Diastereomer A (zähes Öl)
2,4 g (15 % der Theorie) Mischfraktion
0,6 g (4 % der Theorie) Diastereomer B
¹H-NMR (Cl₂DC-CDCl₂; 80 °C):
Diastereomer A: δ = 5,75 (d, 1H, H an C-5); 5,66 (m, 1H, H an C-4); 4,67 (br, 1H, NH); 4,08 (q, 2H, Ethoxy-CH₂); 3,62 (br, 2H, Hₐ an C-7 und Hₐ an C-9); 3,19 (br, 1H, Hₐ an CH₂-NH); 3,05 (br, H_{b} an CH₂-NH); 2,96 (dd, 1H, H_{b} an C-9); 2,81 (dd, 1H, H_{b} an C-7); 2,24-2,34 (m, 2H, Hₐ an C-3 und H an C-6); 1,78-1,94 (m, 2H, H an C-2 und H_{b} an C-3); 1,54 (m, 1H, H an C-1); 1,43 (s, 9H); 1,22 ppm (t, 3H, Ethoxy-CH₃).
Diastereomer B: δ = 5,69 (m, 1H, H an C-4); 5,57 (m, 1H, H an C-5); 4,65 (br, 1H, NH); 4,08 (q, 2H, Ethoxy-CH₂); 3,52 (dd, 1H, Hₐ an C-7); 3,41 (dd, 1H, Hₐ an C-9); 3,29 (dd, 1H, H_{b} an C-9); 3,24 (dd, 1H, Hₐ an CH₂-NH); 3,03-3,12 (m, 2H, H_{b} an C-7 und H_{b} an CH₂-NH); 2,68 (m, 1H, H an C-6); 2,12-2,22 (m, 2H, H an C-1 und Hₐ an C-3); 1,74-1,87 (m, 2H, H an C-2 und H_{b} an C-3); 1,43 (s, 9H); 1,22 ppm (t, 3H, Ethoxy-CH₃).

### F.3. (1RS,2RS,6SR)-2-Ethyloxycarbonylaminomethyl-8-azabicyclo [4.3.0]non-4-en

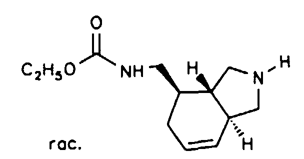

1,6 g (5,7 mmol) der Titelverbindung A aus Beispiel F.2. in 10 ml Methanol vorlegen. 8 ml konz. Salzsäure rasch zulaufen lassen und 30 min nachrühren. Mit Wasser auf das doppelte Volumen verdünnen dann 45 %-ige Natronlauge unter Rühren und Eiskühlung zutropfen, bis zu einem pH-Wert von ≧12. Viermal mit Methylenchlorid extrahieren, die vereinigten organischen Phasen mit Natriumsulfat trocknen, einengen, im Hochvakuum Lösungsmittelreste abziehen.
Ausbeute: 0,8 g (63 % der Theorie) zähes Öl
Rf = 0,16
Methylenchlorid/Methanol/17 %-iges wäßriges Ammoniak (15:4:0,5)
¹H-NMR (CDCl₃): δ = 5,81 (d, 1H, H an C-5); 5,67 (m, 1H, H an C-4); 5,00 (br, 1H, NH); 4,10 (q, 2H, Ethoxy-CH₂); 3,18-3,28 und 3,08 (m, 3H und m, 1H: Hₐ an C-7, Hₐ an C-9, Hₐ und H_{b} an CH₂-NH-CO); 2,67 (dd, 1H, H_{b} an C-9); 2,53 (dd, 1H, H_{b} an C-7); 2,34 (m, 1H, Hₐ an C-3); 2,25 (m, 1H, H an C-6); 1,79-1,96 (m, 2H, H an C-2 und H_{b} an C-3); 1,50 (m, 1H, H an C-1); 1,24 ppm (t, 3H, Ethoxy-CH₃).

### Beispiel G:

### (1RS,2SR,6RS)-2-Hydroxymethyl-8-azabicyclo[4.3.0]non-4-en

### G.1. (E)-1-tert.-Butyloxycarbonylamino-2,4-pentadien

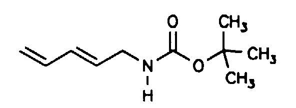

8,3 g (0,1 mol) (E)-1-Amino-2,4-pentadien (Titelverbindung aus Beispiel B.2.) in 50 ml MTBE vorlegen und 20 mg 4-Hydroxyanisol zusetzen. Anschließend bei einer Innentemperatur von 20-30°C 22,9 g (0,105 mol) Di-tert.-butyl-dicarbonat in 50 ml MTBE gelöst zutropfen. Nach vollständiger Zugabe 20 h bei Raumtemperatur rühren. Einengen, Reste von Di-tert.-butyl-dicarbonat im Hochvakuum bei 40 °C abziehen.
Ausbeute: 18,9 g (Rohprodukt) farbloses Öl
Rf = 0,25
Cyclohexan/Aceton (4:1)
¹H-NMR (CDCl₃): δ = 6,05-6,43 (m, 2H, H an C-3 und C-4); 5,68 (dd, 1H, H an C-2, J= 14 und 6 Hz); 5,17 (dd, 1H, Hₐ an C-5, J = 16 Hz); 5,07 (dd, 1H, H_{b} an C-5, J = 10 Hz); 4,75 (br, 1H, NH); 3,77 (t, 2H, H an C-1); 1,45 ppm (s, 9H).

### G.2. (1RS,2RS,6RS)-2-tert.-Butyloxcarbonylaminomethyl-7,9-dioxo-8-oxabicyclo[4.3.0]non-3-en

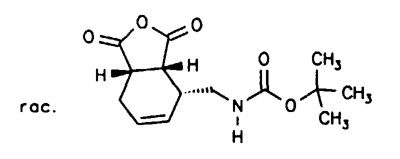

83,2 g (1,0 mol) (E)-1-Amino-2,4-pentadien (Titelverbindung aus Beispiel B.2.) in 250 ml MTBE vorlegen und 0,1 g 4-Hydroxyanisol zusetzen. Anschließend bei einer Innentemperatur von 20-30°C 229,2 g (1,05 mol) Di-tert.-butyl-dicarbonat in 250 ml MTBE gelöst zutropfen. Nach vollständiger Zugabe 20 h bei Raumtemperatur rühren. Reaktionsgemisch einengen und in 1 l Toluol aufnehmen. 103,0 g (1,05 mol) Maleinsäureanhydrid zusetzen und 24 h bei einer Innentemperatur von 60°C rühren. Niederschlag absaugen, mit Toluol waschen und bei 50°C bis zur Gewichtskonstanz trocknen.
Ausbeute: 208,2 g (74 % der Theorie) weißer, kristalliner Feststoff
Schmelzpunkt: 157-159 °C
¹H-NMR (d₆-DMSO): δ = 5,81 (m, 1H, H an C-4); 5,59 (d, 1H, H an C-3); 3,77 (dd, 1H Hₐ an CH₂-NH); 3,44 (m, 2H, H an C-1 und H_{b} an CH₂-NH); 2,94 (m, 1H, H an C-2); 2,66 (m, 1H, H an C-6); 2,16 (m, 1H, Hₐ an C-5); 2,06 (m, 1H, H_{b} an C-5); 1,43 ppm (s, 9H).

### G.3. (1RS,2SR,6RS)-9-Oxo-8-azabicyclo[4.3.0]non-4-en-2-carbonsäuremethylester

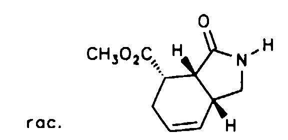

83,2 g (1,0 mol) (E)-1-Amino-2,4-pentadien (Titelverbindung aus Beispiel B.2.) in 250 ml THF vorlegen und 0,1 g 4-Hydroxyanisol zusetzen. Anschließend bei einer Innentemperatur von 20-30°C 229,2 g (1,05 mol) Di-tert.-butyl-dicarbonat in 250 ml THF gelöst zutropfen. Nach vollständiger Zugabe 20 h bei Raumtemperatur rühren. 103,0 g (1,05 mol) Maleinsäureanhydrid zusetzen und 5 h zum Rückfluß erhitzen. Einengen und den Rückstand in 500 ml Methanol aufnehmen, 30 ml p-Toluolsulfonsäure zusetzen, dann erneut 5 h zum Rückfluß erhitzen. Nach Abkühlung unter Eiskühlung und Rühren eine Lösung von 20 g Natriumcarbonat in 500 ml Wasser gelöst rasch zutropfen, Ansatz noch 30 min im Eisbad stehen lassen, Niederschlag absaugen, mit wenig Wasser waschen, bei 50°C bis zur Gewichtskonstanz trocknen.
Ausbeute: 125-148 g (64-76 % der Theorie) weißer, kristalliner Feststoff
Schmelzpunkt: 190-193 °C
¹H-NMR (d₆-DMSO): δ = 7,50 (s, 1H, NH); 5,77 (m, 1H, H an C-4); 5,56 (m, 1H, H an C-5); 3,60 (s, 3H, CH₃O); 3,42 (dd, 1H, Hₐ an C-7); 3,16 (dd, 1H, H an C-1); 3,00 (m, 1H, H an C-6); 2,88 (dd, 1H, H_{b} an C-7); 2,67 (m, 1H, H an C-2); 2,02-2,18 ppm (m, 2H, Hₐ und H_{b} an C-3).

### G.4. (1RS,2SR,6RS)-2-Hydroxymethyl-8-azabicyclo[4.3.0]non-4-en

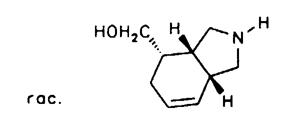

19,6 g (0,1 mol) der Titelverbindung aus Beispiel G.3. in 100 ml THF unter Inertgasatmosphäre vorlegen (Suspension). 100 ml (0,15 mol) 1,5 m DIBAH-Lösung in Toluol bei einer Innentemperatur von 10-20°C zutropfen. Die so erhaltene klare, homogene Lösung zu einer Suspension von 1,9 g Lithiumalanat in 50 ml THF zutropfen. Nach vollständiger Zugabe 15 min bei Raumtemperatur, anschließend 30 min bei Rückflußtemperatur rühren. Nach Abkühlung portionsweise 3,8 g (0,1 mol) Lithiumalanat zugeben, dann 24 h zum Rückfluß erhitzen. Nach Abkühlung nacheinander 50 ml Wasser und 10 ml 1M-Natronlauge zutropfen, den Niederschlag absaugen und dreimal mit je 150 ml Ethanol auskochen. Filtrat und Extrakte vereinigen und einengen.
Ausbeute: 16,4 g (Produkt enthält Lithium- und Aluminiumhydroxid)
Rf = 0,3
Methylenchlorid/Methanol/17 %-iges wäßriges Ammoniak (2:4:1)

### Beispiel H:

### (1RS,2SR,6RS)-2-Ethyloxycarbonylaminomethyl-8-azabicyclo[4.3.0]non-4-en

### H.1. (1RS,2SR,6RS)-8-tert.-Butyloxycarbonyl-2-hydroxymethyl-8-azabicyclo[4.3.0]non-4-en

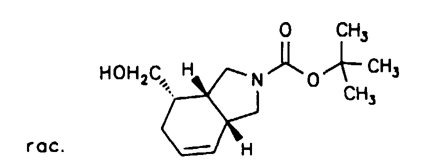

16,4 g Rohprodukt aus Beispiel G.4. (entspricht 0,1 mol der Titelverbindung aus Beispiel G.4.) in 100 ml THF lösen. Bei einer Innentemperatur von 0-5°C 22,9 g (0,105 mol) Di-tert.-butyl-dicarbonat in 100 ml THF gelöst zutropfen, 24 h bei 0°C, anschließend weitere 24 h bei Raumtemperatur rühren. Einengen, Rohprodukt an 250 g Kieselgel (63-200 µm) mit Cyclohexan/Aceton (2:1) säulenchromatographisch reinigen.
- Ausbeute:: 13,7 g (54 % der Theorie über 2 Stufen); zähes Öl
- Rf =: 0,21 Titelverbindung
0,08 Titelverbindung aus Beispiel G.4.

### H.2. (1RS,2SR,6RS)-8-tert.-Butyloxycarbonyl-2-(4-toluolsulfo-nyloxymethyl)-8-azabicyclo[4.3.0]non-4-en

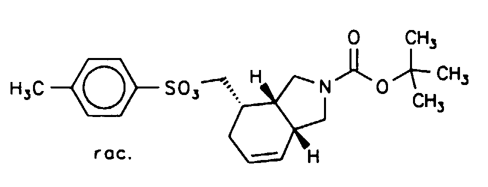

Analog Beispiel F.1. erhält man aus der Titelverbindung aus Beispiel H.1. die Titelverbindung.
Ausbeute: 81-83 % der Theorie
Schmelzpunkt: 160-162 °C
¹H-NMR (CDCl₃): δ = 7,79 (m, 2H, Aryl-H); 7,37 (d, 2H, Aryl-H); 5,67 (m, 1H, H an C-4); 5,47 (m, 1H, H an C-5); 3,78-3,97 (m, 2H, Tosyl-OCH₂-); 3,13-3,42 (m, 3H, CH₂-N); 2,95 (t, 1H, CH₂-N); 2,74 (m, 1H); 2,54 (m, 1H); 2,47 (s, 3H, Aryl-CH₃); 2,32 (m, 1H, H an C-2); 2,06 (m, 1H, Hₐ an C-3); 1,66-1,83 (m, 1H, H_{b} an C-3); 1,44 ppm (s, 9H).

### H.3. (1RS,2SR,6RS)-8-tert.-Butyloxycarbonyl-2-ethyloxycarbonylaminomethyl-8-azabicyclo[4.3.0]non-4-en

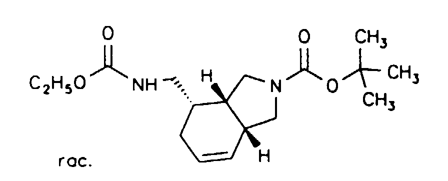

Analog Beispiel F.2. erhält man aus der Titelverbindung aus Beispiel H.2. die Titelverbindung.

Rohprodukt an Kieselgel (63-200 µm) mit Cyclohexan/Aceton (2:1) säulenchromatographisch reinigen.
Ausbeute: 76 % der Theorie; klares, zähes Öl
Rf = 0,35 (Cyclohexan/Aceton 2:1)
¹H-NMR (Cl₂DC-CDCl₂; 80 °C): δ = 5,69 (m, 1H, H an C-4); 5,47 (d, 1H, H an C-5); 4,59 (br, 1H, NH); 4,10 (q, 2H, Ethoxy-CH₂); 3,38 (dd, 1H); 3,32 (m, 1H); 3,24 (m, 1H); 3,01-3,08 (m, 3H); 2,79 (m, 1H); 2,47 (m, 1H); 2,07 (m, 2H); 1,78 (m, 1H); 1,42 (s, 9H); 1,22 ppm (t, 3H, Ethoxy-CH₃).

### H.4. (1RS,2SR,6RS)-2-Ethyloxycarbonylaminomethyl-8-azabicyclo [4.3.0]non-4-en

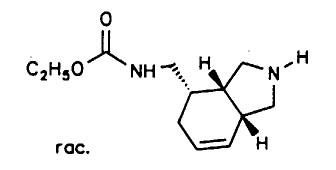

Analog Beispiel C.3. erhält man aus der Titelverbindung aus Beispiel H.3. die Titelverbindung.
- Ausbeute:: 42 % der Theorie
- Rf =: 0,93 Titelverbindung aus Beispiel H.3.
0,23 Titelverbindung
Methylenchlorid/Methanol/17 %-iges wäßriges Ammoniak (15:4:0,5)

### Beispiel I:

### (1SR,2RS,3RS,6SR)-2-Ethyloxycarbonylamino-3-methyl-8-azabicyclo[4.3.0]-non-4-en

### I.1. N-[(2E,4E)-2,4-Hexadienyl]-phthalimid

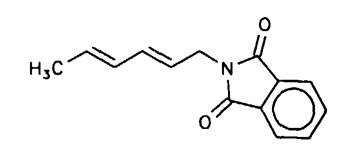

Analog Beispiel B.1. erhält man aus (2E,4E)-1-Brom-2,4-hexadien die Titelverbindung.
Ausbeute: 77-79 % der Theorie
Schmelzpunkt: 114-117 °C (Probe aus Ethanol umkrist.)
¹H-NMR (CDCl₃): δ = 7,85 (m, 2H); 7,72 (m, 2H); 6,25 (dd, 1H); 6,00 (ddd, 1H); 5,5-5,8 (m, 2H); 4,29 (d, 2H); 1,74 ppm (d, 3H).

### I.2. (2E,4E)-1-Amino-2,4-hexadien

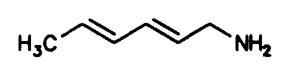

Analog Beispiel B.2. erhält man aus der Titelverbindung aus Beispiel I.1. die Titelverbindung; Siedebereich: 40-70 °C bei 16-18 mbar.
Ausbeute: 67-83 % der Theorie

### I.3. (E)-4-[(2E,4E)-2,4-Hexadienylamino]-2-butensäureethylester

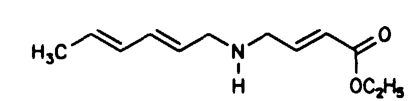

Analog Beispiel B.3. erhält man aus der Titelverbindung aus Beispiel I.2. die Titelverbindung.
Ausbeute: 46 % der Theorie
¹H-NMR (CDCl₃): δ = 6,98 (dt, 1H); 5,9-6,25 (m, 3H); 5,5-5,8 (m, 2H); 4,19 (q, 2H); 3,40 (dd, 2H); 3,27 (d, 2H); 1,76 (d, 3H); 1,29 ppm (t, 3H).

### I.4. (1RS,2RS,3RS,6SR)-8-tert.-Butyloxycarbonyl-3-methyl-8-azabicyclo[4.3.0]non-4-en-2-carbonsäureethylester

### (Diastereomer A) und

### (1RS,2RS,3SR,6RS)-8-tert.-Butyloxycarbonyl-3-methyl-8-azabicyclo[4.3.0]non-4-en-2-carbonsäureethylester

### (Diastereomer B)

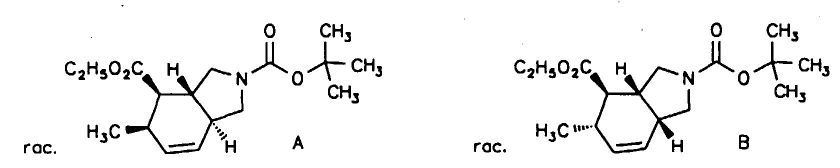

Analog Beispiel B.4. erhält man aus der Titelverbindung aus Beispiel I.3. die Titelverbindungen.
Ausbeute: 70 % der Theorie; Gemisch aus 2 Diastereomeren A und B im Verhältnis A:B = 4:1.
Rf= 0,49 (Cyclohexan/Aceton 2:1)

### I.5. (1RS,2RS,3RS,6SR)-8-tert.-Butyloxycarbonyl-3-methyl-8-azabicy clo[4.3.0]non-4-en-2-carbonsäure

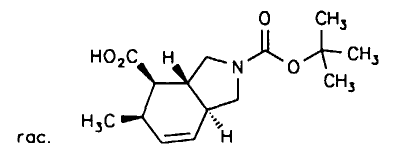

1,17 g (21 mmol) Kaliumhydroxid in 20 ml Wasser gelöst vorlegen. 5,9 g (19 mmol) der Titelverbindung aus Beispiel I.4. in 20 ml Methanol gelöst zugeben und 48 h unter einer Stickstoffatmosphäre zum Rückfluß erhitzen. Einengen, in Wasser aufnehmen, einmal mit Methylenchlorid extrahieren, wässrige Phase mit Essigsäure auf pH 3-4 einstellen, Niederschlag absaugen, mit Wasser waschen, bei Raumtemperatur trocknen, aus Cyclohexan/Aceton 6:1 umkristallisieren.
Ausbeute: 2,25 g (42 % der Theorie)
Schmelzpunkt: 189 °C
¹H-NMR (d₆-DMSO): δ = 5,77 (d, 1H); 5,61 (m, 1H); 3,67 (m, 1H); 3,54 (m, 1H); 2,61-2,95 (m, 4H); 2,30 (m, 1H); 1,82 (m, 1H); 1,40 (s, 9H); 0,90 ppm (d, 3H).

### I.6. (1SR,2RS,3RS,6SR)-8-tert.-Butyloxycarbonyl-2-ethyloxycarbonylamino-3-methyl-8-azabicyclo[4.3.0]non-4-en

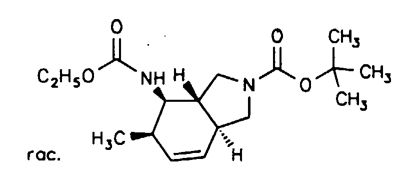

Analog Beispiel C.2. erhält man aus 2,25 g (8 mmol) der Titelverbindung aus Beispiel I.5. die Titelverbindung. Gegenüber Beispiel C.2. geändert: 8 h Rückfluß in Ethanol anstatt 4 h; Reinigung durch Säulenchromatographie an 100 g Kieselgel (63-200 µm) mit Toluol/Essigester (2:1).
Ausbeute: 1,6 g (59 % der Theorie) klares Öl
¹H-NMR (CDCl₃): δ = 5,68 und 5,72 (2d, 1H); 5,61 (m, 1H); 4,81 (m, 1H); 4,0-4,2 (m, 3H); 3,53 (m), 3,62 (m) und 3,72 (dd) [2H]; 3,08 (t, 1H); 2,92 (t, 1H); 2,75 (m, 1H); 2,47 (m,1H); 1,83 (m, 1H); 1,47 (m, 9H); 1,25 (m, 3H); 0,97 ppm (d, 3H).

### I.7. (1SR,2RS,3RS,6SR)-2-Ethyloxycarbonylamino-3-methyl-8-azabicyclo[4.3.0]non-4-en

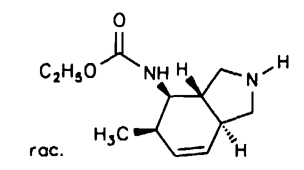

Analog Beispiel C.3. erhält man aus 1,6 g (4,7 mmol) der Titelverbindung aus Beispiel I.6. die Titelverbindung.
Ausbeute: 0,7 g (70 % der Theorie) gelbliches Öl;
Rf = 0,09
Methylenchlorid/Methanol/17 %-iges wäßriges Ammoniak (15:4:0,5)

### Beispiel K:

### (1RS,2RS,6RS)-2-Ethyloxycarbonylaminomethyl-8-azabicyclo[4.3.0]non-4-en

### K.1. 3-Phthalimidomethyl-cyclohex-4-en-1,2-dicarbonsäurediethylester

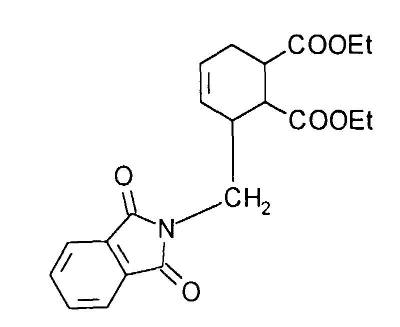

10.67 g (50 mmol) N-[(E)-2,4-Pentadienyl]-phthalimid (Titelverbindung aus Beispiel B.1.) und 8.61 g Fumarsäurediethylester werden in 50 ml Toluol 2 Tage zum Rückfluß erhitzt. Der Ansatz wird eingeengt und der Rückstand an Kieselgel chromatographiert (Laufmittel: Cyclohexan/Aceton 8:1).
Ausbeute: 14,8 g (77 % der Theorie)
Schmelzpunkt 80-84°C

### K.2. Ethyl-(1RS,2RS,6RS)-9-oxo-8-azabicyclo[4.3.0]non-4-en-2-carboxylat (A) und

### Ethyl-(1RS,2RS,6SR)-9-oxo-8-azabicyclo[4.3.0]non-4-en-2-carboxylat (B)

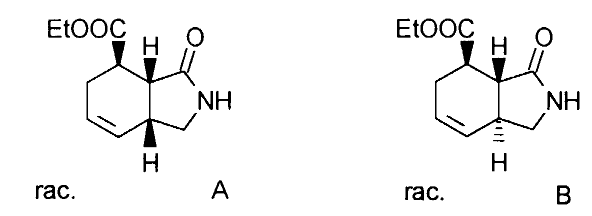

150,3 g (0,39 mol) der Titelverbindung aus Beispiel K.1. werden in 720 ml Ethanol vorgelegt und unter Eiskühlung 173,3 g (2,9 mol) Ethylendiamin zugetropft. Man rührt 20 h bei Raumtemperatur, engt in vacuo ein, verdünnt mit Wasser (ca. 700 ml), stellt mit konz. Salzsäure pH 2-3 ein und extrahiert dreimal mit jeweils 500 ml Dichlormethan. Die organische Phase wird getrocknet (Natriumsulfat) und in vacuo eingeengt. Die Trennung der Diastereomeren gelingt durch Chromatographie (Laufmittel: Cyclohexan/Aceton 1:1).
- Ausbeute:: 36,7 g Produkt A (45 % der Theorie)
RF = 0.47 (Cyclohexan/Aceton 1:1)
27,0 g Produkt B (45 % der Theorie)
RF = 0.22 (Cyclohexan/Aceton 1:1)

### K.3. (1RS,2RS,6RS)-2-Hydroxymethyl-8-azabicyclo[4.3.0]non-4-en

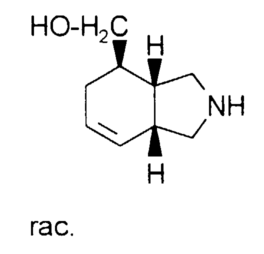

5,2 g (25 mmol) Ethyl-(1RS,2RS,6RS)-9-oxo-8-azabicyclo[4.3.0]non-4-en-2-carboxylat (Produkt A aus Beispiel K.2.) werden unter Stickstoff-Atmosphäre in 50 ml Tetrahydrofuran gelöst und nachfolgend 130 ml einer 1.5molaren Di(isobutyl)aluminiumhydrid-Lösung (195 mmol) zugetropft. Die Lösung wird 16 h unter Rückfluß erwärmt. Nach vollständiger Umsetzung werden nacheinander 60 ml Methanol, 30 ml tert.-Butylmethylether und 10 ml Wasser zugetropft und unter Zusatz von Tonsil abgesaugt. Der Nutschrückstand wird zweimal mit einer Mischung aus Ethanol/konz. Ammoniak/Wasser (10:1:1) verrührt und erneut abgesaugt. Die vgereinigten Filtrate werden eingeengt und das Rohprodukt chromatographisch gereinigt (Laufmittel: Dichlormethan/Methanol/konz. Ammoniak 2:4:1).
Ausbeute: 2,7 g (71 % der Theorie)
¹H-NMR (DMSO-d₆): 5.69 (m, 1H, 4-H); 5.60 (m, 1H, 5-H); 3.39 (dd, 1H, 10a-H); 3.26 (dd, 1H, 10b-H); 2.97 (m, 2H, 7a-H, 9a-H), 2.63 (m, 1H, 9b-H); 2.38 (bs, 1H, 6-H)M; 2.32 (dd, 1H, 7b-H); 2.06 (m, 1H, 3a-H); 1.95 (m, 1H, 1-H); 1.77 (m, 1H, 3b-H); 1.44 ppm (m, 1H, 2-H).

### K.4. (1RS,2RS,6RS)-8-tert.-Butuloxycarbonyl-2-hydroxymethyl-8-azabicyclo[4.3.0]non-4-en

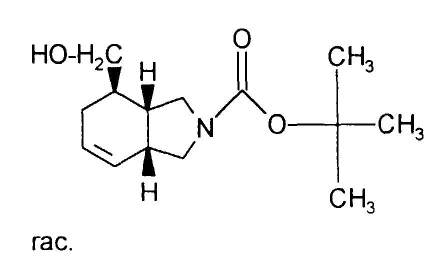

Das Produkt aus Beispiel K.3. (8,87 g; 58 mmol) wird umgesetzt wie im Beispiel H.1. beschrieben.
Ausbeute: 11,0 g (75 % der Theorie)
RF = 0.25 (Cyclohexan/Aceton 2:1)

### K.5. (1RS,2RS,6RS)-8-tert.-Butyloxycarbonyl-2-(4-toluolsulfonyloxymethyl)-8-azabicyclo[4.3.0]non-4-en

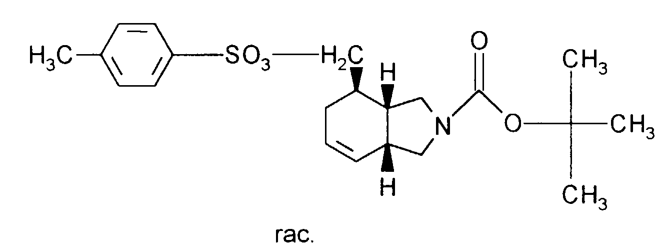

Die Titelverbindung wird aus dem Produkt von Beispiel K.4. in Analogie zum Beispiel F.1. erhalten.
Ausbeute: 97 % der Theorie
RF = 0.40 (Cyclohexan/Aceton 2:1)

### K.6. (1RS,2RS,6RS)-8-tert.-Butyloxycarbonyl-2-azidomethyl-8-azabicyclo[4.3.0]non-4-en

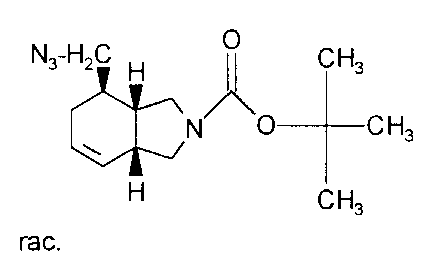

Eine Lösung von 33 g (0,08 mol) (1RS,2RS,6RS)-8-tert.-Butyloxycarbonyl-2-(4-toluolsulfonyloxymethyl)-8-azabicyclo[4.3.0]non-4-en (Titelverbindung aus Beispiel K.5.) und 15,8 g (0,24 mol) Natriumazid in 200 ml N,N-Dimethylformamid wird 40 h bei 70°C gerührt. Die abgekühlte Lösung wird mit Wasser (500 ml) verdünnt und dreimal mit je 250 ml Petrolether extrahiert. Die vereinigte organische Phase wird mit 5 %iger Natriumhydrogencarbonat-Lösung gewaschen, getrocknet (Natriumsulfat) und eingeengt.
Ausbeute: 21,6 g (97 %)
¹H-NMR (CDCl₃): 5.71 (m, 1H, C=CH); 5.58 (m, 1H, C=CH); 3.61-3.22 (m, 2H); 3.10 (m, 1H); 2.70 (bs, 1H); 2.24 (m, 2H); 1.91 (m, 2H), 1.47 ppm (s, 9H, tert.-Butyl).

### K.7. (1RS,2RS,6RS)-8-tert.-Butyloxycarbonyl-2-aminomethyl-8-azabicyclo[4.3.0]non-4-en

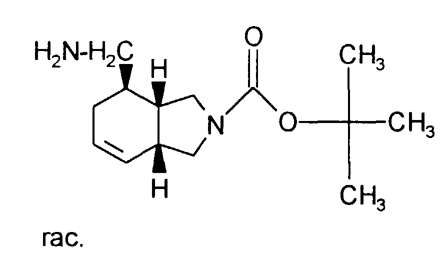

Eine Lösung der Azidoverbindung aus Beispiel K.6. (21,6,g; 78mmol) in 150 ml Pyridir/Wasser (5:1) wird unter Eiskühlung mit Schwefelwasserstoff gesättigt und anschließend 20 h bei Raumtemperatur belassen. Nach vollständigem Umsatz wird in vacuo eingeengt, mehrfach mit Toluol nachdestilliert und der Rückstand chromatographiert (Laufmittel: Cyclohexan/Aceton 1:1).
Ausbeute: 11,0 g (66 % der Theorie)
RF = 0.12 (Cyclohexan/Aceton 1:1)

### K.8. (1RS,2RS,6RS)-8-tert.-Butyloxycarbonyl-2-(ethyloxycarbonylaminomethyl-8-azabicyclo[4.3.0]non-4-en

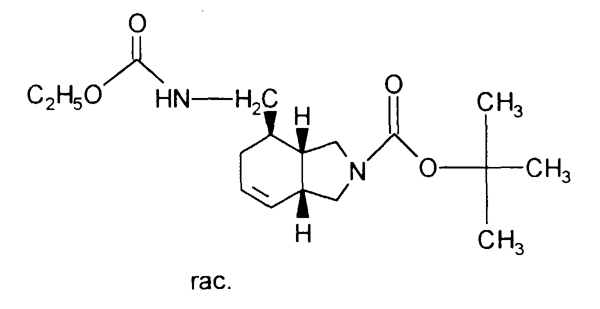

3,7 g (15 mmol) (1RS,2RS,6RS)-8-tert.-Butyloxycarbonyl-2-aminomethyl-8-azabicyclo[4.3.0]non-4-en werden in 40 ml Dioxan und 15 ml Wasser vorgelegt, 2,3 g (16 mmol) Kaliumcarbonat zugegeben und 1,75 g (16 mmol) Chlorameisensäureethylester bei Raumtemperatur zugetropft. Nach zweistündigem Rühren wird in vacuo eingeengt, der Rückstand in Dichlormethan (70 ml) aufgenommen, zweimal mit je 25 ml Wasser ausgeschüttelt, getrocknet (Natriumsulfat) und eingeengt. Das Rohprodukt wird durch Chromatographie (Cyclohexan/Aceton 2:1) gereinigt.
Ausbeute: 2,8 g (59 % der Theorie)
RF = 0.53 (Cyclohexan/Aceton 1:1)

### K.9. (1RS,2RS,6RS)-2-(Ethyloxycarbonylaminomethyl)-8-azabicyclo[4.3.0]non-4-en

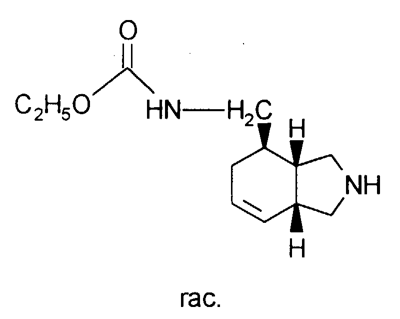

7,6 g (23 mmol) des Produktes aus Beispiel K.8. werden in 100 ml Methanol/Wasser (1:1) vorgelegt und bei Raumtemperatur 30 ml halbkonzentrierte Salzsäure zulaufen gelassen. Nachdem die Gasentwicklung beendet ist, wird 30 Minuten nachgerührt, mit Eiswasser (ca. 100 ml) verdünnt und mit konz. Natronlauge pH 12 eingestellt. Die wäßrige Phase wird viermal mit je 100 ml Dichlormethan extrahiert. Die Extrakte werden vereinigt, über Natriumsulfat getrocknet und in vacuo eingeengt.
Ausbeute: 3,9 g (76 % der Theorie)
RF = 0.45 (Dichlormethan/Methanol/konz. Ammoniak (2:4:0.1)

### Beispiel L:

### (1RS,2RS,6RS)-2-Aminomethyl-8-azabicyclo[4.3.0]non-4-en-bis-trifluormethansulfonat

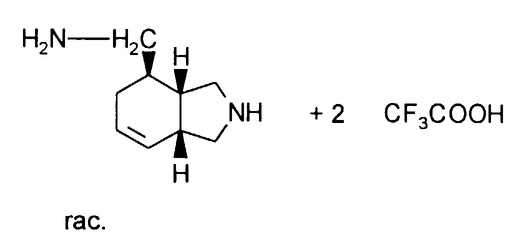

Eine Lösung von 2,0 g (8 mmol) (1RS,2RS,6RS)-8-tert.-Butyloxycarbonyl-2-aminomethyl-8-azabicyclo[4.3.0]non-4-en (Produkt aus Beispiel K.7.) in 30 ml Dichlormethan wird mit 30 ml Trifluoressigsäure versetzt und 30 Minuten bei Raumtemperatur belassen. Das Lösungsmittel und die Säure werden in Gegenwart von Toluol abdestilliert, mehrfach mit Toluol nachdestilliert. Das Produkt wird im Vakuum-Exsikkator über Kaliumhydroxid/Phosphorpentoxid (1:1) getrocknet.
Ausbeute: 1,5 g braunes Öl
¹H-NMR (DMSO-d₆): 5.78 (m, 1H, C=CH); 5.60 (m, 1H, C=CH); 3.34 (M, 2H); 3.03 (m, 1H), 2.87 (m, 2H), 2.73 (m, 1H); 2.45 (m, 1H); 2.34 (m, 1H); 2.22 (M, 1H); 1.94 ppm (m, 2H).
FAB-MS: M+1 = 153.

### Beispiel M:

### (1RS,2RS,6RS)-2-Ethyloxycarbonylaminomethyl-8-azabicyclo[4.3.0]non-4-en

(Das Produkt ist identisch mit der Titelverbindung aus Beispiel F.)

### M.1. (1RS,2RS,6SR)-2-Hydroxymethyl-8-azabicyclo[4.3.0]non-4-en

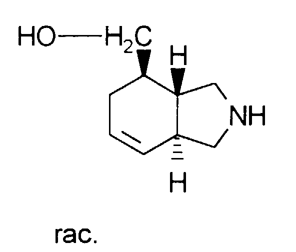

Ethyl-(1RS,2RS,6RS)-9-oxo-8-azabicyclo[4.3.0]non-4-en-2-carboxylat (Produkt B aus Beispiel K.2.) wird analog zu Beispiel K.3. umgesetzt.
Ausbeute: 75 % der Theorie
RF = 0.22 (Dichlormethan/Methanol/konz. Ammoniak (15:4:0.5)

### M.2. (1RS,2RS,6SR)-8-tert.-Butuloxycarbonyl-2-hydroxymethyl-8-azabicyclo[4.3.0]non-4-en

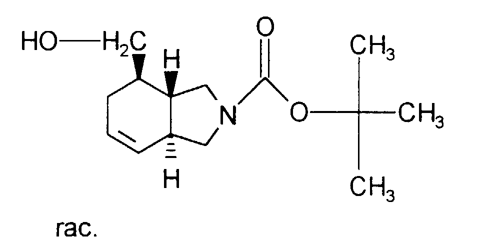

Das Produkt aus Beispiel M.1. wird analog zu Beispiel K.4. umgesetzt.
Ausbeute: 64 % der Theorie
RF = 0.23 (Cyclohexan/Aceton 2:1)

### M.3. (1RS,2RS,6SR)-8-tert.-Butyloxycarbonyl-2-(4-toluolsulfonyloxymethyl)-8-azabicyclo[4.3.0]non-4-en

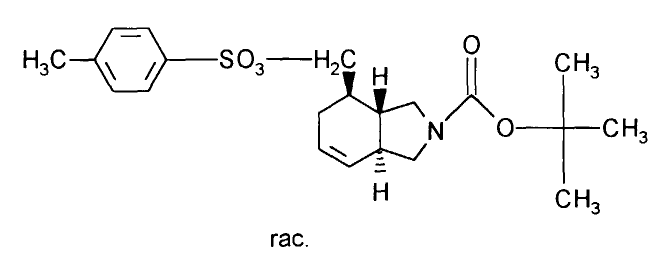

Die Titelverbindung wird aus dem Produkt von Beispiel M.2. in Analogie zum Beispiel F.1. erhalten.
Ausbeute: 91-98 % der Theorie
RF = 0.59 (Cyclohexan/Aceton 2:1)

### M.4. (1RS,2RS,6SR)-8-tert.-Butyloxycarbonyl-2-azidomethyl-8-azabicyclo[4.3.0]non-4-en

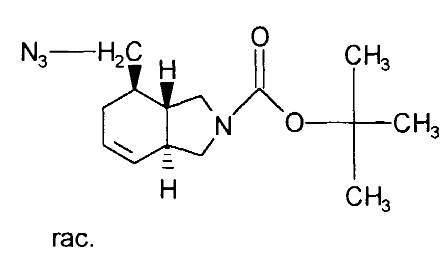

Eine Lösung von 13,0 g (32 mmol) des Produktes aus Beispiel M.3. in 80 ml N,N-Dimethylformamid wird mit 4,15 g (64 mmol) Natriumazid versetzt und 4 h bei 70°C gerührt. Sodann wird nochmals die gleiche Menge Natriumazid nachgesetzt und weitere 6 h bei 100°C gerührt. Anschließend wird aufgearbeitet, wie im Beispiel K.6. beschrieben.
Ausbeute: 7,0 g (79 % der Theorie)
RF = 0.55 (Cyclohexan/Aceton 2:1)

### M.5. (1RS,2RS,6SR)-8-tert.-Butyloxycarbonyl-2-aminomethyl-8-azabicyclo[4.3.0]non-4-en

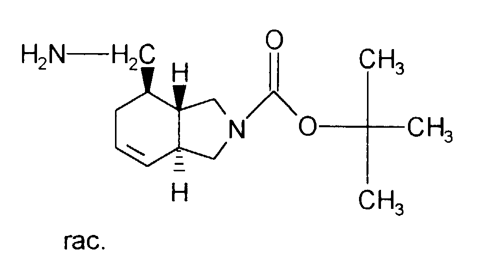

Die Azidoverbindung aus Beispiel M.4. wird umgesetzt wie im Beispiel K.7. beschrieben.

Die Chromatographie erfolgt mit Methanol/Dichlormethan/konz. Ammoniak (15:2:0.1).
Ausbeute: 75 % der Theorie
RF = 0.12 (Methanol/Dichlormethan/konz. Ammoniak 15:2:0.1)

### M.6. (1RS,2RS,6SR)-8-tert.-Butyloxycarbonyl-2-(ethyloxycarbonylmethyl)-8-azabicyclo[4.3.0]non-4-en

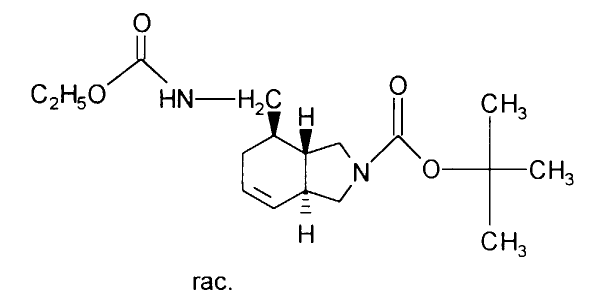

4.3 g (17 mmol) der Aminoverbindung aus Beispiel M.5. und 1,9 g (19 mmol) Triethylamin werden in 50 ml Dichlormethan vorgelegt, bei 0°C 2,2 g (20 mmol) Chlorameisensäureethylester, gelöst in 10 ml Dichlormethan, zugetropft und 24 h bei Raumtemperatur nachgerührt. Die Lösung wird mit Wasser (50 ml) versetzt und die Phasen getrennt. Die wäßrige Phase wird noch dreimal mit je 40 ml Dichlormethan extrahiert. Die organischen Phasen werden vereinigt, getrocknet (Natriumsulfat) und eingeengt.
Ausbeute: 5,3 g (96 % der Theorie)
¹H-NMR (CDCl₂-CDCl₂, 80°C): 5.79 (ddd, 1H, C=CH); 5.58 (m, 1H, C=CH); 4.61 (bs, 1H, Carbamat-NH); 4.23 (m, 1H); 4.12 (q, 2H, Ethyl-CH₂); 3.99 (m, 1H); 3.20-3.08 (m, 2H); 2.82 (m, 2H); 2.25 (m, 2H); 2.09 (m, 1H); 1.84 (m, 2H); 1.42 (s, 9H, tert.-Butyl); 1.37 ppm (t, 3H, Ethyl-CH₃).

### M.7. (1RS,2RS,6SR)-2-(Ethyloxycarbonylaminomethyl)-8-azabicyclo[4.3.0]non-4-en

(1RS,2RS,6SR)-8-tert.-Butyloxycarbonyl-2-(ethyloxycarbonylaminomethyl)-8-azabicyclo[4.3.0]non-4-en wird umgesetzt, wie im Beispiel K.9. beschrieben.
Ausbeute: quantitativ
RF = 0.55 (Methanol/Dichlormethan/konz. Ammoniak 15:4:0.5)

### Beispiel N:

### (1SR,2RS,6RS)-2-Methylamino-8-azabicyclo[4.3.0]non-en

### N.1. (1RS,2RS,6RS)-9-Oxo-azabicyclo[4.3.0]non-4-en-2-carbonsäure

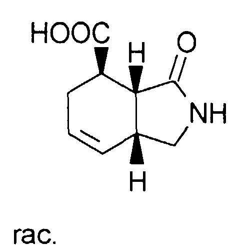

8,36 g (40 mmol) Ethyl-(1RS,2RS,6RS)-9-oxo-8-azabicyclo[4.3.0]non-4-en-2-carboxylat (Produkt A aus Beispiel K.2.) werden mit 30 ml Wasser und 5 ml konz.

Schwefelsäure 40 h bei 60°C gerührt. Beim Abkühlen fällt das Produkt aus. Der Niederschlag wird mit wenig kaltem Wasser gewaschen und im Vakuum-Trockenschrank bei 50°C getrocknet.
Ausbeute: 4,80 g (66 % der Theorie)
¹H-NMR (DMSO-d₆): 12.35 (s, 1H, COOH); 7.60 (s, 1H, Lactam-NH); 5.74 (m, 1H, C=CH); 5.59 (m, 1H, C=CH); 3.45 (dd, 1H, 7a-H); 2.95-2.85 (m, 4H, 1-H, 2-H, 6-H, 7b-H); 2.29 (m, 1H, 3a-H); 2.00 ppm (m, 1H, 3b-H).

### N.2. (1SR,2RS,6RS)-2-Ethoxycarbonylamino-9-oxo-8-azabicyclo[4.3.0]non-4-en

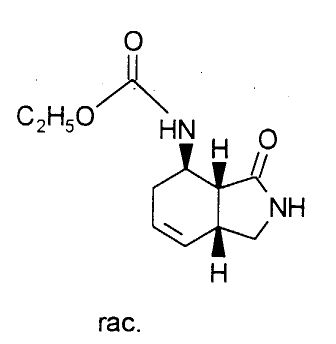

(1RS,2RS,6RS)-9-Oxo-8-azabicyclo[4.3.0]non-4-en-2-carbonsäure (Titelverbindung aus Beispiel N.1.) wird analog Beispiel C.2. umgesetzt.
Ausbeute: 68 % der Theorie
RF = 0.06 (Cyclohexan/Aceton 1:1)

### N.3. (1SR,2RS,6RS)-2-Methylamino-8-azabicyclo[4.3.0]non-4-en

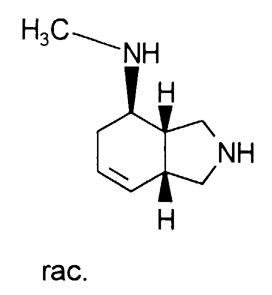

Die Titelverbindung wird erhalten, indem das Produkt aus Beispiel N.2. mit 10 Equivalenten Di(isobutyl)aluminiumhydrid analog Beispiel K.3. umgesetzt und aufgearbeitet wird.
Ausbeute: 51 % der Theorie
¹H-NMR (CDCl₃): 5.72 (m, 1H, C=CH); 5.68 (m, 1H, C=CH); 3.19-3.10 (m, 2H); 2.88 (dd, 1H); 2.60 (dd, 1H); 2.50 (m, 1H); 2.44 (s, 3H, N-CH₃); 2.33-2.28 (m, 2H); 2.19 (m, 1H); 1.89 ppm (m, 1H).

### Beispiel O:

### (1SR,2SR,6RS)-2-Methylamino-8-azabicyclo[4.3.0]non-4-en

### 0.1. (1RS,2SR,6RS)-9-Oxo-8-azabicyclo[4.3.0]non-4-en-2-carbonsäure

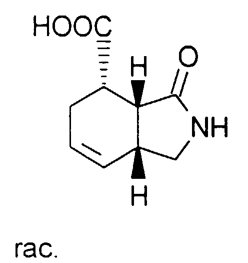

0,2 g konz. Schwefelsäure, 25 ml Wasser und 25 ml Essigsaure werden bei 60°C vorgelegt. 9,8 g (50 mmol) des Produktes aus Beispiel G.3. werden in kleinen Portionen zugegeben. Man rührt 5 h bei 60°C nach. Zur Aufarbeitung wird eine Lösung von 0,8 g Natriumhydrogencarbonat in 10 ml Wasser zugegeben und in vacuo eingeengt. Der Rückstand wird in 40 ml Wasser suspendiert und unter Eiskühlung durch Zugabe konz. Natronlauge in Lösung gebracht. Nachdem von unlöslichen Anteilen abgesaugt worden ist, wird mit halbkonzentrierter Salzsäure sauer gestellt und wieder auf 0°C abgekühlt. Das ausfallende Produkt wird mit wenig kaltem Wasser gewaschen und nachfolgend im Vakuum-Trockenschrank bei 50°C getrocknet.
Ausbeute: 4,8 g (53 % der Theorie)
Schmelzpunkt: 192-193°C

### O.2. (1SR,2SR,6RS)-2-Ethoxycarbonylamino-9-oxo-8-azabicyclo[4.3.0]non-4-en

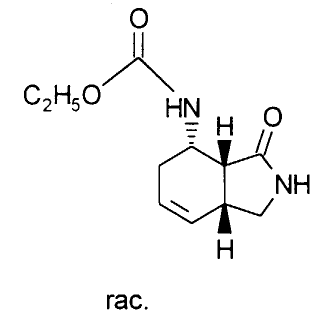

(1RS,2SR,6RS)-9-Oxo-8-azabicyclo[4.3.0]non-4-en-2-carbonsäure (Titelverbindung aus Beispiel O.1.) wird umgesetzt wie im Beispiel C.2. beschrieben.
Ausbeute: 68 % der Theorie
Schmelzpunkt: 160-164°C

### O.3. (1SR,2SR,6RS)-2-Methylamino-8-azabicyclo[4.3.0]non-4-en

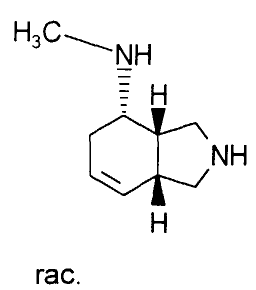

Die Titelverbindung wird erhalten, indem das Produkt aus Beispiel O.2. mit 10 Equivalenten Di(isobutyl)aluminiumhydrid analog Beispiel K.3. umgesetzt und aufgearbeitet wird.
Ausbeute: 81 % der Theorie
¹H-NMR (CDCl₃): 5.72 (m, 1H, C=CH); 5.50 (m, 1H, C=CH); 3.04-2.77 (m, 6H); 2.60 (m; 1H); 249 (s, 3H, N-CH₃); 2.31 (bs, 2H, 2xNH); 2.25 (m, 1H); 1.89 ppm (m, 1H).

### Beispiel P

### 9,10-Difluor-3-methyl-8-nitro-7-oxo-2,3-dihydro-7H-pyrido[1,2,3-d,e][1,3,4]benzoxadiazin-6-carbonsäure

Zu 7,0 g (24,8 mmol) 9,10-Difluor-3-methyl-7-oxo-2,3-dihydro-7H-pyrido[1,2,3-d,e][1,3,4]-benzoxadiazin-6-carbonsäure gelöst in 90 ml konzentrierter Schwefelsäure gibt man portionsweise 3,7 g (36,6 mmol) Kaliumnitrat und läßt eine Stunde bei Raumtemperatur rühren. Die Reaktionsmischung wird auf 270 ml Eiswasser gegeben. Der resultierende Niederschlag wird abgesaugt, mit Wasser gewaschen und getrocknet.
Ausbeute: 6,9 g (85 % der Theorie)
Schmelzpunkt: >300°C

### Beispiel Q

### 8-Amino-9,10-difluor-3-methyl-7-oxo-2,3-dihydro-7H-pyrido[1,2,3-d,e][1,3,4]benzoxadiazin-6-carbonsäure

4,0 g (12,2 mmol) 9,10-Difluor-3-methyl-8-nitro-7-oxo-2,3-dihydro-7H-pyrido[1,2,3-d,e][1,3,4]benzoxadiazin-6-carbonsäure und 1,2 g Palladium auf Aktivkohle (10 % Palladium) werden in 280 ml Ethanol suspendiert und zwei Tage unter Normaldruck bei Raumtemperatur hydriert. Die Reaktionsmischung wird mit 280 ml Wasser versetzt und anschließend mit 2 N Natronlauge auf pH 10-11 eingestellt. Der Hydrierkatalysator wird abfiltriert und das Filtrat mit 2 N Salzsäure auf pH 5-6 eingestellt. Der resultierende Niederschlag wird abfiltriert, mit Methanol gewaschen und getrocknet (Fraktion A). Der abfiltrierte Hydrierkatalysator wird dreimal in je 100 ml Dimethylformamid (DMF) für eine Stunde unter Rückfluß erhitzt und anschließend wieder abfiltriert. Die vereinigten DMF-Lösungen werden im Vakuum eingeengt und getrocknet (Fraktion B).
Ausbeute: 3,0 g (83 % der Theorie)
Schmelzpunkt: >300°C

## Patentansprüche

1. 8-Amino-10-(azabicycloalkyl)-pyrido[1,2,3-d,e][1,3,4]benzoxadiazinderivate der allgemeinen Formel (I) 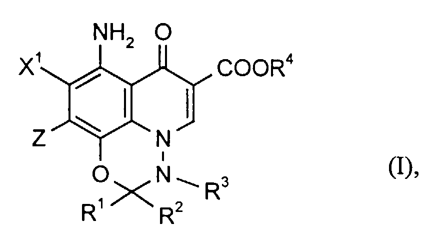 in welcher
R¹ für Wasserstoff oder gegebenenfalls durch Hydroxy oder Halogen substituiertes C₁-C₄-Alkyl steht,
R² unabhängig von R¹ für Wasserstoff oder Methyl steht,
R³ für Wasserstoff oder C₁-C₄-Alkyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
X¹ für Wasserstoff oder Halogen steht,
Z für Reste der Strukturen 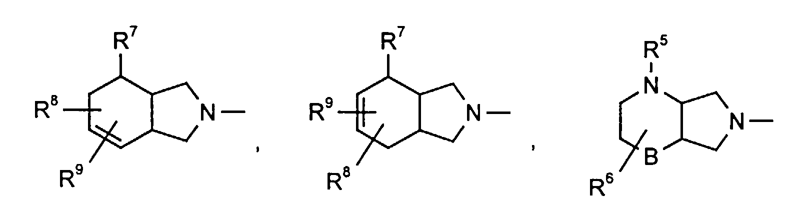
steht,
worin
R⁷ für Wasserstoff, Hydroxy, -NR¹⁰R¹¹, Hydroxymethyl, -CH₂-NR¹⁰R¹¹, Carboxyl, Methoxycarbonyl oder Ethoxycarbonyl steht,
wobei
R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoff oder Methyl steht,
R⁶ für Wasserstoff oder Methyl steht,
R⁵ für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
R^{5'} für Methyl oder Ethyl steht,
B für -CH₂-, O oder eine direkte Bindung steht, und deren pharmazeutisch verwendbaren Hydrate und Säureadditionssalze sowie ihre Alkali-, Erdalkali-, Silber- und Guanidiniumsalze.

2. Verfahren zur Herstellung der
8-Amino-10-(azabicycloalkyl)-pyrido[1,2,3-d,e][1,3,4]benzoxadiazin-derivate der allgemeinen Formel (I) 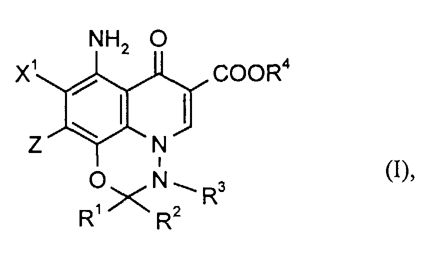 in welcher
R¹ für Wasserstoff oder gegebenenfalls durch Hydroxy oder Halogen substituiertes C₁-C₄-Alkyl steht,
R² unabhängig von R¹ für Wasserstoff oder Methyl steht,
R³ für Wasserstoff oder C₁-C₄-Alkyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
X¹ für Wasserstoff oder Halogen steht,
Z für Reste der Strukturen 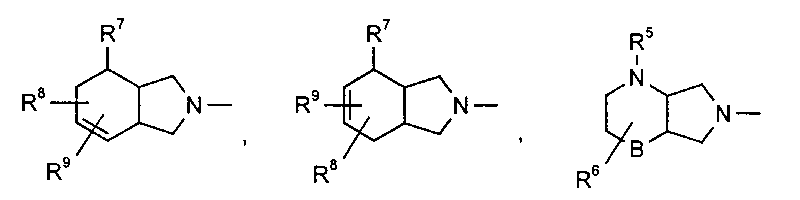
steht,
worin
R⁷ für Wasserstoff, Hydroxy, -NR¹⁰R¹¹, Hydroxymethyl, -CH₂-NR¹⁰R¹¹, Carboxyl, Methoxycarbonyl oder Ethoxycarbonyl steht,
wobei
R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoffoder Methyl steht,
R⁶ für Wasserstoff oder Methyl steht,
R⁵ für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
R^{5'} für Methyl oder Ethyl steht,
B für -CH₂-, O oder eine direkte Bindung steht,
dadurch, gekennzeichnet, daß man Verbindungen der Formel (II) 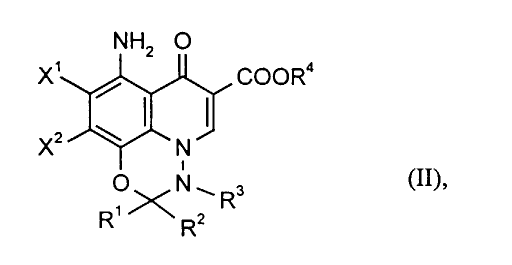 in welcher
R¹, R², R³, R⁴ und X¹ die oben angegebene Bedeutung haben und
X² für Halogen, insbesondere Fluor oder Chlor, steht,
mit Verbindungen der Formel (III)
Z-H (III),
in welcher
Z die oben angegebene Bedeutung hat,
gegebenenfalls in Gegenwart von Säurebindemitteln umsetzt.

3. Verbindungen der Formel (II) 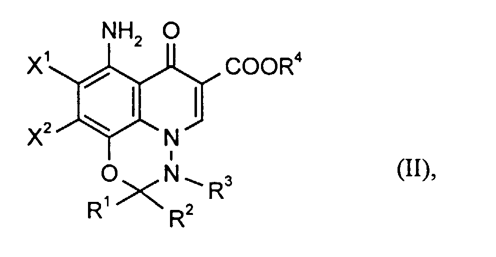 in welcher
R¹, R², R³, R⁴ und X¹ die in Anspruch 1 angegebene Bedeutung haben und
X² für Halogen steht.

4. Verfahren zur Herstellung der Verbindungen der Formel (II) gemäß Anspruch 3, dadurch gekennzeichnet, daß man Verbindungen der Formel (IV) 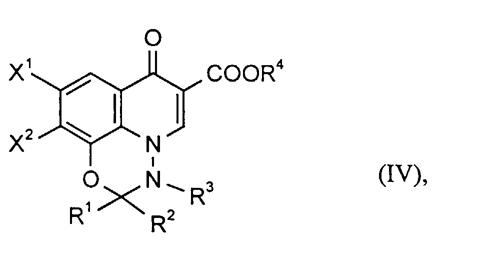 in welcher R¹, R², R³, R⁴, X¹ und X² die in Anspruch 3 angegebene Bedeutung haben, mit Nitrierungsreagenzien umsetzt und anschließend die erhaltenen Nitroverbindungen reduziert.

5. Verbindungen der Formel (I) gemäß Anspruch 1, in welcher
R¹ für Wasserstoff oder gegebenenfalls durch Hydroxy substituiertes C₁-C₃-Alkyl steht,
R² unabhängig von R¹ für Wasserstoff oder Methyl steht,
R³ für Wasserstoff, Methyl oder Ethyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)methyl steht,
X¹ für Wasserstoff, Fluor oder Chlor steht,
Z für Reste der Strukturen 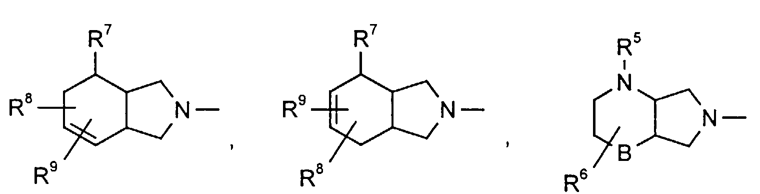
steht,
worin
R⁷ für Wasserstoff, Hydroxy, -NR¹⁰R¹¹, Hydroxymethyl oder -CH₂-NR¹⁰R¹¹ steht,
wobei
R¹⁰ für Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoff oder Methyl steht,
R⁵ für Wasserstoff oder Methyl steht,
R⁶ für Wasserstoff steht,
B für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

6. Verbindungen der Formel (I) gemäß Anspruch 1, in welcher
R¹ für Wasserstoff oder Methyl steht,
R² für Wasserstoff,
R³ für Methyl oder Ethyl steht,
R⁴ für Wasserstoff, Methyl oder Ethyl steht,
X¹ für Fluor steht,
Z für Reste der Strukturen 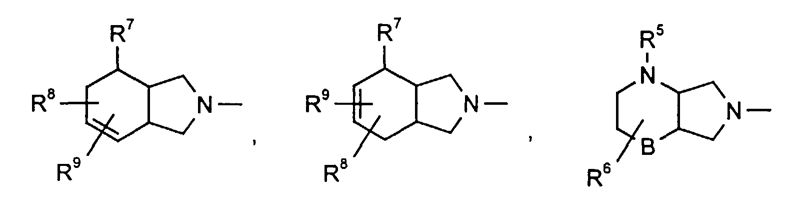
steht,
worin
R⁷ für Wasserstoff, Hydroxy, -NR¹⁰R¹¹, Hydroxymethyl oder -CH₂-NR¹⁰R¹¹ steht,
wobei
R¹⁰ für Wasserstoff, Methyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁶ für Wasserstoff steht,
R⁹ für Wasserstoff oder Methyl steht,
R⁵ für Wasserstoff oder Methyl steht,
B für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

7. Arzneimittel enthaltend Verbindungen der Formel (I) gemäß Anspruch 1.

8. Verwendung von Verbindungen der Formel (I) gemäß Anspruch 1 zur Herstellung von Arzneimitteln.

9. Verwendung von Verbindungen der Formel (I) gemäß Anspruch 1 zur Herstellung von antibakteriellen Mitteln.

## Claims

1. 8-Amino-10-(azabicycloalkyl)-pyrido[1,2,3-d,e][1,3,4]benzoxadiazine derivatives of the general formula (I) 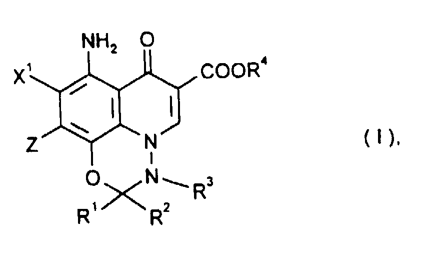 in which
R¹ represents hydrogen or C₁-C₄-alkyl which is optionally substituted by hydroxyl or halogen,
R² independently of R¹ represents hydrogen or methyl,
R³ represents hydrogen or C₁-C₄-alkyl,
R⁴ represents hydrogen, alkyl having 1 to 4 carbon atoms, which is optionally substituted by hydroxyl, methoxy, amino, methylamino or dimethylamino, or (5-methyl-2-oxo-1,3-dioxol-4-yl)-methyl,
X¹ represents hydrogen or halogen,
Z represents radicals with the structures 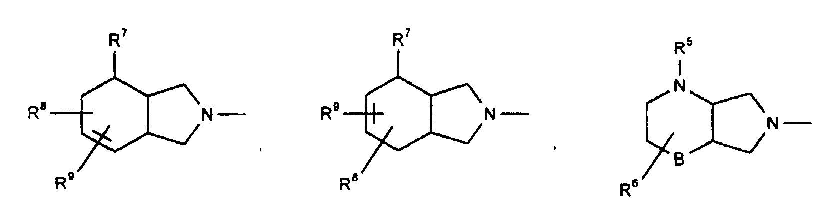
in which
R⁷ represents hydrogen, hydroxyl, -NR¹⁰R¹¹, hydroxymethyl, -CH₂-NR¹⁰R¹¹, carboxyl, methoxycarbonyl or ethoxycarbonyl,
where
R¹⁰ represents hydrogen, C₁-C₃-alkyl which is optionally substituted by hydroxyl, alkoxycarbonyl having 1 to 4 C atoms in the alkoxy moiety or C₁-C₃-acyl,
R¹¹ represents hydrogen or methyl,
R⁸ represents hydrogen, straight-chain or branched C₁-C₃-alkyl or cyclopropyl,
R⁹ represents hydrogen or methyl,
R⁶ represents hydrogen or methyl,
R⁵ represents hydrogen, methyl or radicals with the structures -CH=CH-CO₂R^{5'}, -CH₂-CH₂-CO₂R^{5'}, -CH₂-CO-CH₃, -CH₂-CH₂-CN,
R^{5'} represents methyl or ethyl,
B represents -CH₂-, O or a direct bond and their pharmaceutically utilizable hydrates and acid addition salts, and their alkali metal, alkaline earth metal, silver and guanidinium salts.

2. Process for the preparation of the
8-amino-10-(azabicycloalkyl)-pyrido[1,2,3-d,e][1,3,4]benzoxadiazine derivatives of the general formula (I) 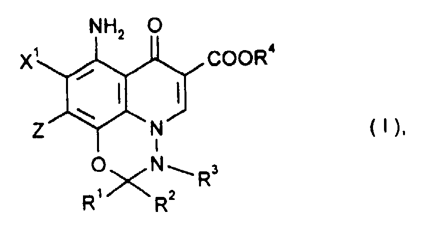 in which
R¹ represents hydrogen or C₁-C₄-alkyl which is optionally substituted by hydroxyl or halogen,
R² independently of R¹ represents hydrogen or methyl,
R³ represents hydrogen or C₁-C₄-alkyl,
R⁴ represents hydrogen, alkyl having 1 to 4 carbon atoms, which is optionally substituted by hydroxyl, methoxy, amino, methylamino or dimethylamino, or (5-methyl-2-oxo-1,3-dioxol-4-yl)-methyl,
X¹ represents hydrogen or halogen,
Z represents radicals with the structures 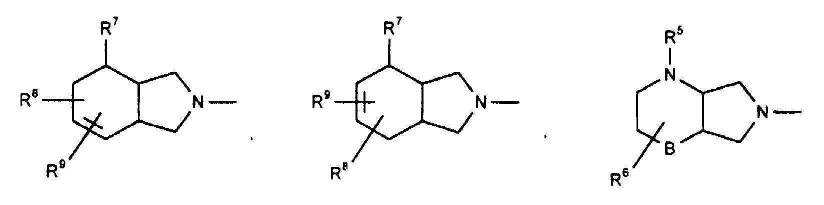
in which
R⁷ represents hydrogen, hydroxyl, -NR¹⁰R¹¹, hydroxymethyl, -CH₂-NR¹⁰R¹¹, carboxyl, methoxycarbonyl or ethoxycarbonyl,
where
R¹⁰ represents hydrogen, C₁-C₃-alkyl which is optionally substituted by hydroxyl, alkoxycarbonyl having 1 to 4 C atoms in the alkoxy moiety or C₁-C₃-acyl,
R¹¹ represents hydrogen or methyl,
R⁸ represents hydrogen, straight-chain or branched C₁-C₃-alkyl or cyclopropyl,
R⁹ represents hydrogen or methyl,
R⁶ represents hydrogen or methyl,
R⁵ represents hydrogen, methyl or radicals with the structures -CH=CH-CO₂R^{5'}, -CH₂-CH₂-CO₂R^{5'}, -CH₂-CO-CH₃, -CH₂-CH₂-CN,
R^{5'} represents methyl or ethyl,
B represents -CH₂-, O or a direct bond,
characterized in that compounds of the formula (II) 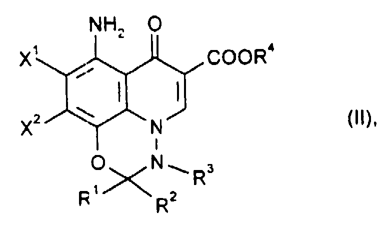 in which
R¹, R², R³, R⁴ and X¹ have the meaning given above and
X² represents halogen, in particular fluorine or chlorine,
are reacted with compounds of the formula (III)
Z-H (III)
in which
Z has the meaning given above,
if appropriate in the presence of acid-binding agents.

3. Compounds of the formula (II) 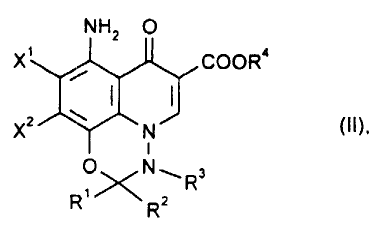 in which
R¹, R², R³, R⁴ and X¹ have the meaning given in Claim 1 and
X² represents halogen.

4. Process for the preparation of the compounds of the formula (II) according to Claim 3, characterized in that compounds of the formula (IV) 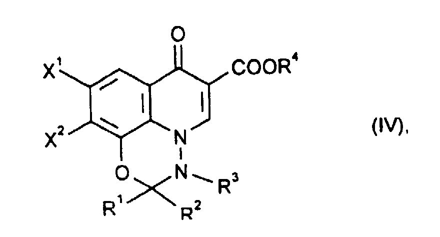 in which R¹, R², R³, R⁴, X¹ and X² have the meaning given in Claim 3, are reacted with nitrating reagents and the nitro compounds obtained are then reduced.

5. Compounds of the formula (I) according to Claim 1, in which
R¹ represents hydrogen or C₁-C₃-alkyl which is optionally substituted by hydroxyl,
R² independently of R¹ represents hydrogen or methyl,
R³ represents hydrogen, methyl or ethyl,
R⁴ represents hydrogen, alkyl having 1 to 4 carbon atoms, which is optionally substituted by hydroxyl, methoxy, amino, methylamino or dimethylamino, or (5-methyl-2-oxo-1,3-dioxol-4-yl)-methyl,
X¹ represents hydrogen, fluorine or chlorine,
Z represents radicals with the structures 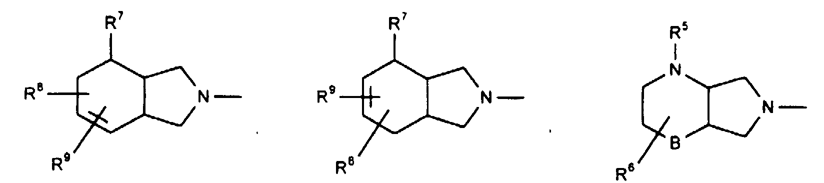
in which
R⁷ represents hydrogen, hydroxyl, -NR¹⁰R¹¹, hydroxymethyl or -CH₂-NR¹⁰R¹¹,
where
R¹⁰ represents hydrogen, C₁-C₂-alkyl which is optionally substituted by hydroxyl, alkoxycarbonyl having 1 to 4 C atoms in the alkoxy moiety or C₁-C₃-acyl,
R¹¹ represents hydrogen or methyl,
R⁸ represents hydrogen, straight-chain or branched C₁-C₃-alkyl or cyclopropyl,
R⁹ represents hydrogen or methyl,
R⁵ represents hydrogen or methyl,
R⁶ represents hydrogen,
B represents -CH₂-, O or a direct bond
and their pharmaceutically utilizable hydrates and acid addition salts, and their alkali metal, alkaline earth metal, silver and guanidinium salts.

6. Compounds of the formula (I) according to Claim 1, in which
R¹ represents hydrogen or methyl,
R² represents hydrogen,
R³ represents methyl or ethyl,
R⁴ represents hydrogen, methyl or ethyl,
X¹ represents fluorine,
Z represents radicals with the structures 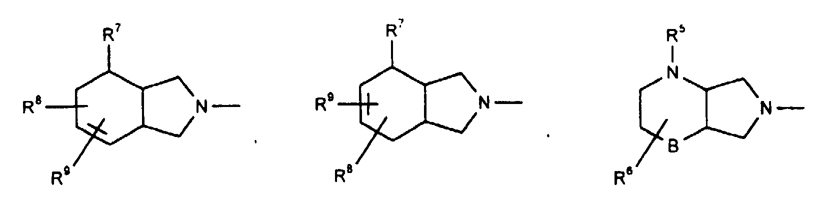
in which
R⁷ represents hydrogen, hydroxyl, -NR¹⁰R¹¹, hydroxymethyl or -CH₂-NR¹⁰R¹¹,
where
R¹⁰ represents hydrogen, methyl, alkoxycarbonyl having 1 to 4 C atoms in the alkoxy moiety or C₁-C₃-acyl,
R¹¹ represents hydrogen or methyl,
R⁸ represents hydrogen, straight-chain or branched C₁-C₃-alkyl or cyclopropyl,
R⁶ represents hydrogen,
R⁹ represents hydrogen or methyl,
R⁵ represents hydrogen or methyl,
B represents -CH₂-, O or a direct bond
and their pharmaceutically utilizable hydrates and acid addition salts, and their alkali metal, alkaline earth metal, silver and guanidinium salts.

7. Medicaments containing compounds of the formula (I) according to Claim 1.

8. Use of compounds of the formula (I) according to Claim 1 for the production of medicaments.

9. Use of compounds of the formula (I) according to Claim 1 for the production of antibacterial compositions.

## Revendications

1. Dérivés de 8-amino-10-(azabicycloalkyl)pyrido[1,2,3-d,e][1,3,4]benzoxadiazine de formule générale (I) 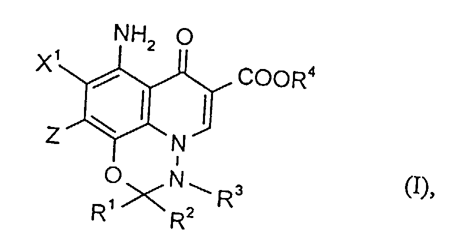 dans laquelle
R¹ représente l'hydrogène ou un groupe alkyle en C₁ à C₄ éventuellement substitué par un radical hydroxy ou un halogène,
R² représente, indépendamment de R¹, l'hydrogène ou le groupe méthyle,
R³ est l'hydrogène ou un groupe alkyle en C₁ à C₄,
R⁴ représente l'hydrogène, un groupe alkyle ayant 1 à 4 atomes de carbone éventuellement substitué par un radical hydroxy, méthoxy, amino, méthylamino ou diméthylamino, ou représente un groupe (5-méthyl-2-oxo-1,3-dioxole-4-yl)-méthyle,
X¹ est l'hydrogène ou un halogène,
Z représente les restes de formules 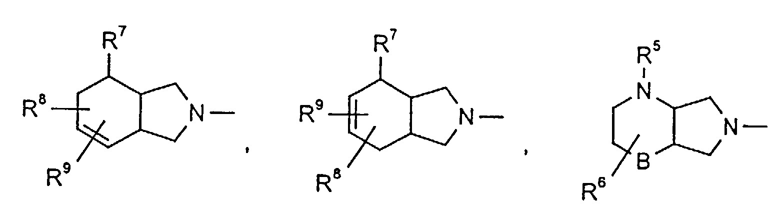
dans lesquelles
R⁷ est l'hydrogène, un groupe hydroxy, -NR¹⁰R¹¹, hydroxyméthyle, -CH₂-NR¹⁰R¹¹, carboxyle, méthoxy-carbonyle ou éthoxycarbonyle,
où
R¹⁰ est l'hydrogène, un groupe alkyle en C₁ à C₃ portant éventuellement un substituant hydroxy, un groupe alkoxycarbonyle ayant 1 à 4 atomes de carbone dans la partie alkoxy, ou un groupe acyle en C₁ à C₃,
R¹¹ est l'hydrogène ou le groupe méthyle,
R⁸ représente l'hydrogène, un groupe alkyle en C₁ à C₃ linéaire ou ramifié ou le groupe cyclopropyle,
R⁹ est l'hydrogène ou le groupe méthyle,
R⁶ est l'hydrogène ou le groupe méthyle,
R⁵ représente l'hydrogène, le groupe méthyle ou des restes de formules -CH=CH-CO₂R^{5'}, -CH₂-CH₂-CO₂R^{5'}, -CH₂-CO-CH₃, -CH₂-CH₂-CN,
R^{5'} est le groupe méthyle ou éthyle,
B représente le groupe -CH₂-, O ou une liaison directe,
et leurs hydrates et sels d'addition d'acides acceptables du point de vue pharmaceutique ainsi que leurs sels de métaux alcalins, de métaux alcalino-terreux, d'argent et de guanidinium.

2. Procédé de production des dérivés de 8-amino-10-(azabicycloalkyl)-pyrido[1,2,3-d,e][1,3,4]benzoxadiazine de formule générale (I) 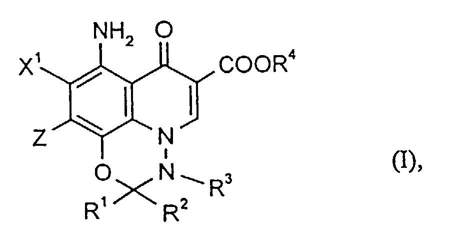 dans laquelle
R¹ représente l'hydrogène ou un groupe alkyle en C₁ à C₄ éventuellement substitué par un radical hydroxy ou un halogène,
R² représente, indépendamment de R¹, l'hydrogène ou le groupe méthyle,
R³ est l'hydrogène ou un groupe alkyle en C₁ à C₄,
R⁴ représente l'hydrogène, un groupe alkyle ayant 1 à 4 atomes de carbone éventuellement substitué par un radical hydroxy, méthoxy, amino, méthylamino ou diméthylamino, ou représente un groupe (5-méthyl-2-oxo-1,3-dioxole-4-yl)-méthyle,
X¹ est l'hydrogène ou un halogène,
Z représente les restes de formules 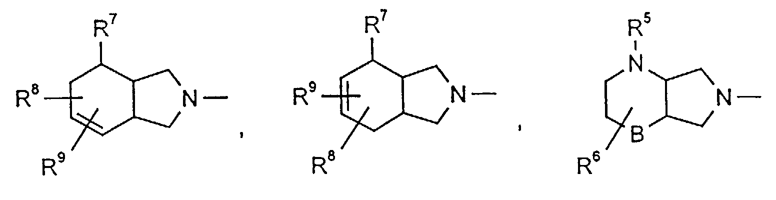
dans lesquelles
R⁷ est l'hydrogène, un groupe hydroxy, -NR¹⁰R¹¹, hydroxyméthyle, -CH₂-NR¹⁰R¹¹, carboxyle, méthoxycarbonyle ou éthoxycarbonyle,
où
R¹⁰ est l'hydrogène, un groupe alkyle en C₁ à C₃ portant éventuellement un substituant hydroxy, un groupe alkoxycarbonyle ayant 1 à 4 atomes de carbone dans la partie alkoxy, ou un groupe acyle en C₁ à C₃,
R¹¹ est l'hydrogène ou le groupe méthyle,
R⁸ représente l'hydrogène, un groupe alkyle en C₁ à C₃ linéaire ou ramifié ou le groupe cyclopropyle,
R⁹ est l'hydrogène ou le groupe méthyle,
R⁶ est l'hydrogène ou le groupe méthyle,
R⁵ représente l'hydrogène, le groupe méthyle ou des restes de formules -CH=CH-CO₂R^{5'}, -CH₂-CH₂-CO₂R^{5'}, -CH₂-CO-CH₃, -CH₂-CH₂-CN,
R^{5'} est le groupe méthyle ou éthyle,
B représente le groupe -CH₂-, O ou une liaison directe,
caractérisé en ce qu'on fait réagir des composés de formule (II) 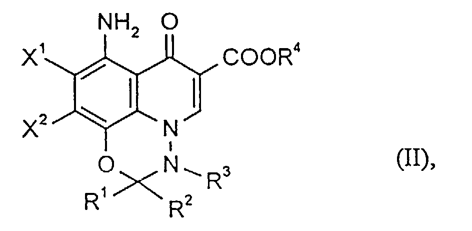 dans laquelle
R¹, R² , R³ , R⁴ et X¹ ont la définition indiquée ci-dessus et
X² représente un halogène, en particulier le fluor ou le chlore,
avec des composés de formule (III)
Z-H (III),
dans laquelle
Z a la définition indiquée ci-dessus,
le cas échéant en présence d'accepteurs d'acides.

3. Composés de formule (II) 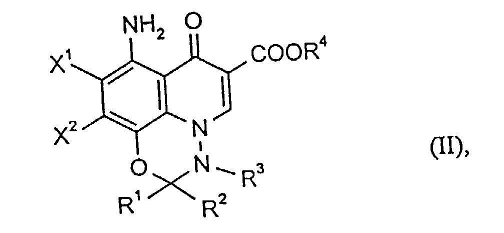 dans laquelle
R¹, R², R³, R⁴ et X¹ ont la définition indiquée ci-dessus et
X² représente un halogène.

4. Procédé de production de composés de formule (II) suivant la revendication 3, caractérisé en ce qu'on fait réagir des composés de formule (IV) 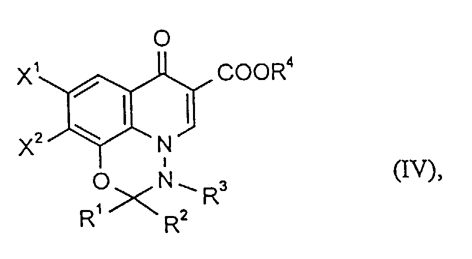 dans laquelle R¹, R², R³, R⁴, X¹ et X² ont la définition indiquée dans la revendication 3, avec des réactifs de nitration, puis on réduit les composés nitrés obtenus.

5. Composés de formule (I) suivant la revendication 1, dans laquelle
R¹ représente l'hydrogène ou un groupe alkyle en C₁ à C₃ éventuellement substitué par un radical hydroxy,
R² représente, indépendamment de R¹, l'hydrogène ou le groupe méthyle,
R³ est l'hydrogène, le groupe méthyle ou le groupe éthyle,
R⁴ représente l'hydrogène, un groupe alkyle ayant 1 à 4 atomes de carbone éventuellement substitué par un radical hydroxy, méthoxy, amino, méthylamino ou diméthylamino, ou représente un groupe (5-méthyl-2-oxo-1,3-dioxole-4-yl)-méthyle,
X¹ est l'hydrogène, le fluor ou le chlore,
Z représente les restes de formules 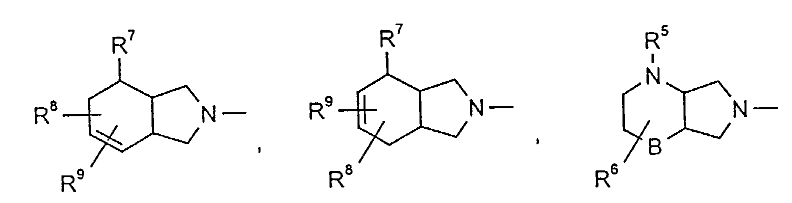
dans lesquelles
R⁷ est l'hydrogène, un groupe hydroxy, -NR¹⁰R¹¹, hydroxyméthyle ou -CH₂-NR¹⁰R¹¹,
où
R¹⁰ est l'hydrogène, un groupe alkyle en C₁ ou C₂ éventuellement substitué par un radical hydroxy, un groupe alkoxycarbonyle ayant 1 à 4 atomes de carbone dans la partie alkoxy, ou un groupe acyle en C₁ à C₃,
R¹¹ est l'hydrogène ou le groupe méthyle,
R⁸ représente l'hydrogène, un groupe alkyle en C₁ à C₃ linéaire ou ramifié ou le groupe cyclopropyle,
R⁹ est l'hydrogène ou le groupe méthyle,
R⁵ est l'hydrogène ou le groupe méthyle,
R⁶ est l'hydrogène,
B est le groupe -CH₂-, O ou une liaison directe
et leurs hydrates et sels d'addition d'acides utilisables en pharmacie ainsi que leurs sels de métaux alcalins, de métaux alcalino-terreux, d'argent et de guanidinium.

6. Composés de formule (I) suivant la revendication 1, dans laquelle
R¹ est l'hydrogène ou le groupe méthyle,
R² est l'hydrogène,
R³ est le groupe méthyle ou éthyle,
R⁴ est l'hydrogène, le groupe méthyle ou éthyle,
X¹ est le fluor,
Z représente des restes de formules 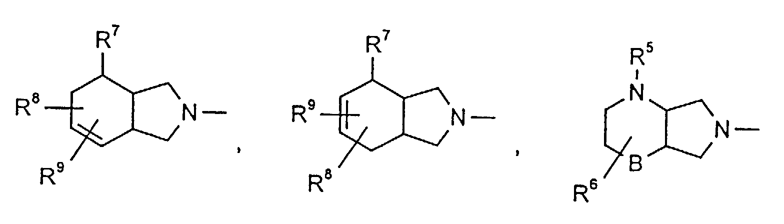
dans lesquelles
R⁷ est l'hydrogène, un groupe hydroxy, -NR¹⁰R¹¹, hydroxyméthyle ou -CH₂-NR¹⁰R¹¹,
où
R¹⁰ est l'hydrogène, le groupe méthyle, un groupe alkoxycarbonyle ayant 1 à 4 atomes de carbone dans la partie alkoxy ou un groupe acyle en C₁ à C₃,
R¹¹ est l'hydrogène ou le groupe méthyle,
R⁸ représente l'hydrogène, un groupe alkyle en C₁ à C₃ linéaire ou ramifié ou le groupe cyclopropyle,
R⁶ est l'hydrogène,
R⁹ est l'hydrogène ou le groupe méthyle,
R⁵ est l'hydrogène ou le groupe méthyle,
B représente le groupe -CH₂-, O ou une liaison directe
et leurs hydrates et leurs sels d'addition d'acides utilisables en pharmacie ainsi que leurs sels de métaux alcalins, de métaux alcalino-terreux, d'argent et de guanidinium.

7. Médicaments contenant des composés de formule (I) suivant la revendication 1.

8. Utilisation de composés de formule (I) suivant la revendication 1 pour la préparation de médicaments.

9. Utilisation de composés de formule (I) suivant la revendication 1 pour la préparation de compositions antibactériennes.
